(19) Europäisches Patentamt
European Patent Office
Office européen des brevets

(11)  **EP 3 858 392 B1**

(12)  **EUROPEAN PATENT SPECIFICATION**

(45) Date of publication and mention
of the grant of the patent:
**03.01.2024  Bulletin 2024/01**

(51) International Patent Classification (IPC):
**A61L 15/24** *(2006.01)*   **A61L 15/26** *(2006.01)*
**A61L 15/40** *(2006.01)*   **A61L 15/42** *(2006.01)*
**A61L 15/46** *(2006.01)*

(21) Application number: **20212485.5**

(22) Date of filing: **03.02.2016**

(52) Cooperative Patent Classification (CPC):
(C-Sets available)
**A61K 38/443; A01N 63/14; A61L 15/24;
A61L 15/26; A61L 15/40; A61L 15/425;
A61L 15/46; A61P 17/02; A61P 29/00; A61P 31/00;
A61P 31/04; A61P 43/00; C12Y 101/03004;**
A61L 2300/11; A61L 2300/232;        (Cont.)

(54) **ANTIMICROBIAL FIBERS AND COMPOSITIONS**

ANTIMIKROBIELLE FASERN UND ZUSAMMENSETZUNGEN

COMPOSITIONS ET FIBRES ANTIMICROBIENNES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.02.2015  GB 201501805
14.05.2015  GB 201508311
03.06.2015  GB 201509652
23.07.2015  GB 201513046
15.10.2015  GB 201518258
04.11.2015  GB 201519484
24.11.2015  GB 201520771
18.12.2015  GB 201522450**

(43) Date of publication of application:
**04.08.2021  Bulletin 2021/31**

(62) Document number(s) of the earlier application(s) in
accordance with Art. 76 EPC:
**16711333.1 / 3 253 424**

(73) Proprietor: **Matoke Holdings Limited
Abingdon, Oxfordshire OX13 5HR (GB)**

(72) Inventors:
• **STAPLES, Ian
Oxfordshire, OX13 5HR (GB)**
• **ELDER, Iain
Oxfordshire, OX13 5HR (GB)**
• **CALLAGHAN, Annette
Oxfordshire, OX13 5HR (GB)**

• **DRYDEN, Matthew
Oxfordshire, OX13 5HR (GB)**
• **KERSHAW, David
Oxfordshire, OX13 5HR (GB)**

(74) Representative: **Carridge, Andrew Edward
Reddie & Grose LLP
The White Chapel Building
10 Whitechapel High Street
London E1 8QS (GB)**

(56) References cited:
**WO-A1-2008/049251      US-A- 4 576 817
US-A1- 2009 291 122**

• **Matoke Holdings Ltd: "STIN parent application", ,
19 November 2019 (2019-11-19), pages 1-8,
XP055809595, Retrieved from the Internet:
URL:https://register.epo.org/application?n
umber=EP16711333 [retrieved on 2021-06-01]**

Remarks:
The file contains technical information submitted after
the application was filed and not included in this
specification

(52) Cooperative Patent Classification (CPC): (Cont.)
A61L 2300/254; A61L 2300/404; A61L 2400/12

C-Sets
**A01N 63/14, A01N 63/50;**
**A61L 15/24, C08L 29/04;**
**A61L 15/24, C08L 39/04;**
**A61L 15/26, C08L 71/02**

**Description**

This invention relates to fibers, wound dressings and compositions for generating antimicrobial activity, and their use, particularly for wound healing.

Honey has been used for treatment of microbial infections since ancient times. In recent years there has been a resurgence of interest in the therapeutic efficacy of honey, particularly in the area of wound healing. Clinical trials have shown that honey is an effective broad-spectrum antimicrobial agent which is effective against common wound-infecting organisms, such as *Pseudomonas aeruginosa, Staphylococcus aureus, Candida albicans and Escherichia coli,* and is effective against antibiotic-resistant strains of bacteria. As a natural product, honey also offers an attractive alternative to drug-based treatments.

Many different types of honey have antimicrobial activity. This activity is attributed largely to osmolarity, pH, hydrogen peroxide production and the presence of phytochemical components. Manuka honey, which originates from the Manuka tree (*Leptospermum scoparium*), has been recognised to have superior antimicrobial activity, compared to most other honeys, due to the high levels of antibacterial phytochemical activity present in this type of honey. However, manuka honey is in relatively limited supply and cannot meet the demands of a global market.

In addition to its antimicrobial action, several other activities of honey are believed to assist in wound healing, particularly healing of chronic wounds. In particular, honey has an ability to autolytically debride and deodorise wounds. Debridement is the removal of dead, damaged or infected tissue to improve the healing potential of the remaining healthy tissue. Honey has also been reported to have anti-inflammatory properties, to be able to stimulate tissue growth, and to manage pain and minimise scarring.

Several honey-based wound care dressings are currently available on the market. Many of these use manuka honey, or other honey with a high level of non-peroxide antibacterial activity, because the acidity, catalase activity, and protein-digesting enzymes present in wound fluids can reduce the antibacterial effectiveness of hydrogen peroxide.

Currently marketed honey-based dressings include the Medihoney range of products, manufactured by Comvita/Derma Sciences, which contain manuka honey, and Activon, a pure manuka honey with no additives, manufactured by Advancis. Other honey-based dressings contain honey and one or more additional ingredients. These include Melladerm Plus, which is an antibacterial wound gel made by SanoMed. The gel contains honey from a multiflower mountain region in Bulgaria that has a naturally high glucose oxidase and phenolic content (the main phytochemical components in honey are phenolic compounds). During processing, the honey is not heated or irradiated because this is considered to destroy its healing properties, in particular the production of hydrogen peroxide by glucose oxidase. It is instead sterilized using an ozonation method, described in WO 2008/049578, in which ozone gas is bubbled through liquefied honey in an ozone-resistant container. However, ozone has been approved by the US Food and Drug Administration (FDA) as a sterilant only for reusable medical devices, and so is not currently authorised by the US FDA for sterilisation of honey-based products for use in wound healing.

The Mesitran range is a further range of honey-enriched wound care products. L-Mesitran ointment, manufactured by Triticum, contains 48% medical grade honey, and several other components, including lanolin, sunflower oil, cod liver oil, marigold, Aloe vera, Vitamins C and E, and zinc oxide.

The difference in antimicrobial potency among honeys can be more than one hundred-fold, depending on the geographical, seasonal and botanical source of the honey, as well as the harvesting, processing and storage conditions. Honey-based dressings, therefore, have varying antimicrobial efficacy depending on the type of honey used. A study authored by Jenkins, Burton, and Cooper ("The determination of antimicrobial activity of three honey impregnated wound dressings by challenge test with EMRSA-15", Advancis) found that honey-based dressings containing Activon, Medihoney, or Mesitran product all had differing efficacies against EMRSA-15. The Activon impregnated dressing was the most effective, followed by the Medihoney dressing, with the Mesitran product having a much reduced effect.

There is, therefore, a need to reduce variability in the antimicrobial potency of honeys, and to improve the antimicrobial activity of honeys with low antimicrobial potency. It is also desirable to provide effective honey-based wound care products that do not rely on use of honeys with high levels of phytochemical components. It is also desirable to provide effective honey-based wound care products that have not been sterilised by ozonation.

Several other anti-bacterial wound care dressings are available that do not include honey. These include silver-containing dressings, such as ConvaTec's Aquacel® Ag dressing, which releases ionic silver in a controlled manner as wound exudate is absorbed into the dressing. However, silver-resistant organisms, such as *Staphylococcus aureus*, *Pseudomonas aeruginosa* and *enterococci*, have been reported, and may contribute to indolence in wound healing.

Povidone-iodine (PVP-I) is a stable chemical complex of polyvinylpyrrolidone (povidone, PVP) and elemental iodine. It is a broad spectrum antiseptic for topical application in the treatment and prevention of infection in wounds. It has been demonstrated that bacteria do not develop resistance to PVP-I. Since 1994 PVP-I has been approved by the US FDA for the first aid treatment of small, acute wounds. However, there has been some controversy over its safety and efficacy, and it was not recommended for use with pressure ulcers by the US Department of Health & Human Services.

Chlorhexidine has rapid, bactericidal activity against a wide spectrum of non-sporing bacteria. Antibacterial activity against *Staphylococcus aureus*, *Pseudomonas aeruginosa* and a range of clinical isolates has been documented. However, *Methicillin-resistant Staphylococcus aureus* (MRSA) has been observed to be resistant to chlorhexidine. In the UK, the Medicines and Healthcare Products Regulatory Agency (MHRA) has also issued a patient safety alert on the risk of anaphylactic reactions from the use of medical devices and medicinal products containing chlorhexidine.

There is also a need, therefore, to provide anti-microbial wound care products with broad-spectrum activity that are non-toxic, and which can be used for the treatment of chronic wounds.

The applicant has appreciated that the antimicrobial activity of honey depends on a delicate inter-relationship between natural inhibitors and activators, and has developed compositions in which the natural antimicrobial activity of honey can be released with reduced variability between different types and harvests of honey, and in which the antimicrobial properties of honeys with poor antimicrobial potency can be improved. The applicant has also found that such compositions have remarkable wound healing properties, even following sterilisation by exposure to gamma irradiation. The applicant has appreciated that these findings also have application to other natural substances.

The Applicant has found that the antimicrobial potency of the compositions can be precisely enhanced and controlled over a wide range. This makes it possible to provide compositions with antimicrobial potency that is most suited to the intended use.

In the field of wound healing, treatment of chronic wounds poses particular challenges. Chronic wounds are those wounds which fail to progress as expected through the typical healing processes in a timely manner. A chronic wound has been defined as a wound that has been in existence for more than three weeks, or that has failed to proceed through an orderly and timely process to produce anatomic and functional integrity or to proceed through the repair process without establishing a sustained and functional result (Lazarus et al, Arch Dermatol. 1994;130(4):489-493).

Chronic wounds are a significant health problem (Cowan et al, Ulcers 2013, Article ID 487024). Health care costs related to the management and treatment of chronic wounds in the USA has been reported to exceed $20 billion annually. The treatment and management of non-healing wounds is challenging. Traditionally, basic wound care has consisted of surgical debridement, manual irrigation, moisture retentive dressings, and topical and/or systemic antimicrobial therapy. Although there has been tremendous progress in the science of wound healing, the prevalence and incidence of chronic wounds and their associated complications continue to escalate.

The presence and complexity of bacterial biofilms in chronic wounds have recently been recognized as key aspects of non-healing wounds. Bacterial biofilms are sessile colonies of polymicrobial organisms (bacterial, fungal, and possibly, viral), which are often symbiotic. These biofilm colonies produce a protective coating to protect the colonies from host defenses. The character of this protective substance unique to biofilms is dynamic, and the production of its components seems to be triggered by hostile environments in the wound bed (such as the presence of topical antibiotics). Biofilms have been shown to have survival and defense mechanisms that inhibit the healing aspects of inflammatory cells, resist antibiotics (topical and systemic) and other therapies, and initiate cell-to-cell communication pathways (quorum sensing), which facilitate new biofilm growth, resulting in recalcitrant non-healing wounds.

A bacterial biofilm is characterized as an aggregated bacteria attached to a surface or formed at a surface interface and organized as a complex community embedded in a self-secreted extracellular polymeric substance (EPS). These dynamic bacterial communities may consist predominately of single bacterial or fungal species or, more commonly, may be polymicrobial, containing multiple diverse species that are continuously changing.

All biofilms, regardless of their location, share several common features. These include the synthesis of an extracellular polymeric matrix that holds the bacterial cells together, and an increase in resistance to killing by host defenses and antimicrobial agents compared with the resistance exhibited by free-living or 'planktonic' cells. The inherent protective nature of the biofilm colony makes most biofilm-associated infections difficult or impossible to eradicate.

Biofilm development can be divided into three distinct stages (Kaplan, J Dent Res 89(3) 2010: 205-218): attachment of cells to a surface, growth of the cells into a sessile biofilm colony, and detachment of cells from the colony into the surrounding medium. The initial, reversible interaction between a bacterial cell and a surface is mediated by non-specific Lifshitz-van der Waals, Lewis acid-base, and electrostatic forces. This transient attachment is reinforced by host- and tissue-specific adhesins that are located on the bacterial cell surface or on cellular appendages such as pili and fimbriae. This results in the irreversible attachment of the bacterial cell to the surface.

The second stage of biofilm development involves the multiplication of bacteria on the surface and the concomitant synthesis of an extracellular polymeric matrix. The matrix holds the bacterial cells together in a mass and firmly attaches the bacterial mass to the underlying surface. Some examples of polymeric biofilm matrix components include glucan polysaccharides, proteinaceous fimbriae, and extracellular, double-stranded DNA. In addition to providing a structural 'scaffold' for the biofilm colony, the matrix also contributes to biofilm-mediated antimicrobial resistance, either by acting as a diffusion barrier, or by binding directly to antimicrobial agents and preventing their access to the biofilm cells.

Continued growth of bacterial cells on a surface leads to the development of mature biofilm colonies containing millions of tightly packed cells gathered into pillar- and mushroom-shaped masses that project outward into the surrounding medium for hundreds of microns. These structures are interspersed with fluid-filled channels which act as a primitive circulatory system, allowing for the exchange of nutrients and waste products with the bulk fluid phase. In addition, masses of biofilm cells often contain demarcated internal spaces that are devoid of cells. Thus, mature biofilm colonies are complex, highly differentiated structures. Numerous microenvironments that differ with respect to pH, oxygen concentration, nutrient availability, and cell density exist within the biofilm colony. This results in a great deal of heterogeneity in metabolic and reproductive activity among cells located in different parts of the colony. Metabolically inactive cells located in the interior of the colony may be resistant to the actions of antimicrobial agents that target actively growing cells.

The final stage of biofilm development is the detachment of cells from the biofilm colony and their dispersal ('seeding') into the environment. This is an essential stage of the biofilm life cycle that contributes to biological dispersal, bacterial survival, and disease transmission. Like other stages of biofilm development, dispersal can be a complex process that involves numerous environmental signals, signal transduction pathways, and effectors. No single mechanism of biofilm dispersal is utilized by all bacteria.

Biofilms have been identified on various surfaces of the body including the teeth (plaque), endocardium, gastrointestinal and genitourinary mucosa, and nasal epithelium, as well as foreign objects such as orthopedic prosthetics and invasive catheters.

Evidence suggests that biofilms are strongly associated with impaired wound healing in chronic skin wounds. Wound biofilms trigger a chronic inflammatory response resulting in accumulation of neutrophils and macrophages surrounding biofilms. The neutrophils and macrophages secrete high levels of reactive oxygen species (ROS) that affect the biofilm and the surrounding tissues. Inflammatory cells also secrete high levels of proteases (matrix metalloproteinases and elastase) that can help break down the attachments between biofilms and the affected tissue, dislodging the biofilms from the tissue. However, the ROS and proteases also have the capacity to damage the normal surrounding tissue, proteins, immune cells, and tissue cells, delaying healing.

In vulnerable tissue, biofilms are created by planktonic bacteria attaching and forming a protective community before they are killed by the patient's immune system, antibiotics, or by debridement. Several conditions which impair the immune system or reduce the effectiveness of antibiotic drugs encourage the development and spread of biofilms in wounds. These include ischemia or necrosis of tissues, nutritional deficits or compromise, and comorbidities that impair the body's immune function, such as HIV, diabetes, major physical trauma, radiation treatment, or treatment with immune-suppressing drugs.

It has been suggested that the processes employed by biofilms include molecular mechanisms which enable bacteria to attach to host cells and inject proteins to reorganize host cellular pathways. For some bacterial species, the injected bacterial proteins reorganize the host cellular cytoskeleton and prevent migration and mitosis, and inhibit apoptosis. As bacteria begin to form a biofilm, their molecular mechanisms may attract other bacteria to form a sustainable polymicrobial system. A biofilm colony is thought to possess an expanded diverse gene pool representing numerous species of bacteria. Long-term biofilm survival is often directly related to the genetic diversity of the biofilms, resulting in chronic infections that become recalcitrant to treatment. Survival of a bacterial biofilm requires gene expression to ensure attachment to the host, cellular senescence of the host to prevent shedding and to cause local inflammation, and stimulation of the production of plasma in the wound bed to nourish the biofilm colony.

Microorganisms that have the ability to form biofilms also possess quorum-sensing molecules to direct the focus and organization of the biofilm. Directed secretion of molecules and organization of the colonies in biofilms maximize the availability of nutrients and other essential molecules while minimizing the opposing effects of waste products, toxins of competitors, and other environmental hazards on the biofilms. Polymicrobial biofilms likely incorporate quorum-sensing molecules that can regulate pathways and also perform bidirectional signaling. Biofilm organisms have the ability to sense and communicate with many quorum-sensing pathways.

Biofilms have numerous defenses and can be resistant to treatment, limiting the effectiveness of antibiotics. Antibiotics and antiseptics kill single bacteria very easily, but the biofilm barrier blocks most antibiotics and antiseptics from reaching the bacteria, particularly towards the center of the wound matrix. Wound biofilms are resistant to antibodies, antibiotics, disinfectants, and phagocytic inflammatory cells.

There is, therefore, a need to provide effective therapies to prevent and treat microbial infections that include biofilms or microbes capable of forming biofilms, particularly in chronic wounds, such as chronic skin and burn wounds.

In the treatment of wounds, wound dressings can be used to protect wounds and can provide a source of active substances, such as antimicrobial substances, for the treatment of the wound. Wound dressings containing fibers, such as nanofibers produced by electrospinning, can be particularly advantageous as a result of their high-surface area, high porosity with a very small pore size, and the ability to load drugs or other active molecules into the fibers. Examples of wound dressings comprising nanofibers and active substances, and methods of their production, are described in WO 2008/049251 and WO 2011/059497.

The applicant has appreciated the beneficial effects of reactive oxygen species, such as hydrogen peroxide, in applications such as wound healing and preventing infection. In particular, the applicant has appreciated the benefit of generating reactive oxygen species over an extended period of time. The applicant has manufactured dressings containing fibers, such as electrospun fibers, which can generate reactive oxygen species and may thus provide a convenient and effective treatment for wounds.

In its broadest sense, the invention concerns a sterile liquid or gel composition, comprising an enzyme that is able to convert a substrate to release hydrogen peroxide, and a substance that includes a substrate for the enzyme, wherein the composition comprises less than 10% by weight of water, and wherein the composition does not comprise honey or any other unrefined natural substance.

Also disclosed but not according o the invention is a a fiber comprising an enzyme that is able to convert a substrate to release hydrogen peroxide, and an unrefined natural substance that includes a substrate for the enzyme, wherein the enzyme is additional to any enzyme activity able to convert the substrate to release hydrogen peroxide that may be present in the unrefined natural substance.

In some embodiments, the fiber does not include sufficient free water to allow the enzyme to convert the substrate. This may be particularly useful where it is desired to store the fiber for long periods before use. The fiber may be a storage-stable fiber. In other embodiments, the fiber does include sufficient free water to allow the enzyme to convert the substrate.

Optionally, the substance does not comprise catalase activity.

According to the invention, there is also provided a wound dressing comprising one or more fibers of the invention.

Preferably the wound dressing is, or comprises, a fibrous or nanofibrous mat. The wound dressing may comprise woven fibers. In some embodiments, the dressing may comprise a substrate on to which the fibers are applied. Suitable substrates may include gauzes, bandages, tissues, films, gels, foams, hydrocolloids, alginates (such as AMS alginate foam or spun-bond alginate dressing), hydrogels, or polysaccharide pastes, granules, beads. The substrate may be foil, polypropylene or Cyrex®. The wound dressing may comprise a collagen or collagen-glycosaminoglycan matrix.

In some embodiments, the dressing may only comprise one or more fibers of the invention. Consequently, in some embodiments, the dressing may not comprise a substrate on to which the fibers are applied.

According to the invention, there is also provided a fibrous mat comprising one or more fibers of the invention. Preferably, the or each fiber is woven.

Preferably, the fiber is a nanofiber. Use of the term "nanofiber" herein refers to a fiber with a diameter less than or equal to 1000 nanometres (nm). However, the fiber could be a microfiber. The fiber may have a diameter less than or equal to 1 millimetre, less than or equal to 500 micrometres ($\mu$m), less than or equal to 50 $\mu$m or less than or equal to 10 $\mu$m. In some embodiments, the fiber may have a diameter less than or equal to 100 nm.

In preferred embodiments, the fiber is an electrospun fiber. This means that the fiber is obtained or formed by electrospinning.

Typically, electrospinning is carried out using an apparatus having three major components: a high voltage power supply, a spinneret (such as a metallic needle), and a collector (such as a grounded conductor). The spinneret is connected to a syringe containing an electrospinnable composition. With the use of a syringe pump, the composition can be fed through the spinneret at a constant and controllable rate. When a high voltage is applied to the composition, a drop of the composition at the nozzle of the spinneret will become highly electrified and the induced charges are evenly distributed over the surface. As a result, the drop experiences two major types of electrostatic forces: the electrostatic repulsion between the surface charges; and the Coulombic force exerted by the external electric field. Under the action of these electrostatic interactions, the liquid drop will be distorted into a Taylor cone. Once the strength of the electric field has surpassed a threshold value, the electrostatic forces can overcome the surface tension of the composition and thus force the ejection of a jet from the nozzle. This electrified jet undergoes a stretching and whipping process, leading to formation of a long, thin thread. As the composition is continually elongated and dries, its diameter can be greatly reduced from hundreds of micrometres to as small as tens of nanometres. In a typical setup, the applied voltage is 30 kV, the distance between the spinneret and collector is 20 cm and the flow rate of the solution is 1 mL/hour. However, it will be appreciated that these parameters can be varied to optimise results, or to vary properties of the fibers produced. It will also be appreciated that electrospinning can take place using variations or modifications the typical apparatus, described above. For example, different types of electrospinning apparatus are available, such as the Spraybase® and the El Marco NanoSpider™.

According to preferred embodiments of the invention, the fiber or wound dressing is sterile. Sterilisation may occur by any suitable means. The Applicant has found that compositions suitable for forming fibers or dressings of the invention retain glucose oxidase activity (and, therefore, the ability to release hydrogen peroxide) following sterilisation by exposure to gamma irradiation. Thus, the fiber or wound dressing is preferably sterilised by exposure to gamma irradiation. A suitable level of gamma irradiation is 10-70 kGy, preferably 25-70 kGy, more preferably 35-70 kGy.

There is also provided, according to the invention, a method of sterilising a fiber or wound dressing of the invention, which comprises exposing it to gamma irradiation.

Advantageously, hydrogen peroxide may be released for a sustained period from the fiber or dressing. This may depend on the amount of substrate present or the amount or activity of the enzyme. It will be appreciated that the amount of substrate and/or the amount or activity of enzyme may be selected to provide for release of a relatively high level of hydrogen peroxide for a short period, or for release of a lower level of hydrogen peroxide for a longer period, following contact with water. The fiber or dressing may comprise sufficient enzyme and substrate to provide for sustained release of hydrogen peroxide for a period of at least twenty four hours, more preferably at least forty eight hours. Preferably, there is sustained release of hydrogen peroxide at a level of less than 2 mmol/litre for a period of at least twenty four hours.

If the fiber or dressing does not contain sufficient free water to allow the enzyme to convert the substrate, release of hydrogen peroxide may occur when the fiber or dressing is contacted (or mixed) with water. The fiber or dressing may thus comprise sufficient enzyme and substrate to provide for sustained release of hydrogen peroxide for a period of at least twenty four hours, more preferably at least forty eight hours, following contact with sufficient water to allow the enzyme to convert the substrate. Preferably, there is sustained release of hydrogen peroxide at a level of less than 2 mmol/litre for a period of at least twenty four hours following contact with sufficient water to allow the enzyme to convert the substrate.

In other embodiments, the fiber or dressing may comprise sufficient enzyme and substrate to provide for sustained release of at least 0.1, 0.5, 1 or 1.5 mmol/litre hydrogen peroxide for a period of at least 24 hours, more preferably 48 hours.

The invention also provides an electrospinnable composition comprising an enzyme that is able to convert a substrate to release hydrogen peroxide, and an unrefined natural substance that includes a substrate for the enzyme, wherein the enzyme is additional to any enzyme activity able to convert the substrate to release hydrogen peroxide that may be present in the unrefined natural substance.

According to the invention, there is also provided a method of producing a fiber, comprising electrospinning an electrospinnable composition of the invention.

Preferably, the electrospinnable composition is a solution. The solvent in the solution may be, or may comprise, water. The solvent may comprise an aqueous and/or non-aqueous solvent, such as an organic solvent.

The fiber or the electrospinnable composition may comprise one or more electrospinnable components. Any electrospinnable component which facilitates formation of a fiber or dressing according to the invention, may be suitable. Preferably, the one or more electrospinnable component is an electrospinnable polymer. In some embodiments, the polymer may be a synthetic polymer. In some embodiments, the polymer is a natural polymer. In some embodiments, the electrospinnable polymer is selected from polyethylene oxide, polyvinyl alcohol and polyvinylpyrrolidone. Other polymers may include polycaprolactone or phosphino-carboxylic acid (PCA).

The electrospinnable component is preferably biocompatible. Optionally, the electrospinnable component is water soluble. The electrospinnable component may be soluble in an organic, or non-aqueous, solvent. The electrospinnable component may be soluble in a mixture of an aqueous and non-aqueous solvent. Suitable non-aqueous solvents may be, or may comprise, glycerol, dimethyl sulphoxide, ethylene glycol or propylene glycol.

The electrospinnable composition may comprise up to 50%, 25%, 10% or 5% by weight of the electrospinnable component. Optionally, the electrospinnable component may be from 1 to 50% by weight of the composition. The electrospinnable composition may comprise up to 30%, 20% or 10% by weight of the unrefined natural substance.

The electrospinnable composition may be formed by mixing a polymer solution with an unrefined natural substance. For example, the polymer solution may comprise up to 50%, 25%, 10% or 5%, by weight, of an electrospinnable polymer. The electrospinnable composition may be formed by mixing the polymer solution with the unrefined natural substance in a weight ratio (polymer solution:unrefined natural substance) of 90%:10% to 60%:40%. For example, the electrospinnable composition may be formed from a mixture of 80% by weight polymer solution and 20% by weight unrefined natural substance.

It will be appreciated that the relative amounts of the electrospinnable component, the unrefined natural substance and the solvent may be varied to alter the properties of the fibers.

A fiber that has been formed from the electrospinnable composition may comprise up to 80% by weight of the unrefined natural substance. The fiber may comprise 20% or more, by weight, of the electrospinnable component.

Also disclosed but not according to the invention as claimed is the use of a composition for generating antimicrobial activity in the manufacture of a fiber or dressing according to the invention, the composition comprising an enzyme that is able to convert a substrate to release hydrogen peroxide, and a substance that includes a substrate for the enzyme, in which the enzyme is additional to any enzyme activity able to convert the substrate to release hydrogen peroxide that may be present in the substance. Preferably, the composition is a storage-stable composition. In some embodiments, the composition does not include sufficient free water to allow the enzyme to convert the substrate. Optionally, the substance does not comprise catalase activity. The enzyme may be a purified enzyme.

Also disclosed but not according to the invention is a method for producing an antimicrobial composition comprising contacting a fiber of the invention with sufficient water to allow the enzyme to convert the substrate in the unrefined natural substance and release hydrogen peroxide. The invention also provides an antimicrobial composition obtained, or obtainable by this method.

Preferably, the antimicrobial composition is a solution. For example, the fiber or dressing may dissolve on contact with a solvent. The antimicrobial composition may be a suspension. The antimicrobial composition may be formed by applying the fiber or wound dressing to a wound. In some embodiments, wound exudate may provide sufficient water to allow the enzyme to convert the substrate in the unrefined natural substance and release hydrogen peroxide.

In some embodiments, fibers may be formed by first forming a fiber (for example by electrospinning a solution containing a polymer) and then coating the fiber with a composition, the composition comprising an enzyme that is able to convert a substrate to release hydrogen peroxide; and a substance that includes a substrate for the enzyme and wherein the composition does not include honey or any other unrefined natural substance. Coating may be achieved by a process such as electrospraying or by immersing in the composition. This may avoid the need to electrospin a composition comprising the enzyme and substrate.

The invention encompasses a composition for generating antimicrobial activity, comprising an enzyme that is able to convert a substrate to release hydrogen peroxide; and a substance that includes a substrate for the enzyme.

The composition may comprise a polymer.

According to the invention, there is provided a composition comprising an enzyme that is able to convert a substrate to release hydrogen peroxide; a substance that includes a substrate for the enzyme; and a polymer.

In some embodiments, the substance includes a purified substrate for the enzyme.

According to the invention, there is provided a composition comprising an enzyme that is able to convert a substrate to release hydrogen peroxide; and a substance that includes a purified substrate for the enzyme.

According to the invention, there is provided, a composition for generating anti-microbial activity, wherein the composition comprises an enzyme that is able to convert a substrate to release hydrogen peroxide, a substance that includes a purified substrate for the enzyme, and a polymer.

The enzyme of the composition is additional (i.e. added as a result of human intervention) to any enzyme activity able to convert the substrate to release hydrogen peroxide (referred to herein as "substrate conversion activity") that may be present in the substance.

The composition does not comprise an unrefined natural substance. In some embodiments, however, the substance that includes a substrate for the enzyme may be an unrefined natural substance.

The composition may be an electrospinnable composition. Alternatively or additionally, the composition may be a sprayable or atomisable composition. For example, the composition may have rheological properties that permit spraying or atomisation. In some embodiments, the composition is not an electrospinnable composition.

The composition may be an injectable composition. For example, the composition may have rheological properties that permit application through a syringe.

Without the addition of the polymer to the composition, an unrefined natural substance, such as honey, may not have the requisite rheological properties to permit effective spraying or injection. For example, the unrefined natural substance may be too viscous.

The polymer in the composition may be any medically acceptable polymer, such as any Food and Drug Administration-approved (FDA-approved) polymer.

In some embodiments, the polymer may be a synthetic polymer. In some embodiments, the polymer is a natural polymer.

Optionally, the polymer is water soluble. The polymer may be soluble in an organic, or non-aqueous, solvent. The polymer may be soluble in a mixture of an aqueous and non-aqueous solvent. The polymer may be biodegradable or bioerodable. The polymer may be a co-polymer.

In some embodiments, the polymer is selected from polyethylene oxide (or polyethylene glycol), polyvinyl alcohol and polyvinylpyrrolidone.

Other polymers may include poly(lactic-co-glycolic acid), polyglycolic acid, polylactic acid, polycaprolactone or polymeric surfactants. Another suitable polymer may be phosphino-carboxylic acid (PCA).

Further polymers may include polysaccharides such as cellulose (which includes derivatives such as hydroxypropyl methyl cellulose and hydroxypropyl cellulose), alginate, gelatin or cyclodextrins. Suitable polymers may also include chitosan or hyaluronic acid.

The composition may comprise up to 50%, 25%, 10% or 5% by weight of the polymer. For example, the composition may comprise from 0.5 to 3% by weight of the polymer. Optionally, the polymer may be from 0.5 to 50% by weight of the composition. The composition may comprise up to 30%, 20% or 10% by weight of the unrefined natural substance.

The composition may be formed by mixing a polymer solution with the substance (which may be an unrefined natural substance or a substance that comprises a purified substrate). For example, the polymer solution may comprise up to 50%, 25%, 10% or 5%, by weight, of the polymer. The composition may be formed by mixing the polymer solution with the substance (which may be an unrefined natural substance or a substance that comprises a purified substrate) in a weight ratio (polymer solution: substance) of 90%:10% to 60%:40%. For example, the composition may be formed from a mixture of 80% by weight polymer solution and 20% by weight substance.

Preferably, the composition is a storage-stable composition. In some embodiments, the composition does not include sufficient free water to allow the enzyme to convert the substrate. In some embodiments, the composition may comprise sufficient free water to allow the enzyme to convert the substrate.

Hydrogen peroxide may be released for a sustained period, depending on the amount of substrate present in the composition, and the activity of the enzyme. The release of hydrogen peroxide may occur, or be initiated by, dilution of the composition. It will be appreciated that the amount of substrate and/or the activity of enzyme in the composition may be selected to provide for release of a relatively high level of hydrogen peroxide for a short period, or for release of a lower level of hydrogen peroxide for a longer period. Suitably the composition provides for sustained release of hydrogen peroxide for a period of at least twenty four hours, more preferably at least forty eight hours, preferably following dilution of the composition. Suitably compositions of the invention provide for sustained release of hydrogen peroxide at a level of less than 2 mmol/litre for a period of at least twenty four hours (or at least 48 hours), preferably following dilution of the composition. Compositions of the invention may provide for a sustained release of at least 0.1, 0.5, 1 or 1.5 mm/litre hydrogen peroxide for a period of at least twenty four hours, more preferably forty eight hours, optionally following dilution of the composition.

The composition may comprise non-aqueous solvents.

The viscosity of the composition may be adjusted according to the desired application of the composition. For example, viscosity may be adjusted by varying the type and/or amount of polymer in the composition or by adjusting the solvent content of the composition.

The composition may be a gel. Preferably, the composition is a liquid.

According to the invention, there is provided a device for delivering a composition to a patient, the device comprising a composition of the invention.

The device may be a spraying or atomising device, such as a pump-action spray or an aerosol spray.

The device may be for external use, such as for applying the composition to a patient's skin.

The device may be for internal application to a patient. For example, the device may be an inhaler or nebuliser for administering the composition to the patient's respiratory tract. The device may be a douche. The device may be a device for injecting the composition into the patient, such as a syringe.

According to the invention there is provided a kit, the kit comprising: i) a composition of the invention; and ii) a device for delivering a composition to a patient. The composition may be separate from the device.

The compositions may be for prophylactic applications, or for treatment of a condition associated with microbial infection.

For example, there may be provided a prophylactic spray for pre-operation skin sterilisation. Such a spray may be comprise a low-viscosity composition. When applied, the composition may be non-sticky. The spray may allow delivery of a reactive oxygen laminar to the skin. The laminar may continue to deliver reactive oxygen over an extended period of time which could sterilise skin prior to surgical incision and provide prophylactic protection during and after the surgical procedure.

There may be provided an atomising spray for inhalation. An atomising spray for inhalation may be used to treat infections in a patient's airways. An atomising spray may require a composition with low viscosity.

A spray could be used to apply to burn or wound tissue prior to applying a dressing. This may require a more viscous composition.

A spray could be applied internally to a patient, post-surgery, to prevent organ infection and septicaemia. This may require a more viscous composition.

The composition may be diluted with water prior to being used with a device, such as a douche.

Preferably, the composition is substantially homogenous.

Also disclosed is a method of applying or administering a composition according to the invention, to a patient, wherein the method comprises spraying the composition, injecting the composition, inhaling the composition or applying the composition to a patient's nasal cavity or paranasal sinus.

According to the invention, there is provided a composition according to the invention for use as a medicament. The composition may be administered or applied by spraying, by injection, by inhalation, or applying the composition to a patient's nasal cavity or paranasal sinus. The composition may be administered or applied externally or internally, to a patient.

In some embodiments, the composition is not, or does not comprise, a fiber or a wound dressing.

In some embodiments, the composition does not comprise an electrospinnable component. In some embodiments, the composition does not comprise an electrospinnable polymer.

The composition is not, or does not comprise, the following: an electrospinnable composition comprising an enzyme that is able to convert a substrate to release hydrogen peroxide, and an unrefined natural substance that includes a substrate for the enzyme, wherein the enzyme is additional to any enzyme activity able to convert the substrate to release hydrogen peroxide that may be present in the unrefined natural substance.

The composition is not, or does not comprise, the following: a composition obtained or obtainable by contacting a fiber of the invention with sufficient water to allow the enzyme to convert the substrate in the unrefined natural substance and release hydrogen peroxide.

The composition is not, or does not comprise, an aqueous solution containing 4% by weight polyethylene oxide that has been mixed with Surgihoney in a weight ratio of 80% PEO solution to 20% Surgihoney.

The composition is not, or does not comprise, a composition containing 1 % by weight polyethylene oxide, 79% by weight deionised water and 20% by weight Surgihoney.

The composition is not, or does not comprise, the following:

Aqueous Spray

25 ml plastic bottle containing:

| | |
|---|---|
| Active honey | 10 g |
| Triton CF | 0.1 g |
| Maltodextrin, or corn Starch | 1 g |

wherein "Active honey" is honey with added glucose oxidase.

The composition is not, or does not comprise, the following:

Non-Aqueous spray

| | |
|---|---|
| Active honey | 70% |
| Proplyene Glycol | 30% |

The composition is not, or does not comprise, the following:

Powder

Active honey
Maltodextrin
Ibuprofen
Microcrystalline Cellulose (CMC)
Polyvinyl Pyrrolidone (PVP)

The composition is not, or does not comprise, the following:

Alcoholic Gel

| | |
|---|---|
| Carbopol 940 | 0.3 % |
| Triethanolamine | 0.4 % (needed for pH and stability control) |
| Active honey (5+) | 65 % |
| Ethanol | 25.0 % |
| water qs | |

The composition is not, or does not comprise the following:

Fungal Nail Treatment
Plaster with Active Honey (5+) in a foam well
Hydroxypropylcellulose
Glycerol
Isopropyl Alcohol

Citric Acid
Monohydrate

The composition is not, or does not comprise the following:

Cream

| | |
|---|---|
| Honey | 15% |
| Carbomer | 2.63% |
| Dimethicone | 0.13% |
| Disodium Lauryl Sulphosuccinate | 0.05% |
| Disodium Edetate | 0.13% |
| Glycerol | 5.26% |
| Silica Colloidal Hydrated | 0.33% |
| Poloxamer | 0.26% |
| Sodium Hydroxide | 0.41% |
| Purified Water | 85.03% |

In some embodiments, the composition does not comprise one or more of the following: maltodextrin, microcrystalline cellulose, Polyvinyl Pyrrolidone, carbomer, polaxomer, hydroxypropyl cellulose, carbopol 940.

Preferably the enzyme is a purified enzyme. The term "purified enzyme" is used herein to include an enzyme preparation in which the enzyme has been separated from at least some of the impurities originally present when the enzyme was produced. Preferably impurities that have been removed or reduced include those that would otherwise interfere with the ability of the enzyme to convert the substrate to release hydrogen peroxide.

It may not always be necessary or desirable that the purified enzyme is at a high level of purity provided that the enzyme is able to convert the substrate to release hydrogen peroxide. In some circumstances, it may be desirable to used a relatively crude enzyme preparation. Examples of suitable purity levels include at least 10%, 20%, 30%, 40%, 50%, 60%, 70%, 80%, or 90% pure.

It is preferred, however, that the amount of any catalase that may originally have been present when the enzyme was produced has been reduced. The enzyme may have been produced by recombinant or non-recombinant means, and may be a recombinant or non-recombinant enzyme. The enzyme may be purified from a microbial source, preferably from a non-genetically modified microbe.

The level of purity of the enzyme may be selected as appropriate depending on the intended use of the fiber, or composition. For medical use, a medical grade or medical device grade of purity should be used.

Catalase is an enzyme that catalyses the decomposition of hydrogen peroxide to water and oxygen. The use of a substance that lacks catalase activity means that there is no variability in the amount of this activity between similar substances from different sources, or from different harvests from the same source. This reduces the variability in antimicrobial activity that can be generated from such substances. Alternatively, if the substance does include catalase activity, and it is not possible or desirable to inactivate the catalase activity in the substance prior to contacting the substance with the enzyme, then sufficient enzyme may be used such that the effect of catalase activity on the hydrogen peroxide that can be generated from the substance is reduced. This also reduces the variability in antimicrobial activity that can be generated from the substance. In some embodiments it is preferred that the substance lacks catalase activity.

Catalase is present in many plants and animals. Catalase activity may be removed during processing or extraction of the substance, or inactivated before use of the substance in the invention. Catalase activity may be heat inactivated, for example by pasteurisation. A suitable temperature for heat inactivation of catalase activity is at least 60°C, 70°C, or 80°C, preferably for at least 2 minutes.

Preferably, the fiber, dressing or composition of the invention is storage stable. Compositions suitable for (or used for) forming fibers or dressings of the invention may be storage stable. The term "storage-stable" is used herein to mean that the fiber, dressing or composition can be stored at ambient temperature for at least several days, preferably at least a week, more preferably at least one or two months, whilst retaining the ability to generate antimicrobial activity following contacting the fiber, dressing or composition with water. A preferred storage temperature is below 37°C, preferably 20-25°C. Preferably, storage occurs away from exposure to light.

Hydrogen peroxide is generally unstable at ambient temperature. The lack of sufficient free water may thus prevent the enzyme converting the substrate to release hydrogen peroxide, and help to maintain stability for extended periods at ambient temperature. A storage-stable fiber, dressing or composition of the invention, or a composition suitable for forming a fiber or dressing of the invention, may include some water provided there is not sufficient free water to allow the enzyme to convert the substrate. Suitable amounts of water will vary depending on the precise components of the fiber, dressing or composition. However, typically, a storage-stable fiber, dressing or composition may comprise less than 20% total water content, for example, 10%-19%, water.

Fibers, dressings or compositions of the invention, such as storage-stable fibers, dressings or compositions, preferably do not include any detectable hydrogen peroxide. It may be said that they contain substantially no hydrogen peroxide. They may be formed, for example, by contacting the enzyme with the substrate in the absence of sufficient free water to allow the enzyme to convert the substrate.

The enzyme present in the fiber, dressing or composition is additional (i.e. added as a result of human intervention) to any enzyme activity able to convert the substrate to release hydrogen peroxide (referred to herein as "substrate conversion activity") that may be present in the substance, i.e. the fiber, dressing or composition of the invention comprises the substance and added enzyme.

It will be appreciated that there should be sufficient enzyme present to convert the substrate and form hydrogen peroxide as needed following or contact with water, in embodiments in which there is not sufficient free water to allow the enzyme to convert the substrate.

In view of the importance of generation of hydrogen peroxide in the presence of sufficient water, it will be appreciated that the fiber, dressing or composition should not contain any added peroxidase.

In some embodiments, compositions, fibers or dressings of the invention may contain sufficient free water to allow the enzyme to convert the substrate, In some embodiments, such compositions, fibers or dressings may be able to produce hydrogen peroxide for an extended period of time. For example, they may be capable of producing hydrogen peroxide at a level described herein (e.g. at least 0.1, 0.5, 1 or 1.5 mmol/litre hydrogen peroxide) for at least a year, preferably at least 3 years. In some compositions that have sufficient free water to allow the enzyme to convert the substrate, there may be a reduction in the level of hydrogen peroxide produced, over time. This may result from fermentation of the substrate by fungi. Fermentation may reduce the amount of the substrate available for hydrogen peroxide production. In some embodiments, fermentation my be reduced by sealing the composition, fiber or dressing in a container or sachet that prevents any fungal contamination and then treating the composition to kill fungi in the composition, fiber or dressing. For instance, this may be achieved by gamma irradiation.

Preferably the enzyme is an oxidoreductase enzyme. Examples of oxidoreductase enzymes that can convert a substrate to release hydrogen peroxide include glucose oxidase, hexose oxidase, cholesterol oxidase, galactose oxidase, pyranose oxidase, choline oxidase, pyruvate oxidase, glycollate oxidase, and amioacid oxidase. The corresponding substrates for these oxidoreductase enzymes are D-glucose, hexose, cholesterol, D-galactose, pyranose, choline, pyruvate, glycollate and aminoacid, respectively.

A mixture of one or more oxidoreductase enzymes and one or more substrates for the oxidoreductase enzymes may be present.

According to a preferred embodiment of the invention, the oxidoreductase enzyme is glucose oxidase and the substrate is D-glucose.

The substance may be any substance that includes a substrate for the enzyme. Preferably the substance lacks catalase activity. However, the composition does not comprise honey or any other unrefined natural substance. The term "unrefined" is used herein to refer to substances that have not been processed into a pure form. Unrefined substances include substances that may have been concentrated, for example by drying or boiling.

Preferably the substance includes one or more substrates from a natural source (termed herein a "natural substance"). Examples of natural substances include substances from a plant source, including from sap, roots, nectar, flowers, seeds, fruit, leaves, or shoots. However, the composition does not comprise any unrefined natural substance.

Preferably the substance comprises one or more of the following substrates: D-glucose, hexose, cholesterol, D-galactose, pyranose, choline, pyruvate, glycollate or aminoacid.

Preferably the substance is a sugar substance. The term "sugar substance" is used herein to mean any substance that includes one or more sugars. The term "sugar" is used herein to refer to a carbohydrate with the general formula $C_m(H_2O)_n$. Preferred sugars include monosaccharides, such as D-glucose, hexose, or D-galactose. Preferably the sugar substance includes one or more sugars from a natural source (termed herein a "natural sugar substance"). In an embodiment which is not according to the invention, the natural sugar substance is an unrefined natural sugar substance. Such an unrefined natural sugar substance may be (or be derived from) a natural sugar product. In an embodiment which is not according to the invention, the unrefined natural sugar product is a honey. In some embodiments which are not according to the invention, the honey is a honey that has been treated to remove or inactivate catalase activity. Alternatively, but also not according to the invention, the unrefined natural sugar substance may be a processed natural sugar, such as a syrup or an inverted syrup.

As discussed above, the substance itself may preferably lack an enzyme activity that is able to convert the substrate to release hydrogen peroxide (referred to as "substrate conversion activity"). Absence of substrate conversion activity from the substance has the advantage that there is then no variability in the amount of this activity between similar substances from different sources, or from different harvests from the same source. This may further reduce the variability in antimicrobial activity that can be generated from such substances. Substrate conversion activity is then provided only by the enzyme that is contacted with, or added to, the substance, and so the amount of substrate conversion activity present can be controlled.

Substrate conversion activity may be removed during processing or extraction of the substance, or inactivated before use of the substance in the invention. Substrate conversion activity may be inactivated by heat inactivation, for example by pasteurisation. A suitable temperature for heat inactivation of substrate conversion activity is at least 80°C, preferably for at least two minutes. An advantage of heat inactivation is that both catalase activity and substrate conversion activity can be inactivated in a single heat inactivation step.

In some embodiments of the invention, the substance is a processed, extracted, or refined substance (i.e. a substance in which impurities or unwanted elements have been removed by processing). Preferably impurities that have been removed or reduced include those that would otherwise interfere with the ability of the enzyme to convert the substrate to release hydrogen peroxide.

In some embodiments of the invention, the substance comprises a purified substrate for the enzyme. The term "purified substrate" is used herein to include a substrate preparation in which the substrate has been separated from at least some of the impurities originally present when the substrate was obtained or produced. The purified substrate may be obtained from a natural source or may be synthetically produced. The purified substrate may be a processed, extracted, or refined substrate (i.e. a substrate in which impurities or unwanted elements have been removed by processing).

It may not always be necessary or desirable that the purified substrate is at a high level of purity provided that the enzyme is able to convert the substrate to release hydrogen peroxide. In some circumstances, it may be desirable to used a relatively crude substrate preparation. Examples of suitable purity levels include at least 10%, 20%, 30%, 40%, 50%, 60%, 70%, 80%, 90%, 95%, or 99% pure. However, in some embodiments, it may be desirable that the purified substrate is a medical grade, medical device grade, or pharmaceutical grade substrate.

In particular embodiments, the purified substrate is or comprises a purified sugar substance. The purified sugar substance may be obtained from a natural source (for example a processed, extracted, or refined natural sugar substance), or be synthetically produced. The purified sugar substance may be at least 10%, 20%, 30%, 40%, 50%, 60%, 70%, 80%, 90%, 95%, or 99% pure. The purified sugar substance may be a medical grade, medical device grade, or pharmaceutical grade sugar substance,. The purified sugar substance may include one or more purified sugar substances, for example purified D-glucose, hexose, or D-galactose. For example the purified sugar substance may be medical grade, medical device grade, or pharmaceutical grade D-glucose, hexose, or D-galactose.

In particular embodiments, the enzyme and the substrate are purified, for example purified glucose oxidase and purified D-glucose, suitably medical grade, medical device grade, or pharmaceutical grade glucose oxidase and D-glucose. The fiber,dressing or composition of the invention may include an additional antimicrobial agent such as: an antibiotic, an antiviral agent, or an anti-fungal agent.

In preferred embodiments, the fiber, dressing or composition of the invention is medical grade or medical device grade or pharmaceutical grade.

As discussed above, the fiber or wound dressing may be sterile, and is preferably sterilised by exposure to gamma irradiation. Compositions of the invention may also be sterile, preferably sterilised by exposure to gamma irradiation. A suitable level of gamma irradiation may be 10-70 kGy, preferably 25-70 kGy, more preferably 35-70 kGy. Since ozone has not been authorised by the US FDA for sterilisation of honey-based products for use in wound healing, preferably sterilization does not occur by ozonation, and fibers, wound dressings or compositions of the invention preferably do not include ozone, or any components that have been subjected to sterilisation by ozonation. In particular, fibers, wound dressings or compositions of the invention should not comprise ozonized honey or ozonated oil.

Honey is a natural product made by honey bees using nectar from flowers. It is a saturated or super-saturated solution of sugars. Honey is defined in the Codex Alimentarius international food standard as "the natural sweet substance produced by honey bees from the nectar of plants or from secretions of living parts of plants or excretions of plant sucking insects on the living parts of plants, which the bees collect, transform by combining with specific substances of their own, deposit, dehydrate, store and leave in the honey comb to ripen and mature" (Revised Codex Standard for Honey, 2001).

Nectar typically includes approximately 14% simple sugars (w/w), 1% phenol compounds, and 85% water. The phenol compounds give the honey its taste, aroma and colour. In the warm conditions of the hive, typically 36°C, the nectar would very quickly ferment. To prevent this, the nectar is mixed with secretions, containing enzymes, from the salivary and hypopharyngeal glands of foraging bees. In the hive the nectar is passed from bee to bee and more secretions are added before it is stored in the cells of the hive. The amount of enzymes present varies with the age, diet and physiological stage of the bees (when a bee is a forager its glands produce more digestive enzymes), strength of the colony, temperature of the hive, and the nectar flow and its sugar content.

The enzymes added to nectar by bees include diastase, which catalyses the conversion of starch to dextrin and sugar, Invertase, which catalyses the conversion of sucrose to fructose and glucose, and glucose oxidase, which catalyses the conversion of glucose to hydrogen peroxide and gluconic acid. Low doses of hydrogen peroxide prevent the growth of yeasts that would quickly ferment the nectar. As the bees progressively dry the nectar to form honey, the gluconic acid makes the honey acidic (between pH 3.5 and 4.5). Water is effectively trapped to the sugar molecules in the honey and is not available for further chemical reactions. The amount of 'free' water in honey is measured as the water activity ($a_w$). The range of $a_w$ found in honey has been reported to be 0.47-0.70, with mean values of 0.562 and 0.589 (RCIEGG, M; BLANC, B, 1981, The water activity of honey and related sugar solutions. Lebensmittel-Wissenschaft und Technologie 14: 1-6). The $a_w$ of ripened honey is too low to support the growth of any species, with no fermentation occurring if the water content is below 17.1% (Molan, P. C. (1992). The antibacterial activity of honey: 1. The nature of the antibacterial activity. Bee World, 73(1), 5-28). The acidity of the honey and the lack of free water prevent the further risk of fermentation, and stop the glucose oxidase working. Honey also contains variable amounts of catalase originating from the nectar.

A typical chemical composition of blossom honey is:

Table 1

| Component | Blossom honey | |
|---|---|---|
| | Average (% w/w) | Min-Max (% w/w) |
| Water content | 17,2 | 15-20 |
| Fructose | 38,2 | 30-45 |
| Glucose | 31,3 | 24-40 |
| Sucrose | 0,7 | 0,1 -4,8 |
| Other disaccharides | 5 | |
| Total sugars | 79,7 | |
| Minerals | 0,2 | 0,1-0,5 |
| Amino acids, Proteins | 0,3 | 0,2 -0,8 |
| Acids | 0,5 | 0,2 -0,8 |
| pH | 3,9 | 3,5 -4,5 |

In addition, trace amounts of pollen are present, which can be used to identify the botanical origin of the honey, as well as the enzymes invertase, diastase, catalase, and glucose oxidase. There is also phytochemical component. This varies but is typically up to ~1%, depending on the source of the honey.

Once diluted, the glucose oxidase present in natural honey is able to convert glucose substrate in the diluted honey to release hydrogen peroxide. However, the variability in the content of honey (particularly in the content of glucose oxidase activity, glucose, and catalase activity) means that honeys from different sources, or different harvests of honey from the same source, can be very variable in their antimicrobial effectiveness.

Honey may be pasteurised. Pasteurisation of honey inactivates the catalase and glucose oxidase activity present in the honey. Optionally, the pasteurised honey may be filtered to remove any particles (such as wax particles and bee wings) that may be in the honey post harvest. To form a storage-stable composition which is suitable for making a fiber or dressing of the invention, a glucose oxidase may be contacted with the pasteurised honey once it has cooled to a temperature (suitably 35-40°C) that will not inactivate the added glucose oxidase and at which the honey remains sufficiently liquid to facilitate mixing with glucose oxidase.

Honey can be pasteurised at a temperature that is sufficient for the heat inactivation of catalase activity. A suitable minimum temperature is from $60^0$C to $80^0$C. This temperature should be maintained preferably for at least two minutes.

The control of the heat process may be important, since a by-product of heating honey is the formation of HMF (HydroxyMethylFurfuraldehyde) which is used as an indicator of heat and storage changes in honey. HMF is formed by the breakdown of fructose in the presence of acid. Heat increases the speed of this reaction. The increase in speed is exponential with increasing heat. For every degree that the honey is raised above $40^0$C, close to the normal hive ambient temperature, HMF increases rapidly. HMF is not a harmful product. Jams, Molasses, Golden Syrup etc. can have levels of HMF 10 to 100 times that of honey. However HMF levels are used as an indication of degradation of honey and under the Codex Alimentarius Standard 40 mg/l is the maximum permissible level in the EU for table honey.

To prevent the build up of HMF it is preferred that the honey is raised rapidly to temperature levels to inactivate the catalase and then the honey is brought quickly down in temperature to a maximum of between 40 and 45°C using a heat exchange mechanism.

No water is added during the process, and so the resulting composition does not include sufficient free water to allow the glucose oxidase to convert the glucose present to release hydrogen peroxide. A storage-stable composition, which may be used for forming fibers or dressings, may thus comprise: pasteurised honey, and added glucose oxidase. There may be no detectable hydrogen peroxide present. This composition, which is not according to the invention, may be stored at ambient temperature for at least several days.

Creamed honey is a honey that has been processed to control crystallization. Creamed honey contains a large number of small crystals, which prevent the formation of larger crystals that can occur in unprocessed honey. A method for producing creamed honey was described in U.S. Patent 1,987,893. In this process, raw honey is first pasteurised, then previously processed creamed honey is added to the pasteurized honey to produce a mixture of 10% creamed honey and 90% pasteurised honey. The mixture is then allowed to rest at a controlled temperature of 14°C. This method produces a batch of creamed honey in about one week. A seed batch can be made by allowing normal honey to crystallize and crushing the crystals to the desired size. Large scale producers have modified this process by using paddles to stir the honey mixture while holding the mixture at 14°C. In alternative creaming methods, the pasteurisation step may be omitted, with the honey instead being slowly warmed to 37°C.

The glucose oxidase is preferably a purified natural glucose oxidase preparation which is of food standard for human consumption, or of medical grade or medical device grade for medical applications. The activity of the glucose oxidase may be selected depending on the desired rate of production of hydrogen peroxide. Several glucose oxidase preparations are commercially available (glucose oxidase is identified by the reference CAS:9001-37-0). Common microbial sources for glucose oxidase from non genetically modified organisms include selected strains of *Aspergillus niger, Penicillium amagasakiense, Penicillium variabile, Penicillium notatum.* Medical device grade glucose oxidase, from GMO *Aspergillus niger,* is available from Biozyme UK, activity 240iu/mg. Food standard glucose oxidase, from *Aspergillus niger,* is available from BIO-CAT INC, activity 15,000 Units/g. Non-Genetically Modified glucose oxidase is available from BIO-CAT INC, activity 12,000/g. Glucose oxidase (GO3B2), from *Aspergillus niger,* is available from BBI Enzymes Limited, activity 360 Units/mg. Contaminants: alpha amylase no greater than 0.05%, Saccharase no greater than 0.05%, maltase no greater than 0.05% and GO/Cat no less than 2000.

The enzyme activity (for example, the glucose oxidase activity) may range, for example, from 1-400 IU/mg, or 1-300 IU/mg, for example 250-280 IU/mg. The amount of enzyme used is likely to depend on several factors, including the desired use, the amount of any catalase activity present in the substance, the amount of substrate present in the substance, the desired level of hydrogen peroxide release, and the desired length of time for hydrogen peroxide release. A suitable amount of enzyme can readily be determined by a person of ordinary skill in the art, if necessary using a well diffusion assay as described in Example 2 below to determine the extent of hydrogen peroxide release for different amounts of enzyme. Suitable amounts of enzyme (such as glucose oxidase) may be from 0.0001% to 0.5% w/w of the fiber, dressing or composition. The amount of enzyme used may be selected so as to produce a fiber, dressing or composition for generating antimicrobial activity that is equivalent to a selected phenol standard (for example a 10%, 20%, or 30% phenol standard).

A "unit" is defined herein as the amount of enzyme causing the oxidation of 1 micromole of glucose per minute at 25 degrees centigrade at pH 7.0.

The Applicant has found that the antimicrobial potency of fibers, dressings or compositions of the invention may be increased simply by increasing the amount of glucose oxidase activity present.

In some embodiments of the invention, there may be more than 15 units, for example at least 30 units, at least 50 units, or at least 100 units, and suitably less than 685 units, for example 100-500 units, of glucose oxidase per gram of the fiber, dressing or composition. This may provide superior antimicrobial properties compared to fibers, dressings or compositions with up to 15 units of glucose oxidase per gram. In particular, there may be increased potency against a wide range of microorganisms, including MSSA, MRSA, Group A and B *Streptococci, Enterococcus, E.coli, E.coli* ESBL, *Serr.liquefaciens* Amp C, *Kleb.pneumoniae, Pseud.aeruginosa,* and *Candida albicans.*

In other embodiments of the invention, there may be at least 500 units, for example 500-1000 units, or 685-1000 units, of glucose oxidase per gram of the fiber, dressing or composition. These can provide more superior antimicrobial properties, such as increased potency against a wide range of microorganisms, including MSSA, MRSA, *E.coli* ESBL, and *Staphylococcus aureus.*

The pasteurisation process inactivates any enzyme activity present in the honey, and so there is no variability in catalase and substrate conversion activity between pasteurised honeys from different sources, or between different harvests of honey from the same source. The amount of substrate conversion activity can be controlled by addition of a purified glucose oxidase preparation with a defined amount and activity of the enzyme. Thus, the inherent variability in antimicrobial properties between different types and harvests of honey is considerably reduced, and the antimicrobial properties of honeys with low antimicrobial potency are improved.

According to further embodiment, which is not according to the invention, the substance may be an inverted substance, preferably an inverted syrup, such as inverted maple syrup. The term "inverted" is used herein to mean that sucrose originally present in a substance has been converted to glucose and fructose, for example by the activity of an invertase or sucrase enzyme. Such inverted substances may then be used with the enzyme glucose oxidase.

Maple syrup is made from the sap of sugar maple or black maple trees. In cold climate areas, these trees store starch in their stems and roots before the winter, which when converted to sugar, rises in the sap in the spring. Maple trees can be tapped and the exuded sap collected and concentrated to form maple syrup. Maple syrup typically comprises approximately 66% sucrose, 33% water, and 1% glucose and fructose. Because of the low amount of glucose present, maple syrup does not have potent antimicrobial generating properties when combined with glucose oxidase. However, if the sucrose is first converted to glucose and fructose, for example by treatment with an invertase or a sucrase, the resulting inverted syrup includes a much higher level of substrate for glucose oxidase.

Fibers, dressings or compositions of the invention can be used to treat any microbial infection that can be treated by hydrogen peroxide. Examples include infection caused by gram positive bacteria, gram negative bacteria, acid-fast bacteria, viruses, yeasts, parasitic or pathogenic micro-organisms or fungi. In particular, infections caused by the following micro-organisms may be treated: *Escherichia coli, Staphylococcus aureus, Pseudomonas aeruginosa, Candida albicans, Propionibacterium acnes, Staphylococcus aureus, Staphylococcus epidermidis, Staphylococcus saprophytics,* Beta haemolytic *Streptococci* Group A or B, *Campylobacter coli, Campylobacter jejuni,* Methicillin Resistant *Staphylococcus Aureus* (MRSA), Methicillin Sensitive *Staphylococcus Aureus* (MSSA), *Botrytis cinerea, Mycobacterium tuberculosis, Cryptosporidium, Plasmodium,* and *Toxoplasma.*

Fibers, dressing or compositions of the invention may be used to treat lower genital tract infections. For example, compositions of the invention may be applied topically to treat such infections. Examples of such infections include bacterial vaginosis and general bacterial vaginal discharge. Compositions of the invention may be applied to an insertion device, such as a tampon.

Fibers, dressings or compositions of the invention may be used to treat infections comprising Carbapenem-resistant enterobacteriaceae (CRE) or Carbapenemase-producing Enterobacteriaceae (CPE) bacteria,

According to the invention there is also provided use of a fiber or composition of the invention to prevent or inhibit microbial growth.

There is also provided according to the invention, a fiber or composition of the invention for use as a medicament. There is further provided according to the invention a fiber or composition of the invention for the prevention, treatment, or amelioration of a microbial infection. The invention also provides use of a fiber or composition of the invention in the manufacture of a medicament for the prevention, treatment, or amelioration of a microbial infection.

There is further provided but not according to the invention as clamed, a method of preventing, treating, or ameliorating a microbial infection, which comprises administering a fiber, composition or wound dressing of the invention to a subject in need of such prevention, treatment or amelioration. The subject may be a human or animal subject. The fiber, composition or wound dressing of the invention may be topically administered.

Compositions that can be used to form fibers or wound dressings of the invention, have been found by the applicant to have remarkable wound healing properties. In particular, chronic wounds that have persisted for one month to over a year have visibly begun to heal within a few days of treatment. In particular, it is believed that the wound healing activity is due to the sustained, low level release of hydrogen peroxide. This results in the delivery of oxygen to the wound site.

Oxygen has a variety of important roles in support of wound healing. The complex wound healing process demands large amounts of energy. If a wound becomes infected, then there is an even bigger energy demand, which in turn means that there is an even greater demand for oxygen. Oxygen is involved in many of the mechanisms of the natural healing process. It has a key role in metabolic support, matrix repair, antisepsis/infection control and signalling and control of cell responses. Wounds which receive adequate oxygen generally heal at an increased rate compared to those which don't have an adequate oxygenation. Ischemia/hypoxia can directly inhibit wound healing processes such as angiogenesis, collagen synthesis and epithelialisation, and also impedes the ability of leukocytes to kill bacteria. As bacteria multiply, more leukocytes are recruited to the wound site, further increasing oxygen consumption.

For a wound to heal, it must have sufficient energy and nutrients to drive the healing process. Oxygen is essential in these metabolic processes. Tissue regeneration and healing involves formation of granulation tissue, epithelialisation, contraction, and re-modelling. The healing process requires the proliferation of cells of various types, as matrix is built-up, new blood vessels are formed and epithelium is replaced. Such cellular activity depends on oxygen availability to allow unhindered respiration. The biosynthetic pathways needed to build all the biopolymers (e.g. proteoglycans, structural proteins) are dependent on oxygen to satisfy the underlying energy requirements. Various enzymes are required, including the matrix metalloproteinases, and these, too, are costly in terms of energy, and hence oxygen, requirements.

Collagen synthesis is a fundamental part of these processes. The deposition of collagen is crucial for rebuilding of connective tissue and as part of the process of angiogenesis. Oxygen is a co-factor required in the hydroxylation of proline and lysine during the formation of pro-collagen. Mature collagen synthesis requires prolyl-hydroxylase and lysyl-hydroxylase enzymes, both of which are dependent on oxygen for their function.

Neovascularization/angiogenesis is essential for complete healing. This must be triggered by an appropriate signal. In high oxygen environments, macrophage leukocytes can trigger this signal, leading to an orchestrated, complex series of events involving tissue degradation followed by collagen formation and organisation, endothelial cell migration/colonisation and vessel formation.

When a wound is created the body's natural defences are activated. Neutrophils gather at the wound site shortly after trauma and release bactericidal reactive oxygen species (ROS) and hydrogen peroxide ($H_2O_2$) to kill bacteria and prevent infection. Macrophages arrive at the wound in response to environmental stimuli, phagocytose foreign particles, and release vascular endothelial growth factor (VEGF), an angiogenic factor crucial for wound healing. Oxygen has a key role in these events. One of the main microbial killing mechanisms of macrophages and neutrophil leukocytes is the "respiratory burst" - a natural activity by which these cells kill microbes. Individuals with defects in their respiratory burst process fall victim to bacterial infections. Leukocytes need oxygen to deliver the respiratory burst effect, and enhanced oxygen levels can boost its potency. These leukocytes also have other bacterial killing mechanisms, triggered when they swallow-up the microbes (phagocytosis). This is an oxygen-dependent process, as it involves substantial energy expenditure, so it does not work well in an oxygen-deprived environment. It works best in an oxygen-enriched situation. Oxygen is also lethal to anaerobic bacteria and is important in the effective functioning of some antibiotics.

Molecular oxygen is an important cell signal interacting with growth factors and other signals (for example, redox signals) to regulate signal transduction pathways. The molecular signal released by macrophages to trigger angiogenesis is "Vascular Endothelial Growth Factor" (VEGF). High oxygen levels can cause macrophage leukocytes to release VEGF. The genes for a number of other important factors and enzymes are induced by high oxygen levels.

The production of nitric oxide (NO) is also recognised as a pivotal signalling and control event in wound healing. NO is made by the enzyme nitric oxide synthase (NOS), and in the early stages of wound healing the inducible form of the enzyme (iNOS) is up-regulated. However, it can only function if arginine and oxygen are in plentiful supply, to enable production of NO at the appropriate rate.

Oxygen also has a direct role in signalling (stimulating) the process of epithelialisation, a key later-stage healing event in which new epithelial cells proliferate, organise themselves and differentiate into structured epithelium.

Thus, the sustained supply of oxygen to the wound site by fibers, wound dressings or compositions of the invention is believed to be particularly important in promoting wound healing.

Wound healing is characterized by four distinct but overlapping phases. As blood escapes into the site of injury, platelets come into contact with collagen and other extracellular matrix (ECM) components. Blood clotting factors are released and within minutes the wound is covered by a fibrin clot. Hemostasis is followed by the inflammatory phase. Neutrophils enter the wound site and remove damaged tissue and bacteria. Macrophages appear later and continue the process of phagocytosis. Both platelets and macrophages release growth factors and pro-fibrotic cytokines such as the platelet-derived growth factor (PDGF) and the transforming growth factor beta (TGF-β). Mast cells release granules containing histamine and other mediators that cause surrounding vessels to become leaky and allow the passage of cells into the wound. In the proliferative phase endothelial cells form new capillaries. Fibroblasts migrate into the cleaned wound from the neighbouring tissue, proliferate and deposit fibronectin, collagen and other components of new ECM. Keratinocytes proliferate, migrate and restore surface integrity. Finally, during the remodelling phase, fibroblast- rich granulation tissue is gradually replaced with a relatively a-cellular scar. Cross-linked collagen molecules give the scar tensile strength that approximates that of the intact skin.

All clinical wounds have a high probability of a microbial load and excessive infection needs to be managed. When this load is high causing overt infection, it places a high demand on oxygen supply because the $O_2$-dependent respiratory burst represents a primary mechanism to endogenously limit wound infection. In addition, the injured tissue needs to fuel the reparative process by means of oxidative metabolism and thus requires additional oxygen. This increase in oxygen demand on the injured tissue which characteristically suffers from disrupted vasculature leads to an oxygen deficit causing wound hypoxia. Diminished peripheral blood flow and impaired angiogenesis and vasculogenesis are characteristic of retarded wounds in diabetic patients, particularly in lower extremities. This low partial pressure of oxygen reduces the activity of endogenous enzymes producing reactive oxygen species (ROS). Wound hypoxia retards healing and the state of tissue oxygenation is an important determinant limiting ROS formation and successful healing. It is now evident that oxygen-dependent redox-sensitive processes are not only required to disinfect wounds and promote healing but also the signalling and coordination roles in the wound response processes, which represent an integral component of the healing cascade.

The previously published preconception that biological free radicals are automatically mediators of cellular destruction has now been challenged. In vivo, hydrogen peroxide ($H_2O_2$) is actively generated at the wound site, to promote wound healing and that in biological systems oxidants are not only the triggers for oxidative damage but that oxidants such as $H_2O_2$ serve as signalling messengers and drive several aspects of cellular activity, these results raise the possibility that ROS molecules may exert signalling actions beyond the single cellular context and may play an important paracrine signalling role during the wound repair process. Today, this concept has been validated . Evidence supporting oxidants such as $H_2O_2$ performing an integral role in cell stress/ inflammatory responses and the subsequent tissue/neuron repair process is overwhelming.

The primary ROS, $H_2O_2$ functions as a second messenger at low concentrations and plays a prominent role as it is easily synthesized, easily degraded and present within all types of cells. It has a longer half-life than radical ROS and its small uncharged molecule diffuses easily through tissues. It does not react indiscriminately with the neighbouring molecules like many radicals. Low concentrations of $H_2O_2$ (10 $\mu$M) act as a chemoatractant, this activity is not dependent on the presence of blood serum . $H_2O_2$ stimulates the proliferation of human fibroblasts and vascular endothelial cells in a similar range of concentrations.

When a wound is created the body's natural defences are activated, immune cells gather at the wound site shortly after trauma and release high concentrations of bactericidal $H_2O_2$ to prevent contamination. The bulk of ROS are released by neutrophils and macrophages during the inflammatory phase of healing, the primary microbial killing mechanisms is this "respiratory burst". The resulting wound fluid contains micromolar concentrations of ROS including $H_2O_2$, which protect the host against bacterial and fungal infection. The bacteriostatic effect of $H_2O_2$ was observed at concentrations between 25-50 $\mu$M, while at concentrations exceeding 500 $\mu$M bacterial cell killing of viable *E.coli* was observed . Individuals with defects in their respiratory burst process fall victim to bacterial infections. The lack of specificity of endogenous $H_2O_2$ towards particular bacterial molecules, coupled with their ability to be secreted into the extracellular milieu rather than solely retained in the phagosome means that these molecules can exert their antimicrobial properties throughout the wound environment, it has been shown at maximum of ROS production, an $H_2O_2$ gradient was formed around the wound margin, reaching a distance of approximately 100 to 200 $\mu$M.

Wound healing involves an optimal combination of the constructive and destructive roles of cells and their associated ROS to remodel tissue in parallel to eradicating infectious organisms and non-self-material. For diabetic patients suffering from impaired wound healing, particularly in lower extremities through diminished peripheral blood flow and impaired angiogenesis and vasculogenesis, a consequence of the reduced concentration of ROS, the differentiation of myofibroblasts (MFB), responsible for wound contraction, is impaired. The expression of $\alpha$-smooth muscle actin, the main component of myofibroblast cytoskeleton, decreases with the reduced supply of oxygenated blood flow. Higher concentration of $H_2O_2$ (500 $\mu$M) stimulates the production of macrophage inflammatory protein-1$\alpha$ that is chemotactic for mononuclear phagocytes, neutrophils, eosinophils, basophils and lymphocytes. It has been shown that 100 $\mu$M $H_2O_2$ produce vascular endothelial growth factor (VEGF) that stimulates angiogenesis, is chemotactic to keratinocytes in which low levels of $H_2O_2$ promote cell migration and proliferation through activation, and low levels of $H_2O_2$ (10 $\mu$M) have shown to be chemotactic to neutrophils, angiogenesis / chemotaxis processes are disrupted in chronic wounds.

In contrast to the favourable outcome of $H_2O_2$ treatment in patients, there are reports suggesting the need to suppress ROS formation in wounds. In response to excessive ROS concentrations within a wound, leukocytes and stromal cells possess a number of both intracellular, extracellular and membrane bound antioxidant systems to scavenge ROS before healthy tissue can become compromised . They have appropriate systems in place to safely detoxify reactive oxygen metabolites an unavoidable by-product of respiration. The biochemical systems by which ROS can be neutralised are vital to the survival of an organism. However, left undisposed of $H_2O_2$ has the potential to form the most reactive of all the radicals, OH. It has been demonstrated that up-regulations occur in ROS scavengers during cutaneous wound healing, particularly at the wound edge possibly with the goal of protecting proliferative cells from the high levels of oxidants contained within the compromised interstitial fluids.

This ROS detoxification is multi-channelled, genomically controlled by a diverse series of transcription factors with resulting enzymatic systems including three types of processing enzyme superoxide dismutase (SOD) (cytosolic, mitochondrial and extracellular), glutathione peroxidase and catalase, that decrease the potency or render ROS completely harmless. In addition to detoxification, SOD is an important enzyme in facilitating the properties of $H_2O_2$, due to its relatively long half-life compared to other cellular oxidants and its ability to traverse membranes, as the optimal signalling candidate. In addition to proteinaceous ROS control, ROS can interact with non-enzymatic metabolites comprised of small antioxidant molecules, such as vitamin C, vitamin E, $\beta$-carotene, glutathione, coenzyme Q, bilirubin, $\alpha$-tocopherol, nicotinamide adenine dinucleotide phosphate (NADPH) and urate, particularly moieties with a metal ion capable of oxidation/reduction reactions e.g. transferrin and ferritin all possess ROS-scavenging capability.

However it is catalase that 'leads the charge' to eliminate ROS, and accounts for more than 50% of the removal of $H_2O_2$. Catalase is a haeme-containing enzyme which is expressed throughout numerous tissues of the body with higher concentration in the liver, kidneys and erythrocytes, found within intracellular organelles, such as peroxisomes, and relies on diffusion of $H_2O_2$ into these compartments and neutralisation. The serum level of catalase in males has been reported as 50 kU/l, and it has been shown that catalase activity in healing wounds decrease during the first week post-wounding and activity levels of catalase recover to its original level at two weeks post-wounding. Over expression of catalase in the wound has been achieved by gene transfer and the up-regulated degradation of $H_2O_2$ by this enzyme has resulted in the impairment of wound angiogenesis and wound closure. Additionally it has been shown that catalase and the antioxidant N-acetylcystein block the response of cultured smooth muscle cells to platelet-derived growth factor (PDGF), which induces chemotaxis and DNA synthesis which has shown to be mediated by $H_2O_2$.

At present, acute wounds normally heal in a very orderly and efficient manner characterized above and therapeutic regimes employ traditional procedures without assessing the wound environment and patient systemic condition. Pathologic conditions such as chronic wounds e.g. diabetic non-healing pressure ulcers affects about 1% of the European population, along with the dreadful impact on the quality of life for the affected patient, chronic wounds are of vital economic relevance, as potentially 2% of European health budgets are spent on combating chronic wounds. The positive role of oxygen in acute and chronic wound healing has been demonstrated and the introduction of hyperbaric treatments including $O_2$ generating topical dressings to resolve unresponsive ulcers has proven to be beneficial, as this allows for the in-situ generation of ROS. In addition to these, traditional therapies that relied on low levels of $H_2O_2$ production have been well characterised and now new bioengineered therapies that directly introduce continuous low therapeutic doses of $H_2O_2$ have started to demonstrate their effectiveness in preventing chronic wounds. This technology does not rely on swamping the wound site with high levels of ROS that inflame the wound and impair / delay healing due to oxidative damage, and in addition to the fact that the concentration of ROS would be on a step decay curve e.g. cream containing 1.5% $H_2O_2$. This advanced therapy relies on a complex control in the way which $H_2O_2$ is synthesised and degraded, coupled with its membrane permeability within the inflammatory matrix, allowing for accurate delivery of ROS at a therapeutic dose to the wound site for sustained periods of time.

From this research and development of wound healing, a comprehensive image of how endogenous ROS generated in the wound environment stimulates healing and that numerous processes subject to redox control are critically important. Excessive levels of ROS, particularly $H_2O_2$ lead to oxidative damage, prolonged inflammation and possibly neutrophil associated proteolytic damage and cause delayed wound closure, although these elevated levels could be present as a consequence of impaired healing a frequent occurrence among the immobile and debilitated patients. However, the quantity of $H_2O_2$ generated to enhance healing and decontaminate infected wounds is insufficient to cause oxidative damage and redox-based strategies may serve as effective tools to jump-start healing of chronic wounds. Very low concentrations of $H_2O_2$ demonstrated above are beneficial in wound healing especially in chronic wounds such as diabetic ulcers. Such an understanding has resulted in a novel redox-based technology that can mimic this endogenous ROS generation in the wound microenvironment and has potential to benefit chronic wound healing.

It is believed that the wound healing activity may also be contributed to by a number of activities of the fibers, dressings or compositions in addition to their hydrogen-peroxide releasing activity. In particular, preferred embodiments are believed to have ability to debride and deodorise a wound, to have anti-inflammatory properties, to be able to stimulate tissue growth, and to manage pain and minimise scarring.

Malodour is a common feature of chronic wounds and is attributed to the presence of anaerobic bacterial species that produce malodorous compounds from decomposed serum and tissue proteins. In addition to the antimicrobial action, fibers, dressings or compositions of the invention that include glucose (preferably 24-40% by weight) allow bacteria to metabolise this in preference to amino acids, resulting in the production of a non-odorous metabolite, lactic acid.

Fibers, dressings or compositions with a high osmolarity (suitably having an $a_w$ in the range similar to honey, i.e. 0.47-0.7) are believed to facilitate the debridement of wounds by the autolytic action of tissue proteases. They may create a moist wound environment by drawing out lymph fluid from the wound tissues through their strong osmotic action. This provides a constant supply of proteases at the interface of the wound bed and the overlying necrotic tissue. This action also washes the surface of the wound bed from beneath. The activation of proteases by hydrogen peroxide released by fibers, dressings or compositions of the invention may also assist. The debriding action may also contribute to the lowering of a wound's bacterial load by removal of dead tissue. Dead tissue is well known to provide an excellent medium for bacterial growth and increase the risk of infections if left in the wound.

The body's inflammatory response marks the beginning of the healing process, but a prolonged reaction can inhibit healing, causing further damage to the tissues and making it harder to manage the wound. A prolonged inflammatory response is often associated with high levels of exudate. Suppressing inflammation, as well as reducing pain for the patient, reduces the opening of blood vessels, thus lessening oedema and exudate. It is thought that the ability of aspects of the invention to clear infection and debride wounds may contribute to the anti-inflammatory action. Embodiments of the invention that include antioxidants (preferably one or more antioxidants present in honey) may also reduce excessive inflammation by mopping up free radicals.

Prolonged inflammation causes fibrosis that manifests as hypertrophic scarring in wounds. Fibers, dressings or compositions of the invention may reduce scarring by reducing inflammation, promoting angiogenesis and stimulating the formation of granulation tissue. Preferred embodiments may also stimulate the growth of epithelium.

The fibers, dressings and compositions of the invention are also believed to have immunostimulatory effects mediated by interleukin-1 (IL-1). They are believed to promote the release of IL-1 from skin cells. IL-1 is a cytokine which is also secreted by macrophages, monocytes and dendritic cells. It is an important part of the inflammatory response of the body against infection. It increases the expression of adhesion factors on endothelial cells to enable transmigration of leukocytes to sites of infection. It also acts on the thermoregulation centre of the brain leading to an increased body temperature. It is referred to as an endogenous pyrogen. The increased body temperature helps the body's immune system to fight infection. This is the initial phase of an inflammatory immune response which augments the antimicrobial activity of the system. The inflammatory response plays a central role in wound healing through its defence against possible infection and by participating in cell and tissue repair and re-growth. The antimicrobial effect of fibers, dressings or compositions of the invention is believed to be aided and complemented by the immunostimulatory effect which aids the re-growth and repair of damaged tissues and/or cells.

Fibers, dressings or compositions of the invention, may provide a moist healing environment for wound tissue with no risk of maceration of the surrounding skin, and prevent adherence of dressings to the wound bed so that there is no pain and no tissue damage when dressings are changed. They may also stimulate healthy cell development and accelerate granulation and epithelialisation processes in the wound. They may also have an occlusive action to protect the wound and damaged tissues. It is also believed that honey-based compositions cause a significant decrease in wound pH which favours the healing process.

According to a preferred aspect of the invention, the fibers, compositions or wound dressings of the invention may be used in a method of wound care, including the treatment of a wound, or the treatment or management of wound sepsis.

The wound may be an acute wound, chronic wound, surgical wound (for example, a Caesarean wound), chronic burn, or an acute burn. The fiber, composition or wound dressing may be used in the prophylactic prevention of wound sepsis. It will be appreciated that fiber or dressing may be contacted or diluted by liquid present at the wound site, which, in some embodiments, would leads to the release of hydrogen peroxide.

A wound occurs when the integrity of any tissue is compromised (e.g. skin breaks, muscle tears, burns, or bone fractures). A wound may be caused by an act (a trauma) or surgical procedure, by an infectious disease, or by an underlying condition.

Acute wounds include surgical incisions and traumatic injuries such as lacerations, abrasions, avulsions, penetrations or bites, and burn injuries. Acute wounds normally proceed through an orderly and timely reparative process that results in sustained restoration of anatomic and functional integrity.

A chronic wound is a wound that has been in existence for more than three weeks or that has failed to proceed through an orderly and timely process to product anatomic and functional integrity or to proceed through the repair process without establishing a sustained and functional result (Lazarus et al, Arch Dermatol. 1994;130(4):489-493).

Chronic wounds can be classified into four categories: venous ulcers, arterial ulcers, diabetic ulcers, and pressure ulcers. A small number of wounds that do not fall into these categories may be due to causes such as radiation poisoning or ischemia.

Venous ulcers, which usually occur in the legs, account for about 70% to 90% of chronic wounds and mostly affect the elderly. They are thought to be due to venous hypertension caused by improper function of valves that exist in the veins to prevent blood from flowing backward. Ischemia results from the dysfunction and, combined with reperfusion injury, causes the tissue damage that leads to the wounds. In venous disease, ulcers are usually located in the gaiter area between the ankle and the calf, often on the medial aspect of the leg.

Arterial leg ulcers occur as a result of reduced arterial blood flow and subsequent tissue perfusion. Atherosclerosis or peripheral vascular disease is the most common cause of arterial leg ulceration. A reduction in blood supply, if left untreated, can cause death of tissue in the area being fed by the affected artery. Ulcer development is often rapid with deep destruction of tissue. Arterial leg ulcers can occur anywhere on the lower leg and are often deeper and rounded, with clearly defined borders.

Diabetic ulcers: diabetes causes immune compromise and damage to small blood vessels, preventing adequate oxygenation of tissue, which can cause chronic wounds. Diabetes causes neuropathy, which inhibits nociception and the perception of pain. Thus, patients may not initially notice small wounds to legs and feet, and may therefore fail to prevent infection or repeated injury. Pressure also plays a role in the formation of diabetic ulcers. Diabetics have a 15% higher risk for amputation than the general population due to chronic ulcers.

Pressure ulcers usually occur in people with conditions such as paralysis that inhibit movement of body parts that are commonly subjected to pressure such as the heels, shoulder blades, and sacrum. Pressure ulcers are caused by ischemia that occurs when pressure on the tissue is greater than the pressure in capillaries, and thus restricts blood flow into the area. Muscle tissue, which needs more oxygen and nutrients than skin does, shows the worst effects from prolonged pressure. As in other chronic ulcers, reperfusion injury damages tissue.

When treating ulcers it is desirable to: create a barrier to protect the wound while it is healing; provide a moist wound environment; deslough the wound; reduce bacterial load; and ideally promote healing with immunomodulation and improved nutrition. No conventional wound treatment product is able to achieve all of these effects. Some conventional treatments have some of these effects, but are quite toxic, and so are not ideal treatments. However, fibers, dressings or compositions of the invention, in particular those in which the substance is a honey, may have all of these properties. In addition, the antimicrobial potency can be controlled to be far more potent than other honey-based wound care products, and are effective against a range of pathogens involved in chronic wounds, including antibiotic resistant organisms, such as MRSA.

Fibers, compositions or dressings of the invention may be particularly advantageous in the early treatment of wounds, especially in patients, such as diabetic patients, where the wounds are likely to get worse. Early treatment may prevent the complications that arise in chronic wounds, keeps the patients active, and avoids the expense of dealing with complications.

Fibers, dressings or compositions of the invention may also be effective at reducing or preventing infection of surgical wounds. Infection of surgical wounds is a problem, particularly in Caesarean sections which have quite a high infection rate of around 10%. Example 24 below demonstrates that a single application of a composition (which may be used to form a fiber of the present invention) to a Caesarean wound post-surgery reduced the rate of surgical site infection by 60% compared to historical data.

Peripherally inserted central catheters (PICC lines) are used to administer chemotherapy treatment and/or other medicines. A PICC line is a long, thin, flexible tube which is inserted into one of the large veins of the arm near the bend of the elbow. It is then threaded into the vein until the tip sits in a large vein just above the heart. It is, however, possible for an infection to develop inside the line or in the area where it goes into the vein. If an infection develops, patients are typically given antibiotics. If these do not clear the infection, or if the infection is serious, the line may be removed. To reduce the chances of an infection developing, a dressing containing an antimicrobial agent such as chlorhexidine or silver may be applied to the line entry site.

Compositions that can be used to form fibers or dressings or compositions of the invention have been found to be effective at preventing and clearing colonisation of PICC lines by topical application of a composition of the invention to the line entry site (see Example 23 below).

Fibers or dressings of the invention can be easily applied without any specialist training or complex equipment. They may be highly suitable for use in the third world, where patients often have increased susceptibility to infection, and the materials and instruments used in surgery and wound care are often more infected than in developed countries.

Fibers or dressings of the invention may be non toxic, and so may not have any of the problems associated with silver-containing dressings, PVP-I, or chlorhexidine.

Fibers or dressings of the invention may also be used to treat donor or recipient graft sites.

There is provided a method of treating a wound, which comprises administering a fiber, dressing or composition of the invention to the wound.

There is also provided a fiber or composition of the invention for treatment of a wound.

There is further provided use of a fiber or composition of the invention in the manufacture of a medicament for treatment of a wound.

There is also provided a method of treating inflammation which comprises administering a fiber, dressing or composition of the invention to a site of inflammation.

There is also provided a fiber or composition of the invention for treatment of inflammation.

There is further provided use of a fiber or composition of the invention in the manufacture of a medicament for treatment of inflammation.

There is also provided a method of stimulating tissue growth, which comprises administering a fiber, wound dressing or composition of the invention to a site in need of such stimulation.

There is also provided a fiber or composition of the invention for stimulating tissue growth.

There is further provided use of a fiber or composition of the invention in the manufacture of a medicament for stimulating tissue growth.

There is also provided a method of debriding a wound, which comprises administering a fiber, wound dressing or composition of the invention to a wound in need of debridement.

There is also provided a fiber or composition of the invention for debriding a wound.

There is further provided use of a fiber or composition of the invention in the manufacture of a medicament for debriding a wound.

There is also provided a method of deodorising a wound, which comprises administering a fiber, wound dressing or composition of the invention to a wound in need of deodorising.

There is also provided a fiber or composition of the invention for deodorising a wound.

There is further provided use of a fiber or composition of the invention in the manufacture of a medicament for deodorising a wound.

For wound healing applications, fibers, wound dressings or composition of the invention may be administered at an appropriate frequency determined by the healthcare provider. They may be administered at least every several days, for example every week, but preferably every day or every other day.

The amount of a fiber, wound dressing or composition of the invention which is administered will depend on many factors, such as the strength of the antimicrobial properties, and other wound healing properties, on the size of the wound, and on the age and condition of the subject to be treated.

Preferred fibers, wound dressings or compositions of the invention for medical use are sterile, and for single use.

Fibers, wound dressings or compositions of the invention that are stored away from exposure to light may retain stability for at least six months. For example, they may be packaged in high-density polyethylene/low-density polyethylene (HDPE/LDPE) tubes or in polyester-aluminium-polyethylene (PET/Al/PE) sachets.

Also according to the invention, there is provided a pharmaceutical composition comprising a fiber or composition of the invention together with a pharmaceutically acceptable carrier, excipient, or diluent.

A fiber, wound dressing or composition of the invention may comprise at least one suitable antimicrobial or immunostimulatory component, excipient or adjuvant, or any other suitable component where it is desired to provide ability to generate antimicrobial activity. Preferably, however, fibers, dressings or compositions of the invention do not include any antibiotic.

Fibers, wound dressings or compositions of the invention may be used for the treatment of a skin disorder, such as acne, eczema, or psoriasis. Acne and eczema may have a microbial infection component which can be treated by the fibers wound, dressings or compositions, and secondary microbial infections of psoriatic lesions caused by scratching could also be treated.

Fibers, wound dressings or compositions of the invention may be used in veterinary medicine. Important veterinary applications include the treatment of microbial infections and the treatment or management of wound care, or burn treatment. Specific conditions include chronic skin infections in dogs (subcutaneous Staphylococcus infections), Otitis externa (ear infections), oral care in animals, Campylobacter infections in chickens, coliosis, enteric microbial infections in pigs, poultry and cattle, Cryptosporidium infections, clearance of zoonotic infections, wound dressing, e.g. horn removal, and abscess treatment. The present invention has particular advantages in veterinary usage, in that it allows the treatment of microbial infections without introducing antibiotics into the food chain.

The invention provides a method of preventing or treating a microbial infection that includes a biofilm, or a microbe that is capable of forming a biofilm, which comprises administering a fiber, wound dressing or composition according to the invention.

According to the invention, there is provided a fiber or composition according to the invention, for use in the prevention or treatment of a microbial infection that comprises a biofilm, or a microbe that is capable of forming a biofilm, preferably wherein the fiber or composition prevents or inhibits growth or seeding of the biofilm by the microbe.

According to the invention there is provided use of a fiber or composition according to the invention in the manufacture of a medicament for the prevention or treatment of a microbial infection that comprises a biofilm, or a microbe that is capable of forming a biofilm, preferably wherein the fiber or composition prevents or inhibits growth or seeding of the biofilm by the microbe.

The microbial infection may comprise a bacterium, preferably a gram-negative bacterium, that is capable of forming a biofilm, optionally wherein the bacterium is *Pseudomonas aeruginosa or Acinetobacter baumannii.*

Compositions, fibers or wound dressings according to the invention may be used to treat wounds that are critically colonized. The term "critically colonized" is often used to refer to a wound that has reached a critical point at which bacteria begin to negatively affect the wound and begin to elicit signs of their presence. A critically colonized wound may indicate the presence of a biofilm. A bacterial load of greater than $10^5$ organisms/gram of tissue is often accepted as impeding wound healing (Siddiqui AR, Bernstein JM (2010) Chronic wound infection: Facts and controversies. Clinics in Dermatology 28: 519-26; Edmonds, M., & Foster, A. (2004). The use of antibiotics in the diabetic foot. Am J Surg, 187(5A), 25S-28S. Consequently, fibers, wound dressings or compositions of the invention may be used to treat wounds that have a bacterial load of greater than $10^5$ organisms/gram of tissue.

A composition of the invention may be contained within a water-soluble container, such as a sachet or pouch. So, there may be provided a water-soluble container enclosing, or containing, a composition of the invention. Advantageously, this may allow a precise amount of the composition to be delivered for a particular therapeutic application by adding a measured amount of solvent, such as water or saline. For example, a composition of the invention contained in a water-soluble sachet may be used to form a nasal douche solution, or a solution that is to be nebulised or atomised. Consequently, contacting the soluble sachet containing the composition, with an aqueous solvent, may result in formation of an aqueous mixture. The aqueous mixture may contain sufficient free water to allow the enzyme to convert the substrate and produce hydrogen peroxide.

The water-soluble sachet is preferably manufactured from a medical grade material. The sachet may be dissolvable in water at 38°C. Preferably, the water-soluble sachet is non-toxic, anti-static, resistant to degradation by ultraviolet light and resistant to degradation by gases, oils and greases. In one example, the soluble sachet may be manufactured from a polymer or plastics material, such as polyvinyl alcohol.

Compositions of the invention contained in water-soluble sachets may useful for treating one or more of the following conditions: nasal conditions, such as sinusitis and rhinosinusitis; respiratory tract infections, such as upper respiratory tract infections (e.g. tonsillitis, laryngitis and sinusitis or lower respiratory tract infections (e.g. bronchitis, pneumonia, bronchiolitis and tuberculosis); chronic obstructive pulmonary disease; and cystic fibrosis.

There may be provided a wound dressing which comprises a composition of the invention and a water-soluble material which may function as a barrier or layer. The water-soluble material may be in contact with, or adjacent to, the composition. The composition of the invention may be enclosed by, or contained within, the water-soluble material. The water-soluble material may form a sachet, pouch or enclosure. The composition may be positioned between layers of the water-soluble material. The water-soluble material may be manufactured from a medical grade material. The water-soluble material may be dissolvable in water at 38°C. Preferably, the water-soluble material is non-toxic, anti-static, resistant to degradation by ultraviolet light and resistant to degradation by gases, oils and greases. In one example, the water-soluble material may be manufactured from a polymer or plastics material, such as polyvinyl alcohol. In use, the dressing may be applied to a wound, and the water-soluble material may thus dissolve in wound exudate, allowing the composition of the invention to contact the wound and deliver reactive oxygen to the wound. Advantageously, a measurable and accurate dose of reactive oxygen may thus be delivered to a wound. The water-soluble material may control release of the composition from the wound dressing.

The water-soluble material and the composition may be attached to the surface of a conventional aseptic wound dressing.

In some embodiments, it is advantageous for compositions of the invention to have a particularly low water content. This may be the case, for example, if the composition is to be contained within a water-soluble container or sachet. If the water content is too high, it may only be possible to contain the composition in a water-soluble container for a short time. Reducing the water content may thus enable a longer shelf-life. A three-year shelf life, for instance, is particularly desirable.

In some embodiments, there is 10% by weight or less of water in the composition. In some embodiments, there is 5% by weight or less, or even 3% by weight or less of water in the composition. In some embodiments, the composition with reduced water content is in a liquid form. In some embodiments, the composition is in a solid form. Suitable solid forms include powders, flakes or granules. Other forms include lozenges or tablets. In some embodiments, the composition is a paste. A paste, for example, may not readily flow at ambient or room temperature (e.g. 20-26°C), but it may have a soft and/or malleable consistency.

Dry honey or dried honey products are commercially available and are typically obtained by processes such as spray-drying, freeze-drying or vacuum-drying. Compositions of the invention with reduced water content may thus be manufactured using such processes. Compositions of the invention may be obtained, or obtainable, by adding an enzyme that is able to convert a substrate to release hydrogen peroxide to a substance that includes a substrate for the enzyme, wherein the substance has been dried and comprises 10% or less, 5% or less or 3% or less (by weight) of water. Alternatively, the enzyme may be added to the substance, and the resulting composition is dried. The enzyme may be in a dry or dried form when it is added to the substance. For example, the enzyme may have been freeze-dried. The enzyme may contain 12% or less, 10% or less, 5% or less or 3% or less (by weight) of water. Preferably, if the enzyme is in a dry or dried form, it contains less 5% (by weight) of water.

Additives may be added to facilitate the drying process or to improve the properties of the dried composition. For example, dried honey products have a tendency to cake or agglutinate unless processing additives are included. Suitable additives include processing aids, drying aids, bulking agents or anticaking agents. Additives may include starch, milk powder, calcium stearate, bran dextrins, lecithin and soy flour. The remainder of the formulation may thus include suitable additives. In preferred embodiments, there are no additives added to facilitate the drying process or to improve the properties of the composition.

Consequently, according to the invention there may be provided a composition comprising: an enzyme that is able to convert a substrate to release hydrogen peroxide; and a substance that includes a substrate for the enzyme, wherein the enzyme is additional to any enzyme activity able to convert the substrate to release hydrogen peroxide that may be present in the substance, and wherein the composition comprises 10% or less, 5% or less or 3% or less (by weight) of water. The composition may be further defined as described herein. For example, the enzyme may be glucose oxidase and the substance may be a purified substrate (such as glucose). The composition may be obtained or obtainable by freeze-drying. The composition may then be used in the treatment of medical conditions, as described herein. For example, the composition may be used to treat wounds. To treat a medical condition the composition may first be added to water to form an aqueous solution. Addition of water may initiate hydrogen peroxide production.

According to the invention, there may be provided a method comprising: drying a composition, the composition comprising an enzyme that is able to convert a substrate to release hydrogen peroxide, and a substance that includes a substrate for the enzyme, wherein the enzyme is additional to any enzyme activity able to convert the substrate to release hydrogen peroxide that may be present in the substance. The composition may be dried by spray-drying, freeze-drying or vacuum-drying. The drying may reduce the water content in the composition to 12% or less, 10% or less, 5% or less or 3% or less (by weight) of water.

Alternatively, there may be provided a method, comprising: adding an enzyme to a dried substance that includes a substrate for the enzyme. Alternatively, the method may comprise i) drying a substance that includes a substrate for the enzyme, such that the drying results in the substance having 10% or less, 5% or less or 3% or less (by weight) of water; and ii) adding the enzyme to the dried substance, the enzyme being additional to any enzyme activity able to convert the substrate to release hydrogen peroxide that may be present in the substance. The substance may be dried by spray-drying, freeze-drying or vacuum-drying. The enzyme added to the substance may be in a dry or dried form.

Compositions, fibers or dressings of the invention may comprise sufficient enzyme and substrate to provide for sustained release of hydrogen peroxide at a specific level or concentration. For example, compositions, mixtures, fibers or dressings of the invention may provide for sustained release of hydrogen peroxide at a concentration of at least 2 ppm, at least 5 ppm, at least10 ppm, at least 20 ppm or at least 50 ppm. In preferred embodiments, the level may be at least 2 ppm. In some embodiments, the concentration may be, at the most, 500 ppm, 200 ppm, 100 ppm, 50 ppm, 20 ppm or 10 ppm. In preferred embodiments, the level may be 20 ppm or less. In even more preferred embodiments, the level may be 10 ppm or less.. For example, the concentration may be 10 to 500 ppm, 20 to 200 ppm or 50 to 100 ppm, 2 to 50 ppm, 2 to 20 ppm or 5 to 10 ppm. If the composition does not comprise sufficient free water to allow the enzyme to convert the substrate (e.g. if the composition is a dry or dried composition), hydrogen peroxide production may only occur once it has been diluted by water and there is sufficient free water to allow the enzyme to convert the substrate. Addition of water may thus initiate hydrogen peroxide production. Compositions, mixtures, fibers or dressings may provide for sustained release of hydrogen peroxide for at least 1 hour, at least 12 hours, at least 24 hours, at least 2 days, or at least 4 days. Preferably, the level of hydrogen peroxide is sustained for at least 4 days. In preferred embodiments, the level of hydrogen peroxide is sustained at 10 to 500 ppm for at least 1 hour, at least 12 hours, at least 24 hours, at least 2 days, or at least 4 days. In other embodiments, the level of hydrogen peroxide is sustained at 50 to 100 ppm for at least 1 hour, at least 12 hours, at least 24 hours, at least 2 days, or at least 4 days. In other embodiments, the level of hydrogen peroxide is sustained at 2 to 50 ppm for at least 12 hours, at least 24 hours, at least 2 days, or at least 4 days. In other embodiments, the level of hydrogen peroxide is sustained at 5 to 10 ppm for at least 12 hours, at least 24 hours, at least 2 days, or at least 4 days.

Compositions of the invention, or compositions for use in the invention, may comprise a non-aqueous solvent. If a storage-stable composition is desired, it may not be possible to decrease viscosity by adding aqueous solvent.

In compositions of the invention comprising a non-aqueous solvent, the non-aqueous solvent may comprise (or consist of) a mixture of more than one non-aqueous solvent. Reference herein to "non-aqueous solvent" thus includes one or more, or at least one, non-aqueous solvent. However, in some embodiments, the non-aqueous solvent may comprise (or consist of) only one non-aqueous solvent.

By forming a solution in a non-aqueous solvent, the substance in the composition may become more readily processable and less sticky. Such solutions may thus be coated onto substrates, such as fabric substrates, and may thus provide a component of a wound dressing. Furthermore, a less viscous composition may be sprayable.

So, according to the invention, there is provided a composition for generating antimicrobial activity comprising an enzyme that is able to convert a substrate to release hydrogen peroxide, a substance that includes a substrate for the enzyme, and a non-aqueous solvent.

Compositions of the invention comprising non-aqueous solvents may be electrospinnable, as described herein.

Compositions of the invention comprising non-aqueous solvents, may comprise a polymer, as described herein.

According to the invention, there is provided a composition comprising an enzyme that is able to convert a substrate to release hydrogen peroxide; a substance that includes a substrate for the enzyme; a non-aqueous solvent; and a polymer.

In compositions of the invention comprising non-aqueous solvents, the substance may include a purified substrate for the enzyme, as described herein.

According to the invention, there is provided a composition comprising an enzyme that is able to convert a substrate to release hydrogen peroxide; a substance that includes a purified substrate for the enzyme, and a non-aqueous solvent.

According to the invention, there is provided, a composition for generating anti-microbial activity, wherein the composition comprises an enzyme that is able to convert a substrate to release hydrogen peroxide, a substance that includes a purified substrate for the enzyme, a non-aqueous solvent and a polymer.

Compositions comprising a non-aqueous solvent may be storage-stable compositions which do not include sufficient free water to allow the enzyme to convert the substrate, as described herein.

In compositions comprising a non-aqueous solvent, the substance may lack catalase activity, as described herein.

Compositions comprising a non-aqueous solvent may provide for sustained release of hydrogen peroxide for a period of time, as described herein. For example, compositions may provide for sustained release of hydrogen peroxide for at least twenty four hours, more preferably at least forty eight hours, possibly following dilution of the composition. Suitably compositions provide for sustained release of hydrogen peroxide at a level of less than 2 mmol/litre for a period of at least twenty four hours, following dilution of the composition.

Compositions comprising non-aqueous solvents, may comprise sufficient enzyme and substrate to provide for sustained release of at least 0.1, 0.5, 1 or 1.5 mmol/litre hydrogen peroxide for a period of at least 24 hours, more preferably 48 hours.

According to the invention there is provided a composition comprising: an enzyme that is able to convert a substrate to release hydrogen peroxide; a substance that includes a substrate for the enzyme; and a non-aqueous solvent, wherein the enzyme is additional to any enzyme activity able to convert the substrate to release hydrogen peroxide that may be present in the substance, and wherein the composition comprises 12% or less, 10% or less, 5% or less or 3% or less (by weight) of water.

In compositions comprising a non-aqueous solvent, the enzyme is additional (i.e. added as a result of human intervention) to any enzyme activity able to convert the substrate to release hydrogen peroxide (referred to herein as "substrate conversion activity") that may be present in the substance. Compositions comprising non-aqueous solvent may comprise a purified enzyme, as described herein.

There is also provided according to the invention compositions comprising a non-aqueous solvent for use as a medicament. For example, such compositions of the invention may be used for treating, or used in methods of treating, any disease or condition described herein, such as microbial infections.

In compositions of the invention that comprise a non-aqueous solvent, the non-aqueous solvent may comprise ethanol, dimethyl sulphoxide, glycerol, ethylene glycol or propylene glycol. Preferably, the non-aqueous solvent is or comprises glycerol. Glycerol may act as a humectant and so compositions comprising glycerol may assist in softening or moisturising dry skin. The non-aqueous solvent may comprise one or more of ethanol, dimethyl sulphoxide, glycerol, ethylene glycol or propylene glycol. In some embodiments, the non-aqueous solvent may comprise two of ethanol, dimethyl sulphoxide, glycerol, ethylene glycol or propylene glycol. In some embodiments, the non-aqueous solvent may comprise three of ethanol, dimethyl sulphoxide, glycerol, ethylene glycol or propylene glycol. In some embodiments, the non-aqueous solvent may comprise four of ethanol, dimethyl sulphoxide, glycerol, ethylene glycol or propylene glycol.

Solubility parameters and viscosity parameters for various non-aqueous solvents are shown in the following table.

| Substance | Hansen solubility parameter | | | | Solubility of Glucose g/100ml of solvent |
|---|---|---|---|---|---|
| | $\delta_t$/MPa$^{1/2}$ | $\delta_d$/MPa$^{1/2}$ | $\delta_p$/MPa$^{1/2}$ | $\delta_h$/MPa$^{1/2}$ | |
| Ethanol | 26.5 | 15.8 | 8.8 | 19.4 | 1.94 g |
| Dimethyl sulphoxide | 26.7 | 18.1 | 16.4 | 10.2 | 54 g |
| Propylene glycol | 30.2 | 16.8 | 9.4 | 23.3 | |
| Ethylene glycol | 32.9 | 17.0 | 11.0 | 26 | |
| Glycerol | 36.1 | 17.4 | 12.1 | 29.3 | |
| Water | 47.8 | 15.6 | 16.0 | 42.3 | 90g |

| Substance | Viscosity |
|---|---|
| Ethanol | 1.04mPa.s |
| Dimethyl sulphoxide | 1.99mPa.s |
| Glycerol | 1412mPa.s |
| Ethylene glycol | 16.1mPa.s |
| Propylene glycol | 42mPa.s |
| Water | 1.3mPa.s |

In preferred embodiments, non-aqueous solvents may be selected so that they have solubility parameters in the range of the non-aqueous solvents exemplified in the tables above. For example, $\delta_t$/MPa$^{1/2}$ may be from 26 to 50, such as 26.5 to 47.8. $\delta_d$/MPa$^{1/2}$ may be from 15 to 19, such as 15.6 to 18.1. $\delta_p$/MPa$^{1/2}$ may be from 8 to 16, such as 8.8 to 16. $\delta_h$/MPa$^{1/2}$ may be from 10 to 45, such as 10.2 to 42.3.

The non-aqueous solvent may be selected depending on the desired viscosity. For example, if a greater viscosity is desired, glycerol may be preferred. A suitable viscosity for a composition of the invention may be 100 mPa.s. or less at 20°C. In some embodiments, a suitable viscosity may be 75 mPa.s. or less at 20°C. In some embodiments, a suitable viscosity may be 75 mPa.s. or less at 20°C.

In some embodiments of compositions comprising a non-aqueous solvent, the compositions may comprise at least 2% by weight of the non-aqueous solvent. In some embodiments of compositions comprising a non-aqueous solvent, the compositions may comprise at least 5% by weight of the non-aqueous solvent. In some embodiments of compositions comprising a non-aqueous solvent, the compositions may comprise at least 10% by weight of the non-aqueous solvent. In other embodiments, the composition may comprise at least 20% by weight of the non-aqueous solvent. In other embodiments, the composition may comprise at least 25% by weight of the non-aqueous solvent. In other embodiments, the composition may comprise at least 50% by weight of the non-aqueous solvent. In some embodiments, the composition may comprise at least 75% by weight of the non-aqueous solvent. The amount of aqueous solvent may be varied depending on the intended application of the composition. For example, sprayable compositions, or compositions for use with an antibacterial wipe may comprise higher levels of the non-aqueous solvent, such that the compositions have a lower viscosity. In some embodiments, the amount of non-aqueous solvent in the composition may be 50-90%, by weight.

For some applications, it may be desirable to have compositions that comprise lower amounts of non-aqueous solvent, such as compositions for use in forming wound dressings. Consequently, some compositions may comprise a maximum amount of non-aqueous solvent. The maximum amount of non-aqueous solvent in the composition may be 50% by weight or less. In some embodiments, the amount of non-aqueous solvent in the composition may be 1-50, 5-50 or 10-50%, by weight.

If compositions are to be used to coat a substrate, such as a fabric, the weight of the composition is preferably at least 100g per square metre of the substrate. In other embodiments, the weight of the composition may be at least 200g per square metre of the substrate. In other embodiments, the weight of the composition may be at least 300g per square mere of the substrate.

Compositions comprising non-aqueous solvents may include added, or additional, water. The water may thus be additional to any water that may be present in the substance that includes the substrate for the enzyme. For example, but not according to the invention, if the substance in the composition of the invention is an unrefined natural substance, such as honey, then the composition may comprise additional water to that which is already present in the unrefined natural substance. Despite the additional water, the composition may be storage stable, as described herein. The additional water may not be available, or free, to allow the enzyme to convert the substrate because the non-aqueous solvent may bind or lock in the water. A non-aqueous solvent may thus act as a humectant. The non-aqueous solvent or humectant may reduce the water activity ($a_w$) of the composition.

Compositions of the invention may thus be said to comprise additional water and a humectant, wherein the humectant is additional to any humectant that may already be present in the substance. Unrefined natural substances, such as honey, may be considered to be humectants or comprise humectants. So, if the composition comprises an unrefined natural substance, the humectant is additional to the unrefined natural substance or any humectant which may be present in the unrefined natural substance. However, these compositions are not according to theinvention.

In some embodiments, the humectant is not a non-aqueous solvent. However, in preferred embodiments, the humectant is a non-aqueous solvent.

Inclusion of an unrefined natural substance (such as honey) in a composition may render the composition viscous and limit its range of applications. However, such a composition is not according to the invention. Inclusion of both a non-aqueous solvent or humectant, and added water in the composition may reduce the viscosity of the composition, and less viscous compositions may be beneficial if, for example, the composition is to be readily sprayable. An example of a sprayable composition comprising honey, a non-aqueous solvent and added water is shown in Example 40. A suitable viscosity for a composition of the invention may be 100 mPa.s. or less at 20°C. In some embodiments, a suitable viscosity may be 75 mPa.s. or less at 20°C. In some embodiments, a suitable viscosity may be 50 mPa.s. or less at 20°C.

So, according to the invention there is provided a storage-stable composition for generating antimicrobial activity comprising an enzyme that is able to convert a substrate to release hydrogen peroxide, a substance that includes a substrate for the enzyme, a non-aqueous solvent, and water. The water is additional to any water that may be present in the substance and the composition comprises less than 10% by weight of water. The enzyme is additional to any enzyme activity able to convert the substrate to release hydrogen peroxide that may be present in the substance. The composition does not comprise honey or any other unrefined natural substance.

There is provided a storage-stable composition for generating antimicrobial activity comprising an enzyme that is able to convert a substrate to release hydrogen peroxide, a substance that includes a substrate for the enzyme, a humectant, and water. The water is additional to any water that may be present in the substance. The enzyme is additional to any enzyme activity able to convert the substrate to release hydrogen peroxide that may be present in the substance. The humectant is additional to the substance or any humectant that may be present in the substance. The composition comprises less than 10% by weight of water and does not comprise honey or any other unrefined natural substance.

In compositions of the invention comprising a humectant, the humectant may comprise (or consist of) a mixture of more than one humectant. Reference herein to "humectant" thus includes one or more, or at least one, humectant. However, in some embodiments, the humectant may comprise (or consist of) only one humectant.

The relative amounts of the humectant or non-aqueous solvent, and the additional water, in a composition of the invention, may be selected such that the composition does not comprise sufficient free water to allow the enzyme to convert the substrate. When such a composition is contacted with even more water, for example if the composition is diluted or if the composition comes into contact with fluid from a wound, there may be sufficient free water for the enzyme to convert the substrate and produce hydrogen peroxide.

In compositions of the invention comprising humectant or non-aqueous solvent, and additional water, the amount of additional water in the composition may be at least 5%, by weight.

In compositions of the invention comprising humectant or non-aqueous solvent, and additional water, the total amount of water in the composition (i.e., the amount of water which may already be present in the substance combined with the amount of additional water) may be at least 15%, by weight. In other embodiments, the total amount of water may be at least 20%, by weight. The total amount of water in the composition may be 50%, or less, by weight. In other embodiments, the total amount of water may be 40%, or less, by weight. In some embodiments, the total amount of water may be 15-50% by weight, or 20-40% by weight.

In compositions of the invention comprising humectant or non-aqueous solvent, and additional water, the mole fraction of water in the composition may be 50 to 75%, by weight. For example, the mole fraction of water in the composition may be 60 to 70%, by weight.

In compositions of the invention comprising additional water, and humectant or non-aqueous solvent, the water activity ($a_w$) may be less than 0.6, preferably less than 0.5.

In compositions of the invention comprising additional water, and humectant or non-aqueous solvent, the amount of humectant or non-aqueous solvent may be at least 30% by weight. In other embodiments, the amount of humectant or non-aqueous solvent may be at least 40% by weight. In some embodiments, the amount of humectant or non-aqueous solvent may be at least 50% by weight. The amount of humectant or non-aqueous solvent may be 75% or less, by weight. The amount of humectant or non-aqueous solvent may be 60% or less by weight. In some embodiments, the amount of humectant or non-aqueous solvent may be 30-75% or 40-60% by weight.

In compositions of the invention comprising humectant or non-aqueous solvent, and additional water, the humectant or non-aqueous solvent may be selected from ethylene glycol, propylene glycol, glycerol or dimethyl sulphoxide. In some embodiments, the humectant or non-aqueous solvent is glycerol. The humectant or non-aqueous solvent may comprise one or more of ethanol, dimethyl sulphoxide, glycerol, ethylene glycol or propylene glycol. In some embodiments, the humectant or non-aqueous solvent may comprise two of ethanol, dimethyl sulphoxide, glycerol, ethylene glycol or propylene glycol. In some embodiments, the humectant or non-aqueous solvent may comprise three of ethanol, dimethyl sulphoxide, glycerol, ethylene glycol or propylene glycol. In some embodiments, the humectant or non-aqueous solvent may comprise four of ethanol, dimethyl sulphoxide, glycerol, ethylene glycol or propylene glycol.

In some embodiments, compositions that comprise a non-aqueous solvent comprise substantially no polymer, for example less than 1%, 0.5%, 0.1 % or 0.01 % by weight of the polymer.

Embodiments comprising a non-aqueous solvent but are not according to the invention are the following:

i) Alcoholic Gel

| | |
|---|---|
| Carbopol 940 | 0.3 % |
| Triethanolamine | 0.4 % (needed for pH and stability control) |
| Active honey | 65% |
| Ethanol | 25.0 % |
| water qs | |

wherein "Active honey" is honey with added glucose oxidase

ii) Non-Aqueous spray

| | |
|---|---|
| Active honey | 70% |
| Proplyene Glycol | 30% |

iii) Fungal Nail Treatment

Plaster with Active Honey in a foam well
Hydroxypropylcellulose
Glycerol
Isopropyl Alcohol
Citric Acid
Monohydrate

iv) <u>Throat spray</u>
Honey and glycerol, preferably comprising 5-20% honey
v) <u>Cream</u>

| | |
|---|---|
| Honey | 15% |
| Carbomer | 2.63% |
| Dimethicone | 0.13% |
| Disodium Lauryl Sulphosuccinate | 0.05% |
| Disodium Edetate | 0.13% |
| Glycerol | 5.26% |
| Silica Colloidal Hydrated | 0.33% |
| Poloxamer | 0.26% |
| Sodium Hydroxide | 0.41% |
| Purified Water | 85.03% |

vi) <u>Powder</u>

Active honey

Maltodextrin

Ibuprofen

Microcrystalline Cellulose (CMC)

Polyvinyl Pyrrolidone (PVP)

Specific examples of compositions comprising non-aqueous solvent are described in Example 39, a reference example which is not according to the invention.

In some embodiments of the invention, it may be important that fibers or compositions of the invention are used at concentrations, or for periods of time, that are antimicrobial (if necessary, once the fiber or composition is in contact with sufficient free water for hydrogen peroxide to be released) but not substantially cytotoxic or cytostatic.

Surgihoney is the name given to some embodiments of compositions of the invention, described in more detail in Example 20 below. Example 41 below describes the effects of different concentrations of different preparations of Surgihoney on mast cells. Mast cells are sentinel cells located just underneath the epithelium in tissues with close contact to the external environment, such as the nasal mucosal tissues. They play an important role in recognition of pathogens and modulation of immune responses. Example 41 describes the effects of different concentrations of Surgihoney on cells of the human mast cell line HMC-1. However, these reference examples are not according to the invention.

The results in Example 41 indicate that it may be beneficial to use Surgihoney (particularly S1, S2, or S3 preparations, preferably S2 or S3 preparations), or other compositions of the invention, at concentrations of less than 100 g/L, preferably 40g/L or less, 1 0g/L or less, or 1 g/L or less, when treating a human or animal subject with the composition. This may be particularly beneficial where mast cells are present, such as epithelium in tissues with close contact to the external environment, for example nasal mucosal tissues. Such concentrations may be particularly effective for treatment of nasal conditions, such as sinusitis and rhinosinusitis; respiratory tract infections, such as upper respiratory tract infections (e.g. tonsillitis, laryngitis and sinusitis or lower respiratory tract infections (e.g. bronchitis, pneumonia, bronchiolitis and tuberculosis); chronic obstructive pulmonary disease; and cystic fibrosis.

It may also be beneficial to limit contact of Surgihoney, or the compositions of the invention, with human or animal cells, to less than 24 hours, preferably less than 6 hours, or less than 3 hours, when treating a human or animal subject with the composition. This may be particularly beneficial where mast cells are present, such as epithelium in tissues with close contact to the external environment, for example nasal mucosal tissues. Such limited contact may be particularly effective for treatment of nasal conditions, such as sinusitis and rhinosinusitis; respiratory tract infections, such as upper respiratory tract infections (e.g. tonsillitis, laryngitis and sinusitis or lower respiratory tract infections (e.g. bronchitis, pneumonia, bronchiolitis and tuberculosis); chronic obstructive pulmonary disease; and cystic fibrosis.

Preferred embodiments of the invention, and preferred compositions that can be used in making fibers, dressings and compositions of the invention, are now described, by way of example only, with reference to the accompanying drawings in which:

Figure 1 shows the results from well diffusion assays for the antimicrobial effect of pasteurized Ulmo honey with different amounts of added glucose oxidase;

Figure 2 shows the results from well diffusion assays for the antimicrobial effect of compositions with different amounts of enzyme in comparison with the effects of different phenol standards;

Figure 3 shows photographs of the results from well diffusion assays for the antimicrobial effect of pasteurized Tineo honey with added glucose oxidase;

Figure 4 shows the results from well diffusion assays for the antimicrobial effect of different compositions;

Figure 5 shows the results from well diffusion assays for the antimicrobial effect of compositions that include different enzyme preparations;

Figure 6 shows the results from well diffusion assays for the antimicrobial effect of compositions after storage for 60 or 90 days;

Figure 7 shows the results from well diffusion assays for the antimicrobial effect of powdered formulations of compositions;

Figure 8 shows the results from well diffusion assays for the antimicrobial effect of a sterilised honey-based composition compared with Manuka UMF 25+ honey;

Figure 9 shows the effect of treatment of an infected toe using a sterilised honey-based composition;

Figure 10 shows the effect of treatment of a toe ulcer using a sterilised honey-based composition;

Figure 11 shows the effect of treatment of a foot ulcer using a sterilised honey-based composition;

Figure 12 shows the effect of treatment of a foot ulcer using a sterilised honey-based composition;

Figure 13 shows the effect of treatment of a traumatic leg wound using a sterilised honey-based composition;

Figure 14 shows the effect of treatment of an infected leg wound using a sterilised honey-based composition;

Figure 15 shows the effect of treatment of a leg ulcer using a sterilised honey-based composition;

Figure 16 shows the effect of treatment of a pressure ulcer using a sterilised honey-based composition;

Figure 17 shows the effect of treatment of a infection surrounding the entry point of a catheter using a sterilised honey-based composition;

Figure 18 shows time kill curves for Surgihoney 1 (S1), Surgihoney 3 (S3), and Medihoney (MH) for different test organisms: (a) *Staphylococcus aureus;* (b) *Methicillin-resistant Staphylococcus aureus* (MRSA); (c) *E.coli*; (d) vancomycin resistant enterococcus (VRE); (e) *Pseudomonas aeruginosa;* (f) *Klebsiella*; (g) *E.coli* ESBL; (h) *Enterococcus faecalis*;

Figure 19 shows the results of a plaque assay to test anti-viral activity of S1 and S2 Surgihoney;

Figure 20 shows photographs of the results of use of S1 Surgihoney to treat a pressure sore, in which: (a) shows day 1 of treatment; and (b) shows day 30 of treatment;

Figure 21 shows different hydrogen peroxide production rates for Surgihoney and two modified prototypes, PT1 and PT2; and

Figure 22 shows the relationship between phenol activity and maximum hydrogen peroxide activity in modified honeys, Surgihoney, PT1 and PT2.

Figure 23 shows the effect of: (A) neat S1 Surgihoney; or (B) serial dilutions of S1 Surgihoney on formation of biofilms by *Pseudomonas aeruginosa* strain PA01, and clinical burn wound isolate 1054; and (C) neat S1 Surgihoney; or (D) serial dilutions of S1 Surgihoney on formation of biofilms by *Acinetobacter baumannii* control strain AYE, and ACI clone NCTC_13420 (C59);

Figure 24 shows the effect of neat Manuka honey on formation of biofilms by: (A) *Pseudomonas aeruginosa* strain PA01, and clinical burn wound isolate 1054; or (B) *Acinetobacter baumannii* control strain AYE, and ACI clone NCTC_13420 (C59); and (C) serial dilutions of Manuka honey on formation of biofilms by *Pseudomonas aeruginosa* strain PA01, and clinical burn wound isolate 1054 (upper part of Figure 2(C)), or *Acinetobacter baumannii* control strain AYE, and ACI clone NCTC_13420 (C59) (lower part of Figure 2(C));

Figure 25 shows the effect of neat and serial dilutions of S1, S2, and S3 Surgihoney on formation of biofilms by: (A) *Pseudomonas aeruginosa* strain PA01, and clinical burn wound isolate 1054; and (B) *Acinetobacter baumannii* control strain AYE, and ACI clone NCTC_13420 (C59);

Figure 26 shows the effect of Surgihoney preparations S1, S2, and S3, and Manuka honey (MH), on biofilm formation by a biofilm-producing isolate of *Pseudomonas aeruginosa* (PS_1586);

Figure 27 shows the effect of Surgihoney preparations S1, S2, and S3, and Manuka honey (MH), on biofilm formation by a biofilm-producing isolate of *Pseudomonas aeruginosa* (PA_6749);

Figure 28 shows the effect of Surgihoney preparations S1, S2, and S3, and Manuka honey (MH), on biofilm formation by a biofilm-producing isolate of *Acinetobacter baumannii* (ACI_19606);

Figure 29 shows the effect of Surgihoney preparations S1, S2, and S3, and Manuka honey (MH), on biofilm formation by a biofilm-producing isolate of *Acinetobacter baumannii* (ACI_C60);

Figure 30 shows the effect of Surgihoney S1, S2, and S3 preparations, and Manuka honey (MH), on the prevention or reduction of the seeding of pre-formed biofilms produced by *Acinetobacter baumannii* ACI_C59;

Figure 31 shows the effect of Surgihoney S1, S2, and S3 preparations, and Manuka honey (MH), on the prevention or reduction of the seeding of pre-formed biofilms produced by *Acinetobacter baumannii* ACI_AYE;

Figure 32 shows the effect of Surgihoney S1, S2, and S3 preparations on prevention of biofilm formation by *Pseudomonas aeruginosa* (control strain PA01) in comparison with deactivated Surgihoney (DE), Manuka honey (MH: 'Comvita Manukacare 18+'), acetic acid (AA), and several commercially available wound dressings, and wound creams; and

Figure 33 shows the effect of Surgihoney S1, S2, and S3 preparations on prevention of biofilm formation by *Acinetobacter baumannii* (control strain AYE) in comparison with deactivated Surgihoney (DE), Manuka honey (MH: 'Comvita Manukacare 18+'), acetic acid (AA), and several commercially available wound dressings, and wound creams;

Figure 34 shows the activity of electrospun dressings containing Surgihoney (right-hand side of each nutrient agar plate) against *Escherichia coli*, compared to control electrospun dressings (left-hand side of each nutrient agar plate);

Figure 35 shows the activity of electrospun dressings containing Surgihoney (right-hand side of each nutrient agar plate) against *Staphlyococcus aureus*, compared to control electrospun dressings (left-hand side of each nutrient agar plate);

Figure 36 shows an electrospun Surgihoney dressing (left-hand side) and an electrospun control dressing (right hand-side), and in which the hydrogen peroxide generating capability of the electrospun Surgihoney dressing has been confirmed by a peroxide test kit;

Figure 37 shows the results of an assay for the cytotoxic activity of Surgihoney;

Figure 38 shows hydrogen peroxide production by dried Surgihoney granules, and dried Surgihoney that has been dissolved in water to form a solution;

Figure 39 shows hydrogen peroxide production by the solution of Surgihoney of Figure 38 after various time periods, up to four days; and

Figure 40 shows the effect on survival of HMC-1 mast cells of 3, 6 and 24 hour culture with different concentrations of S1, S2, and S3 preparations of Surgihoney.

**Example 1** (Reference Example)

This example describes a preferred process for production of a storage-stable composition for use in the invention, and dilution of the storage-stable composition to release hydrogen peroxide.

Process for Manufacture of "Activated" Honey

Honey is heated for 2 minutes to 80°C using a heat exchanger (a lower temperature could be used if desired, suitably at least 60°C, provided this is sufficient to inactivate catalase). The purpose of this heating process is to pasteurise the honey, reduce its viscosity so that it can be filtered to remove any wax particles and bee wings that may be in the honey post harvest, and inactivate any catalase in the honey that would affect the efficient production of hydrogen peroxide.

The pasteurised honey is then filtered, and left to cool naturally to normal hive temperatures (35-40°C). Glucose oxidase is then added at a low level (equivalent to the level found normally in honey) to replace the glucose oxidase naturally found in the honey but which has been inactivated by the pasteurisation process. The filtered pasteurised honey is fairly liquid at 35-40°C so it can easily be mixed with the glucose oxidase. There is, however, no other reason why the mix could not be done at room temperature. The resultant "activated" honey is then stored at ambient temperature.

Apart from the replacement of the inactivated glucose oxidase there is no modification to the composition of the natural honey. There is no detectable hydrogen peroxide in the "activated" honey.

Dilution of "Activated" Honey

Following dilution of the "activated" honey, hydrogen peroxide is released after a lapse of time, as free water becomes available and the glucose oxidase starts to convert the glucose present in the honey. The hydrogen peroxide level is less than 2 mmol/litre but is released for an extended period.

**Example 2** (Reference Example)

This example describes the results of tests demonstrating the antimicrobial effect of activated Ulmo honey. The honey is described as "activated" if it contains added glucose oxidase.

Well Diffusion Assay

*Staphylococcus aureus* (NCIMB 9518) was grown on nutrient agar or in nutrient broth.

Antibiotic diffusion agar plates were inoculated with culture by swabbing overnight culture onto the surface of agar plates. Plates were allowed to stand at room temperature for 15 minutes. Wells 7mm diameter were bored into the surface of the agar. Two hundred microlitres of sample (phenol standard, or honey) was placed into each well.

Plates were incubated for 16 hrs and zones of inhibition were measured using a dial calliper (+/-0.1mm). The diameter of zones, including the diameter of the well, were recorded.

Phenol standards were prepared by diluting phenol in purified water at the required concentration. For example, a 10% phenol standard was prepared by diluting 1g of phenol in 9g of purified water. The standards were stored at 30°C and shaken before use.

Honey

Honey: Pasteurised Ulmo honey.

Non-activated honey (i.e. honey to which no glucose oxidase had been added) was used as a control.

Enzyme Preparation

Glucose oxidase: medical device grade material, non food grade, from GMO *Aspergillus niger*, supplied by Biozyme UK, activity 240iu/mg. Initially 0.5% w/w enzyme was used, but this could be reduced to 0.005% w/w enzyme to achieve a 20% phenol standard equivalent.

Detection of Hydrogen peroxide

Peroxide test from Merckoquant: Cat. No. 1.10011.0002 Measuring range/colour-scale graduation mg/l H2O2 0.5 - 2 - 5 - 10 - 25.

Procedure determination in aqueous solutions: dissolve honey in water 50/50 w/w. Immerse the reaction zone of the test strip in the measurement sample (15-30°C) for 1 second. Allow excess liquid to run off via the long edge of the strip onto an absorbent paper towel and after 15 seconds (Cat. No. 110011) or after 5 seconds (Cat. No. 110081) determine with which colour field on the label the colour of the reaction zone coincides most exactly. Read off the corresponding result in mg/l $H_2O_2$ or, if necessary, estimate an intermediate value.

To determine that no endogenous hydrogen peroxide is available in the honey, dissolve in methanol 50/50 w/w. Determination in organic solvents (readily volatile ethers): Immerse the reaction zone of the test strip in the measurement sample (15-30°C) for 1 second. After the solvent has evaporated (gently fan the strip back and forth for 3-30 seconds) immerse the reaction zone in distilled water for 1 second and allow excess liquid to run off via the long edge of the strip onto an absorbent paper towel or gently blow on the reaction zone four times, for 3-5 seconds each time. After 15 seconds (Cat. No. 110011) or after 5 seconds (Cat. No. 110081) determine with which colour field on the label the colour of the reaction zone coincides most exactly. Read off the corresponding result in mg/l $H_2O_2$ or, if necessary, estimate an intermediate value.

Results

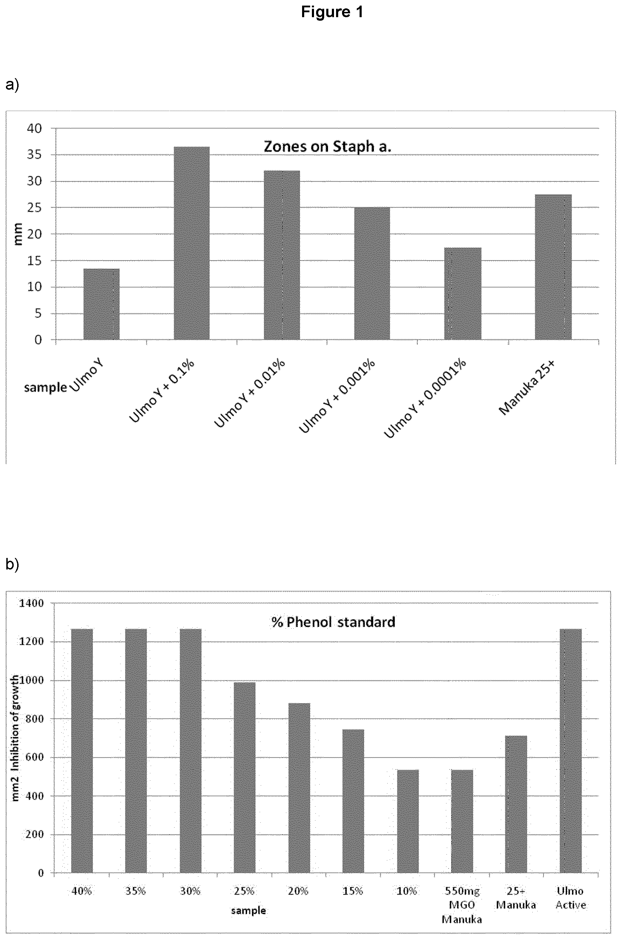

Figure 1a shows the results from a well diffusion assay in which samples containing pasteurized Ulmo honey, pasteurized Ulmo honey to which different amounts of glucose oxidase enzyme preparation had been added (0.1%, 0.01%, 0.01%, 0.0015, or 0.0001% w/w), or Manuka UMF 25+ honey were added to the wells of an agar plate inoculated with *Staphylococcus aureus.* The results show the diameter of the zone of inhibition recorded for each sample.

The antimicrobial effect of activated honey containing at least 0.001% w/w glucose oxidase (240 iu/mg) on *Staphylococcus aureus* was equivalent to that of Manuka 25+ honey.

The effect of the activated honey is bactericidal.

Figure 1b shows the results from a well diffusion assay in which a sample containing pasteurized Ulmo honey to which 0.5% w/w glucose oxidase enzyme preparation had been added was compared with samples of MGO Manuka honey, Manuka 25+ honey, and a range of different phenol standards (10%-40%). The results show the area of the zone of inhibition for each sample.

The results show that the effect of the activated Ulmo honey was equivalent to a 30% phenol standard, and was over twice as effective as MGO Manuka honey, and nearly twice as effective as Manuka UMF 25+ honey.

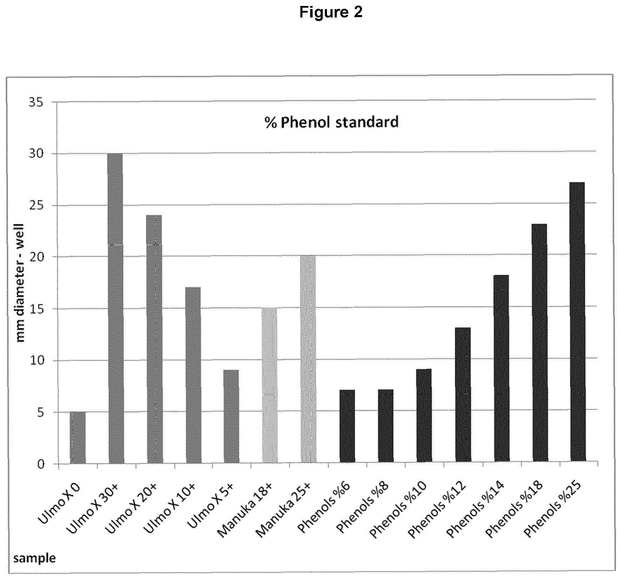

Figure 2 demonstrates that the potency of the activated honey can be tailored to suit requirements by addition of a suitable amount of enzyme.

**Example 3** (Reference Example)

This example describes the results of tests demonstrating the antimicrobial effect of activated Tineo honey. The honey is described as "activated" if it contains added glucose oxidase.

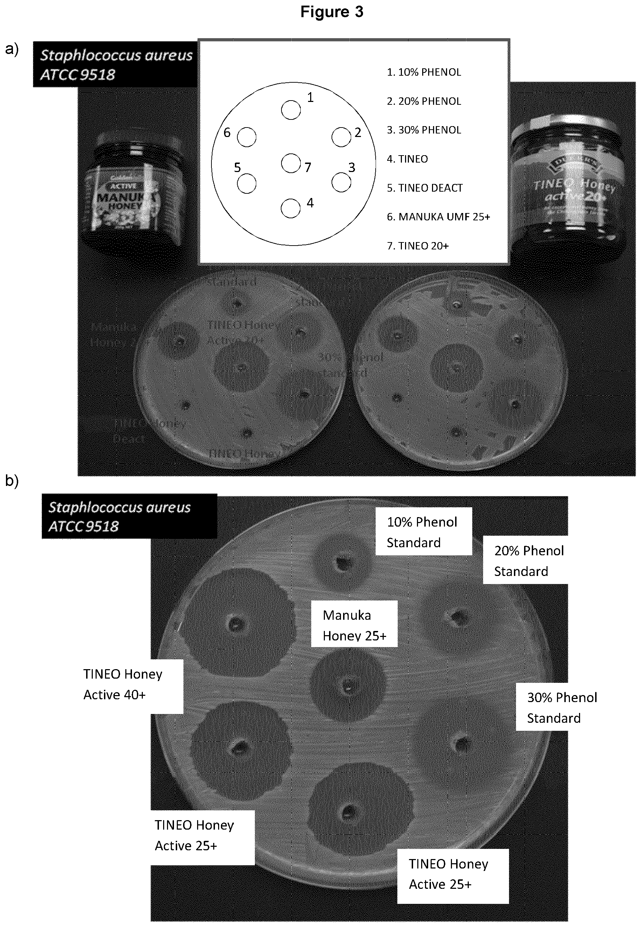

Well diffusion assays as described in Example 2 were carried out using samples of TINEO honey and different phenol standards. Figure 3a is a photograph of duplicate agar plates showing zones of inhibition of *Staphylococcus aureus* caused by the different samples. The figure includes a representation showing the layout of the samples in the wells of the agar plate:

1. 10% Phenol Standard

2. 20% Phenol Standard

3. 30% Phenol Standard

4. TINEO Honey (pasteurized TINEO honey containing no added glucose oxidase)

5. TINEO Deact (pasteurized TINEO honey, with further heat deactivation)

6. Manuka UMF 25+

7. TINEO 20+ (pasteurized TINEO honey with added glucose oxidase, 0.005% Biozyme enzyme)

Figure 3a shows that neither TINEO honey alone, nor TINEO deactivated honey caused inhibition of *Staphylococcus aureus* growth. The effect of TINEO honey with added glucose oxidase (TINEO 20+) was greater than the effect of Manuka UMF 25+, and equivalent to the 30% phenol standard.

Figure 3b is a photograph of an agar plate showing zones of inhibition of *Staphylococcus aureus* caused by different samples, which were arranged as shown below:

1. 10% Phenol Standard

2. 20% Phenol Standard

3. 30% Phenol Standard

4. TINEO Honey Active 25+ (0.005% enzyme w/w)

5. TINEO Honey Active 25+ (0.005% enzyme w/w)

6. TINEO Honey Active 40+ (0.05% enzyme w/w)

7. Manuka Honey UMF 25+

Figure 3b shows that the TINEO Honey Active 25+ and 40+ samples were more effective in inhibiting growth of *Staphylococcus aureus* than the Manuka Honey 25+ sample.

**Example 4** (Reference Example)

This example describes the results of tests demonstrating the antimicrobial effect of activated Ulmo and Tineo honey.

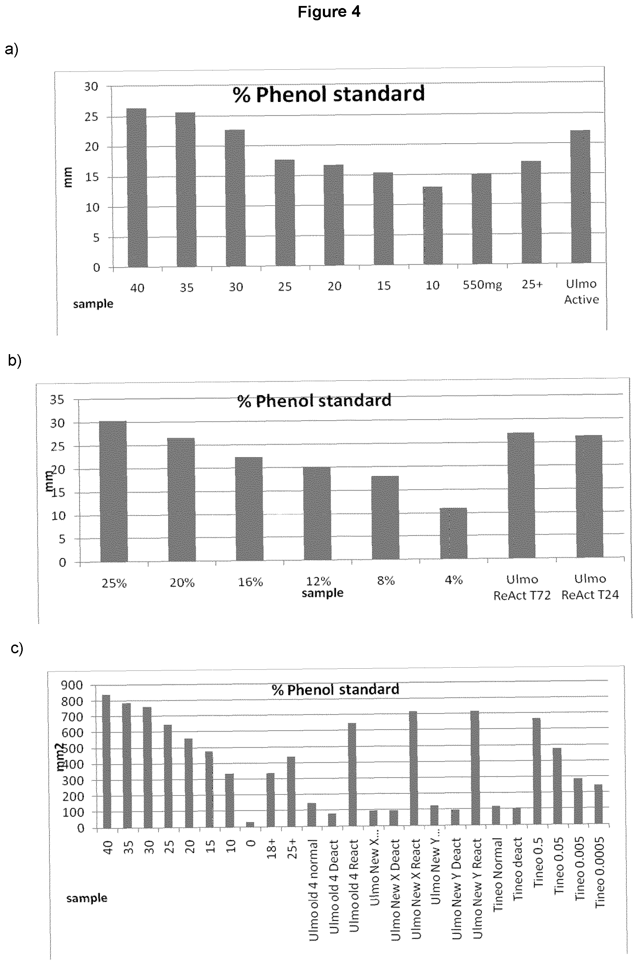

Figure 4a shows the results of three repeats from a well diffusion assay, carried out as described in Example 2, measuring the zones of inhibition of growth of *Staphylococcus aureus* following 24hrs incubation. Samples of phenol in DI water at a range of concentrations from 40-10%, and samples of 2 types of Manuka (Manuka UMF 25+, Manuka 550MGO, and activated Ulmo honey with 0.01% w/w Biozyme glucose oxidase (as in Example 2).

Figure 4b shows the results from a further well diffusion assay. Glucose oxidase (Biozyme preparation as described in Example 2) was added to pasteurized Ulmo honey (0.005% enzyme w/w), and stored at 40°C for 24 or 72 hours. Samples from the stored activated honeys were then tested for activity against *Staphylococcus aureus* in a well diffusion assay, as described in Example 2, and compared with a range of phenol standards from 4-25%. Figure 4b shows that the activated honey was stable when stored at 40°C for 72 hours, and that the activated honey was equivalent to the 20% phenol standard in its effectiveness against *Staphylococcus aureus.*

Figure 4c shows the results from a further well diffusion assay in which the activity of new and old batches (the old batch was received approximately four months before the new batch) of pasteurized Ulmo honey (Biozyme preparation as described in Example 2, 0.5% w/w), and Tineo honey with increasing amounts of added glucose oxidase (Biozyme preparation as described in Example 2, 0.0005-0.5% w/w) were compared with Manuka 18+ and 25+ UMF honey, and a range of phenol standards from 10-40%. In Figure 4c, "normal" refers to pasteurized honey as received, "Deact" refers to pasteurized honey that has been further heat deactivated, and "React" refers to pasteurized honey that has been further heat deactivated, prior to addition of glucose oxidase. Thus, the Ulmo honey was tested without ("Ulmo old 4 normal", "Ulmo New X", and "Ulmo New Y") and with added glucose oxidase. Figure 4c shows the area of the zones of inhibition of *Staphylococcus aureus.* It can be seen that the old batch of pasteurized Ulmo honey ("Ulmo old 4 React") retained equivalent activity to the new pasteurized batches ("Ulmo New X React" and "Ulmo New Y React"). All of these batches showed approximately two-fold higher activity than Manuka honey 18+, and higher activity than Manuka honey 25+. Adding increasing amounts of glucose oxidase to the pasteurized Tineo honey increased the activity of the honey.

In this test, 0.005% w/w enzyme was approximately equivalent to the 10% phenol standard, 0.05% w/w enzyme was approximately equivalent to the 15% phenol standard, and 0.5% w/w enzyme was approximately equivalent to the 25% phenol standard. The Manuka 18+ honey was equivalent to the 10% phenol standard, and the Manuka 25+ honey showed activity that was intermediate between the 10% and 15% phenol standard.

**Example 5** (Reference Example)

In this example, the effect of different glucose oxidase enzyme preparations was tested with pasteurized Tineo honey. The enzyme preparations used were the Biozyme preparation as described in Example 2, and a food grade, non-GMO source, glucose oxidase from ANHUI MINMETALS DEVELOPMENT I/E CO., LTD (referred to as the "Anhui" enzyme preparation below).

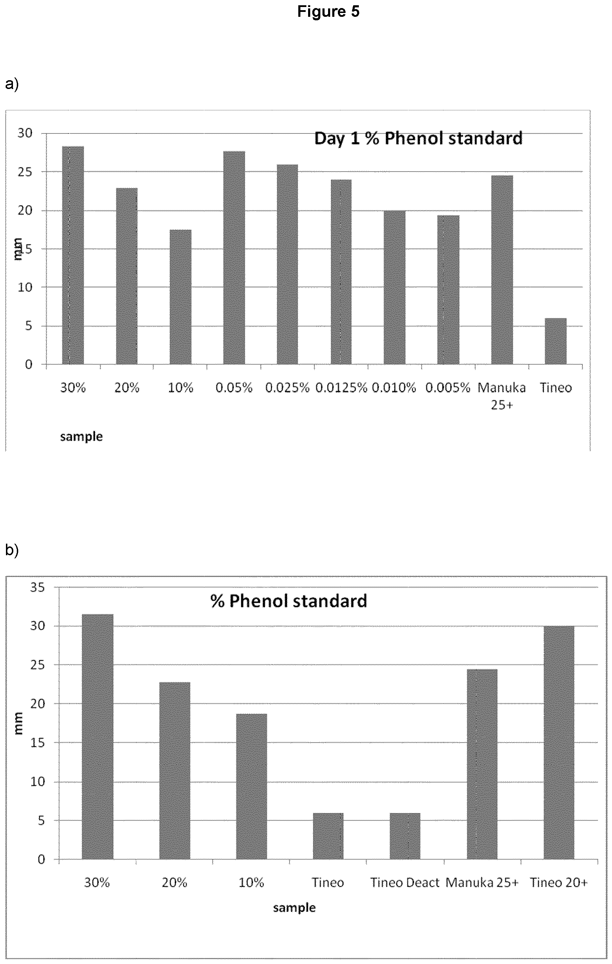

Figure 5a shows the results from a well diffusion assay carried out as described in Example 2, in which samples of pasteurized Tineo honey containing either no enzyme, or 0.005-0.05% w/w Anhui enzyme were compared with different phenol standards (10%, 20%, 30%) and a sample of Manuka honey UMF 25+. The results show that increasing the amount of enzyme increases the effectiveness of the activated honey against *Staphylococcus aureus.* 0.0125% w/w enzyme was approximately equivalent to the 20% phenol standard and the Manuka honey sample, and 0.05% w/w enzyme was approximately equivalent to the 30% phenol standard.

Figure 5b shows the results from a well diffusion assay carried out as described in Example 2, in which different amounts of the enzyme was added to samples of a commercial batch of pasteurized Tineo honey. The "Tineo" sample is the pasteurized honey with no added glucose oxidase. The "Tineo Deact" sample is the pasteurized honey which has been further heat treated, and contains no added glucose oxidase. The "Tineo 20+" sample contains 0.005% w/w of the Biozyme glucose oxidase. The results show that the Tineo sample has no activity against *Staphylococcus aureus.* The Tineo 20+ sample was more effective against *Staphylococcus aureus* than the Manuka 25+, and was intermediate between the 20% and 30% phenol standards.

**Example 6** (Reference Example)

This example describes the results of tests of the stability of activated honeys stored for 60 or 90 days.

Glucose oxidase enzyme from Biozyme, as described in Example 2, was added to pasteurized Ulmo or Tineo honey at 0.005% w/w, and the resulting mixtures were stored at 37°C for two months (Ulmo honey samples) or at room temperature for 90 days (Tineo honey samples). The amount of enzyme used corresponds to the amount that would be used for a commercial food product.

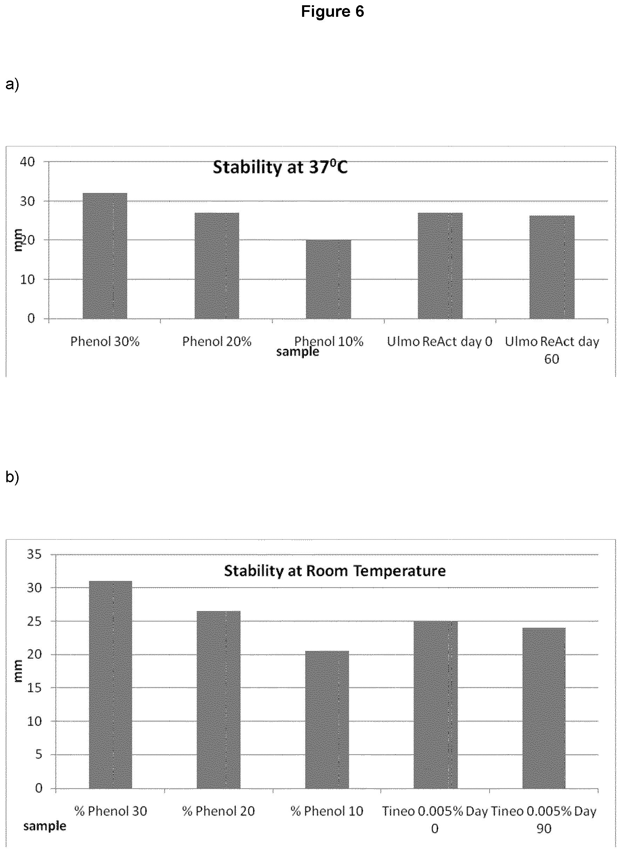

After storage, the samples were tested in a well diffusion assay against *Staphylococcus aureus* carried out as described in Example 2. The stored samples were compared with 10%, 20%, and 30% phenol standards. Figure 6a shows the results for the Ulmo honey samples, and Figure 6b shows the results for the Tineo honey samples. There was no apparent loss of activity for the storage period for either type of honey.

**Example 7** (Reference Example)

This example describes the results of tests of the antimicrobial activity of a powdered activated honey in which glucose oxidase enzyme in powder form is added to a powdered honey.

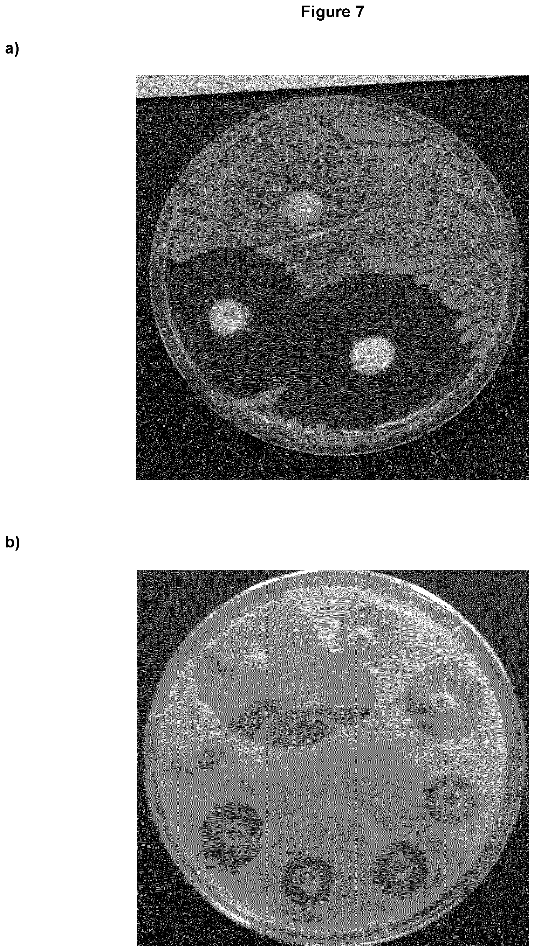

Powdered honey was obtained from Honi Bake from ADM Specialty Ingredients. 0.1% w/w Biozyme glucose oxidase (as described in Example 2) was added to the powdered honey. The antimicrobial activity of the mixture was tested in a well diffusion assay against *Staphylococcus aureus* as described in Example 2. The mixture was added to two different wells of the agar plate. A control was used with honey powder only. The results are shown in Figure 7a. The control well showed no activity, but the activated honey showed clear zones of inhibition.

Figure 7b shows the results of another well diffusion assay in which a different powdered honey was mixed with powdered enzyme from ANHUI MINMETALS DEVELOPMENT I/E CO., LTD (Example 5). Sample well 24a is the honey powder only, and sample well 24b is honey powder mixed with 1% w/w enzyme. Again, a clear zone of inhibition can be seen.

**Example 8** (Reference Example)

This example describes sterilisation of a composition that comprises unpasteurised honey and added purified glucose oxidase.

Ten sealed sachets each containing 50g of the composition were gamma irradiated at a target dose of 11.6-14.2 kGy (the dose was 13.1-13.6 kGy as determined by dosimeters), and subsequently individually tested for sterility.

For the sterility testing, all work was carried out in a cleanroom under a laminar flow. 10g of the same was added to 100ml of sterile Tryptone Soya Broth (TSB: pancreatic digest of casein, 17g/L, papaic digest of soya bean meal, 3g/L, sodium chloride, 5g/L, dibasic potassium phosphate, 2.5g/L, glucose 2.5g/L, pH 7.3 ± 0.2) and shaken to mix, then transferred to a sterile container. A further 100ml of TSB was added to remove any sample residue and added to the same container. TSB was added to the sample and incubated at 30°C ± 2°C for a minimum of 14 days and inspected for signs of microbial growth. Positive controls were performed on all media before testing commenced.

One positive result was noted after the full incubation period. Substantiation of 35 kGy as a sterilization dose was accepted.

Testing of sachets before and after sterilisation by gamma irradiation using a well diffusion assay similar to the assay described in Example 2 confirmed that the effect of irradiation on the antimicrobial activity of the composition was negligible. There was no observable reduction in activity level following sterilisation.

**Example 9** (Reference Example)

This example describes the results of tests demonstrating the antimicrobial effect of a composition that comprises unpasteurised honey and added purified glucose oxidase (referred to as "Surgihoney") that has been sterilised using gamma irradiation, compared with Manuka UMF 25+ honey, and a heat-deactivated honey (Non Active Honey).

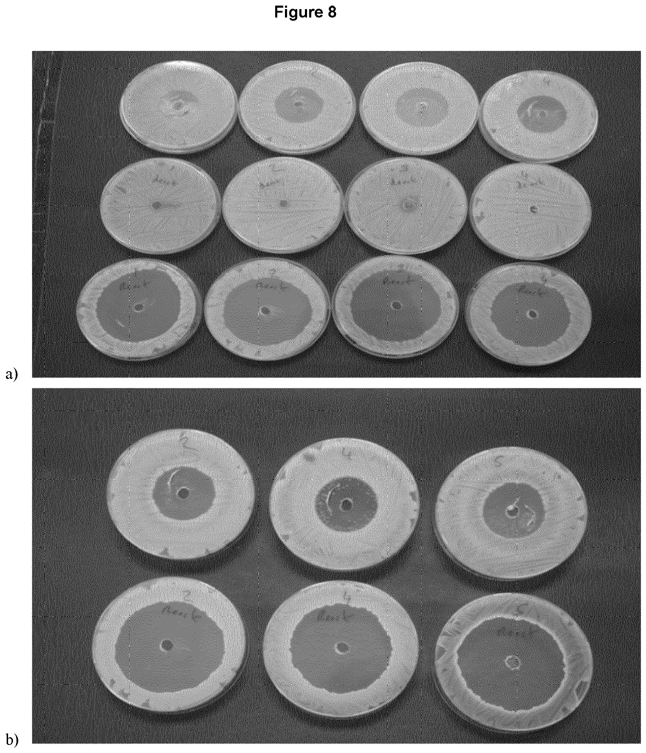

Well diffusion assays as described in Example 2 were carried out using samples of Manuka honey UMF 25+, Non Active Honey, and Surgihoney. Figure 8(a) is a photograph of agar plates showing zones of inhibition of *Staphylococcus aureus* (ATCC 9518) caused by the different samples. Manuka Honey UMF 25+ is in the top row of plates in the figure, Non Active Honey is in the middle row, and Surgihoney is in the bottom row. Figure 8(b) shows plates treated with Manuka UMF 25+ honey (top row) and plates treated with Surgihoney (bottom row).

The results clearly show that Surgihoney retains significant antimicrobial activity against *Staphylococcus aureus* after sterilisation, and that the Surgihoney was more effective against *Staphylococcus aureus* than Manuka honey UMF 25+.

**Example 10** (Reference Example)

Stability testing of a composition that comprises unpasteurised honey and added purified glucose oxidase that has been sterilised using gamma irradiation (at a minimum dose of 35 kGy).

Accelerated aging techniques are based on the assumptions that the chemical reactions involved in the deterioration of materials follow the Arrhenius reaction rate function. This function states that a 10°C increase or decrease in the temperature of a homogenous process, results in approximately a two times or ½ time change in the rate of a chemical reaction. For example, at 55 °C, 5.3 weeks is equivalent to 1 year on-the-shelf, and at 55 °C, two years would be equivalent to 10.6 weeks and five years would be 26.5 weeks.

Products from two different production batches were used for this study. Products were sachets that contained 10g of the composition. The sachets had been sterilized at a minimum dose of 35kGy gamma irradiation. Samples were stored, under accelerated aging conditions, at 55°C (± 2°C).

The relationship between real time and accelerated ageing is as follows:

Table 2

| Real Time equivalent (Months) | Accelerated Aging @ 55°C | |
| --- | --- | --- |
| | Days | Weeks |
| 3 | 9 | 1.3 |
| 6 | 19 | 2.6 |
| 12 | 37 | 5.3 |
| 24 | 74 | 10.6 |

(continued)

| Real Time equivalent (Months) | Accelerated Aging @ 55°C | |
| --- | --- | --- |
| | Days | Weeks |
| 36 | 111 | 15.9 |
| 48 | 148 | 21.2 |
| 60 | 185 | 26.5 |

Testing, test intervals and samples required

Samples from each batch were tested at the same time intervals. The following table summarises the tests performed, and the total number of samples required at each time point for each batch.

Table 3

| Test | Time Intervals (Real = R; Accelerated = A) all in weeks | |
| --- | --- | --- |
| | 26 weeks | |
| | 26R | 2.6A |
| Filled sachet weight | 10 | 10 |
| Pressure test | 10* | 10* |
| pH of honey | 5** | 5** |
| Moisture level | 5** | 5** |
| Colour | 1 | 1 |
| Sterility | | |
| Samples per batch | 11 | 11 |
| Total Samples | 33 | 33 |
| * Use same samples as for filled sachet weight test<br>** Use five samples from pressure test | | |

Test Methods

Filled sachet weight: An empty sachet has an average tare weight of 1.7g. 10 sachets were weighed individually on a calibrated laboratory balance, the tare weight was subtracted, and the results were recorded.
Pressure Test: Each sachet was tested on a Pressure Test Rig in accordance with standard operating procedures. The number of passes and failures was recorded.
pH of honey: The contents of five sachets were pooled into a glass beaker. A Hanna pH meter was calibrated using three standard solutions, then the electrode was rinsed in DI water. The pH electrode and temperature probe were immersed into the honey sample and the pH value and temperature were read from the digital display, and recorded.
Moisture level: Five sachets of honey were taken from each time point. A sample of approximately 1ml from each sachet was taken and placed on the sample plate of a refractometer (one sample at a time). The sample cover was closed and the user looked through the lens whilst pointing the instrument at a source of light such as a window. The value of the scale was read, as indicated by the line of shadow, and the result was recorded.
Colour: One sachet of honey was opened and some of the honey composition was placed onto a white tile. The colour of the honey composition was compared with the Honey Colour Chart Pfund Scale, and the score was recorded (30 - 800).

Results

Filled sachet weights - accelerated ageing

Batch A:

Table 4

| Test interval (weeks) | Weight of honey in grams - total weight minus tare weight | | | | | | | | | |
|---|---|---|---|---|---|---|---|---|---|---|
| | 1 | 2 | 3 | 4 | 5 | 6 | 7 | 8 | 9 | 10 |
| 2.6 | 10.3 | 10.3 | 10.1 | 10.2 | 10.3 | 10.3 | 10.2 | 10.3 | 10.3 | 10.2 |

Batch B:

Table 5

| Test interval (weeks) | Weight of honey in grams - total weight minus tare weight | | | | | | | | | |
|---|---|---|---|---|---|---|---|---|---|---|
| | 1 | 2 | 3 | 4 | 5 | 6 | 7 | 8 | 9 | 10 |
| 2.6 | 10.3 | 12.1 | 10.3 | 10.6 | 10.6 | 9.8 | 10.1 | 10.1 | 10.1 | 9.8 |

Pressure test results - accelerated ageing

Table 6

| Test interval (weeks) | Batch A | Batch B |
|---|---|---|
| 2.6 | 10/10 | 10/10 |

pH test results - accelerated ageing

Table 7

| Test interval (weeks) | Batch A | | Batch B | |
|---|---|---|---|---|
| | pH | Temp ($^0$C) | pH | Temp ($^0$C) |
| 2.6 | 3.8 | 23.1 | 3.71 | 23.0 |

Moisture content test results - accelerate ageing

Batch A:

Table 8

| Test interval (weeks) | Sample 1 (%) | Sample 2 (%) | Sample 3 (%) | Sample 4 (%) | Sample 5 (%) |
|---|---|---|---|---|---|
| 2.6 | 15.8 | 15.8 | 15.8 | 15.8 | 15.6 |

Batch B:

Table 9

| Test interval (weeks) | Sample 1 (%) | Sample 2 (%) | Sample 3 (%) | Sample 4 (%) | Sample 5 (%) |
|---|---|---|---|---|---|
| 2.6 | 15.6 | 15.8 | 15.6 | 16.2 | 16.2 |

Colour test results - accelerate ageing

Table 10

| Test interval (weeks) | Pfund scale score | |
|---|---|---|
| | Batch A | Batch B |
| 2.6 | 90-120 | 90-120 |

Examples 11-19 below describe the results of treatment of wounds using a composition comprising unpasteurised honey with added glucose oxidase (the composition is referred to in the examples as "Surgihoney"). The Surgihoney was provided in sealed sachets, each containing 10g of the composition. The sachets had been sterilised using gamma irradiation. Sachets were used from day 0 of treatment. Each dressing change involved a fresh application of Surgihoney. The dressing was changed at each of the days recorded in the example, or sometimes more frequently. The Surgihoney was applied to a dressing, or directly to the wound, and then covered by a dressing. In both cases the Surgihoney was in direct contact with the wound and was covered by a dressing.

**Example 11** (Reference Example)

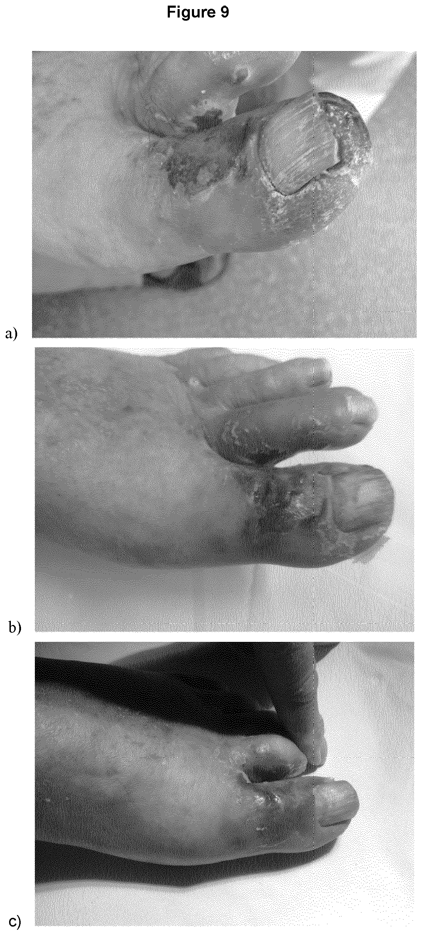

This example describes the results of treatment of an infected toe using Surgihoney. The results are shown in Figure 9.
The patient was a 78 year old diabetic male. The wound on the left foot developed over the month before treatment began and was causing mild pain.

a) Day 0 - Wound Profile: wound: 3 x 2 x 1 cm; 99 % Healthy granulation but 1 % green colonisation; surrounding skin excoriated; small amount of yellow pus exudate giving off an odour;

b) Day 5 - Wound Profile: Wound improved; Second dressing change with 1 honey sachet applied each time; Metrondiazole & amoxicillin; Also Tea tree oil applied;

c) Day 10 - Wound Profile: Wound improved; Green colonisation gone; Wound cleaned, dry skin removed and further sachet of honey applied

**Example 12** (Reference Example)

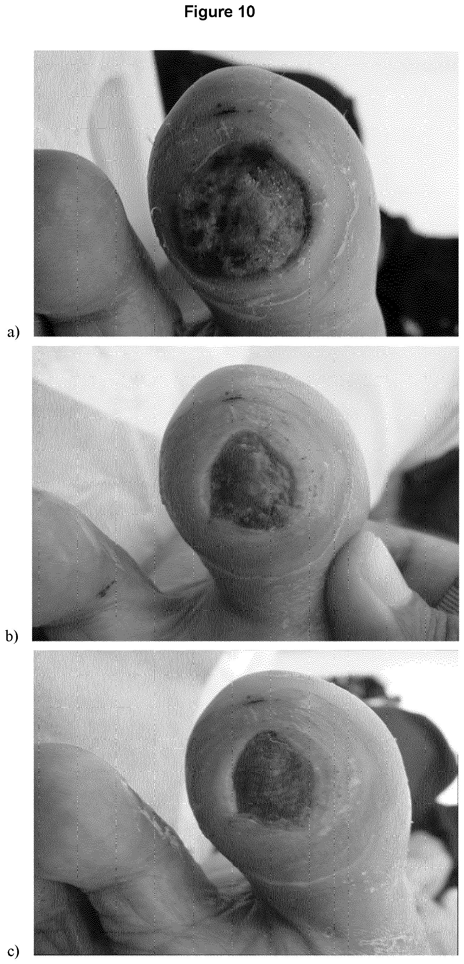

This example describes the results of treatment of a toe ulcer using Surgihoney. The results are shown in Figure 10.
The patient was a 61 year old diabetic female with an infected toe ulcer on the right foot developing over a month-long period prior to treatment, and causing mild pain.

a) Day 0 - Wound Profile: Wound: 2 x 2 x 0.2 cm; 1 % yellow brown slough, rest granulated tissue; Low amount of yellow exudate;

b) Day 7 - Wound Profile: Wound improved; Daily application on 0.5 sachets of Surgihoney with dressing change; Flucox;

c) Day 10 - Wound Profile: Wound improved further; Flucox stopped.

**Example 13** (Reference Example)

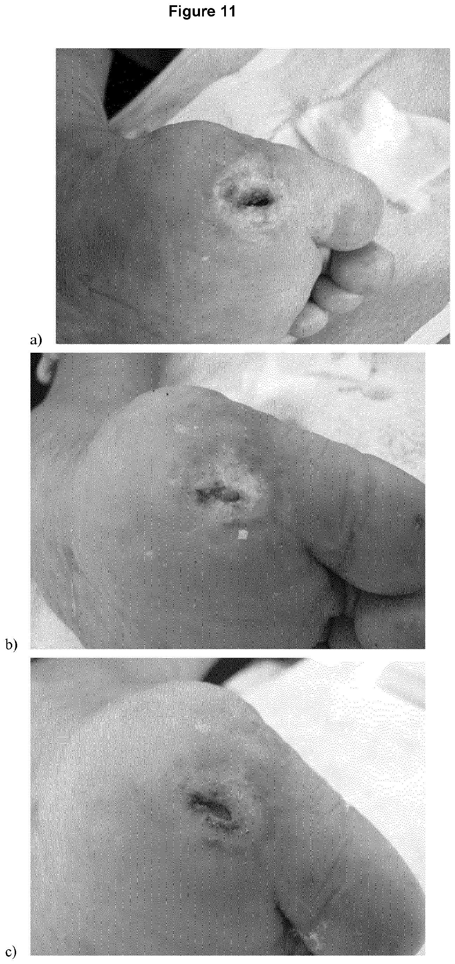

This example describes the results of treatment of a foot ulcer using Surgihoney. The results are shown in Figure 11.
The patient was a 50 year old female diabetic with a foot ulcer that had developed the month before treatment. Fragile skin but no pain was reported.

a) Day 0 - Wound Profile: Wound: 1 x 0.5 x 0.5 cm; 1 % yellow brown slough, rest granulated tissue; Very low amount of low, yellow exudates;

b) Day 7 - Wound Profile: Wound improved; Dry; slough replaced by healthy granulation; Size and depth reduced; Wound cleaned with saline and then honey dressing (0.5 sachet) applied; Nil antibiotics;

c) Day 9 - Wound Profile: Wound much improved; Nearly closed with no exudates present; Dressings continue to be applied with 0.5 sachets on each.

**Example 14** (Reference Example)

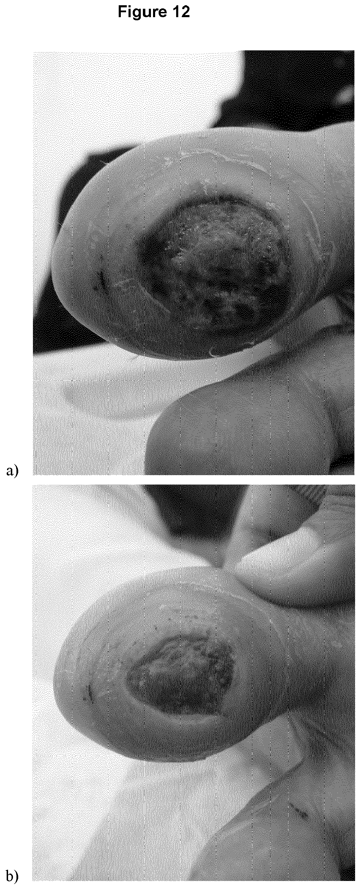

This example describes the results of treatment of a diabetic foot ulcer using Surgihoney. The results are shown in Figure 12.
The patient was a male with a diabetic foot ulcer caused by poor quality shoe irritation. The patient had the ulcer less than 1 month prior to treatment.

a) Day 0 - Wound Profile: Red surrounding skim with mild pain; 1% slough, 99% granulated; Infection and diabetes; Low volume exudate, serous and yellow; Wound size: 2 cm x 2 cm x 0.2 cm;
b) Day 7 - Wound Profile: Assessment done in hospital but the dressing had been changed daily from day 1 until now. The dressings were changed according to the protocol by daughter in law, who is a trained nurse. Wound improved; 100% healthy granulation; Healthy surrounding skin, mild pain; Antibiotics used, Flucoxacillin, 500mg, every 6 hours for 7 days; Wound size: 1.5 cm x 1.3 cm x 0.1.

**Example 15** (Reference Example)

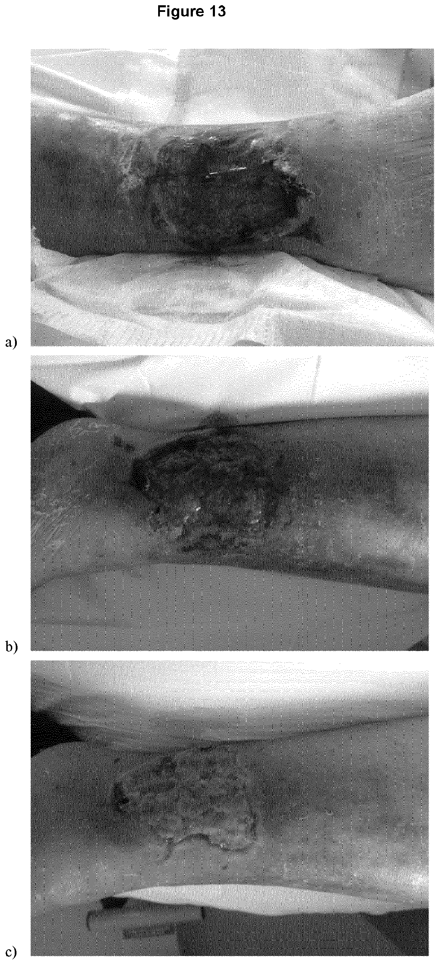

This example describes the results of treatment of a traumatic leg wound using Surgihoney. The results are shown in Figure 13.
The patient was a 95 year old female with a traumatic wound to the lower leg that caused moderate pain.

a) Day 0 - Wound Profile: Wound: 15 x 12 x 1 cm; Mostly granulitic tissue but with 1% of wound bed necrotic and black; Medium amount of haemoserous exudate;

b) Day 3 - Wound Profile: Wound static; No further necrosis but wound much the same; 1 sachet of honey applied; Swab: *Entrecoccus* sp.; nil antibiotics;

c) Day 8 - Wound Profile: Wound improved; Lower volume of exudate and reduced necrotic tissue.

**Example 16** (Reference Example)

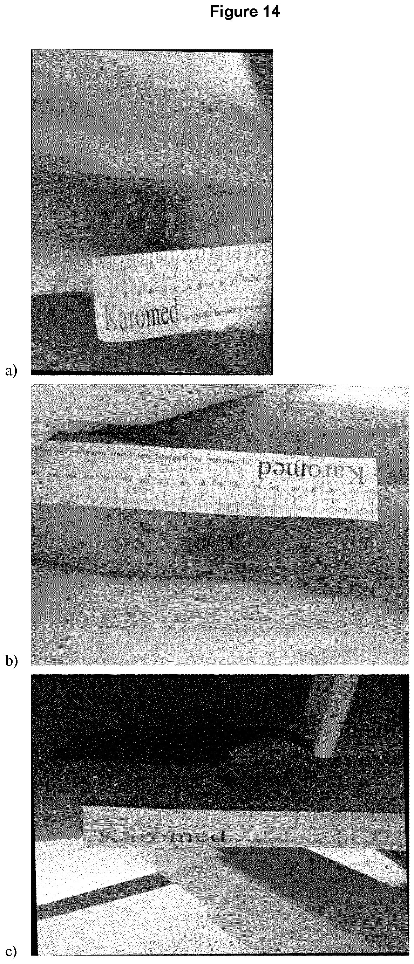

This example describes the results of treatment of an infected leg wound using Surgihoney. The results are shown in Figure 14.
The patient was a 91 year old female with an infected wound to the lower leg. Surrounding skin was fragile and mild pain existed.

a) Day 0 - Wound Profile: Wound Size: 4.5 x 2.5 cm; Mostly red granulitic tissue with 1% yellow/brown slough; Medium volume yellow serous exudate.

b) Day 15 - Wound Profile: Wound improved; Size: 4 x 2 cm; Less slough and serous exudate present;

c) Day 19 - Wound Profile: Wound improved; Surgihoney applied with each dressing.

**Example 17** (Reference Example)

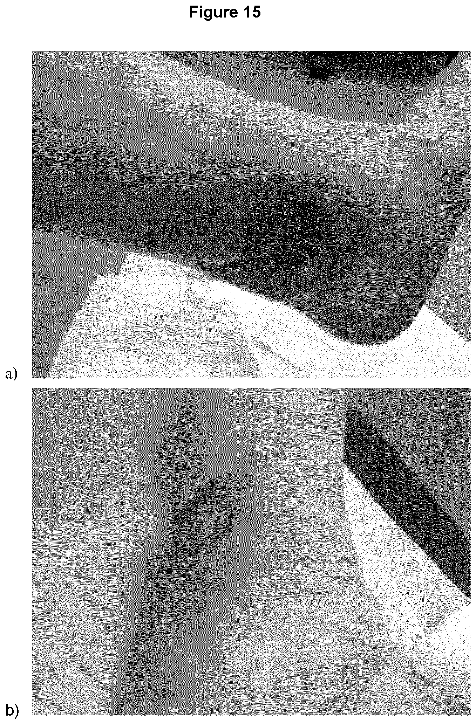

This example describes the results of treatment of a leg ulcer using Surgihoney. The results are shown in Figure 15.

The patient was a female with a Leg Ulcer that had developed over the year before treatment began, and caused mild pain.

a) Day 0 - Wound Profile: Healthy surrounding skin; 1% slough, 99% granulated; Medium volume exudate, serous and yellow; Wound size: 7 cm x 3 cm;

b) Day 7 - Wound Profile: Wound improved and patient in community care; 1 sachet of Surgihoney applied; Size reduced and less exudate present; Pain more bearable.

**Example 18** (Reference Example)

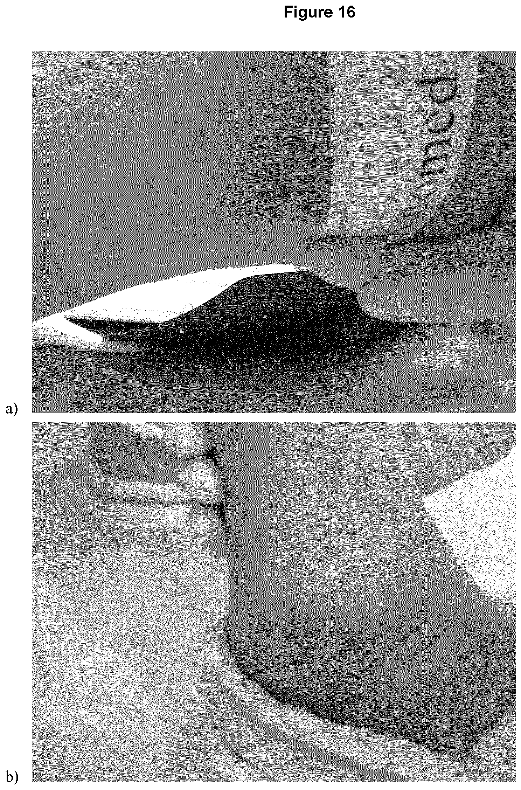

This example describes the results of treatment of a pressure ulcer using Surgihoney. The results are shown in Figure 16.

The patient was an 88 year old female diabetic with poor nutritional status. The patient had a pressure ulcer (grade 3) that had developed over the 6 month period prior to treatment, causing a moderate level of pain with fragile surrounding skin.

a) Day 0 - Wound Profile: Wound size: 0.7 x 0.7 x 0.2 cm; 1% yellow/brown slough, 1%; cellulitic tissue with rest granulitic; Low volume of yellow pus present;

b) Day 3 - Wound Profile: Wound healed (closed up); 1 Sachet of Surgihoney applied.

**Example 19** (Reference Example)

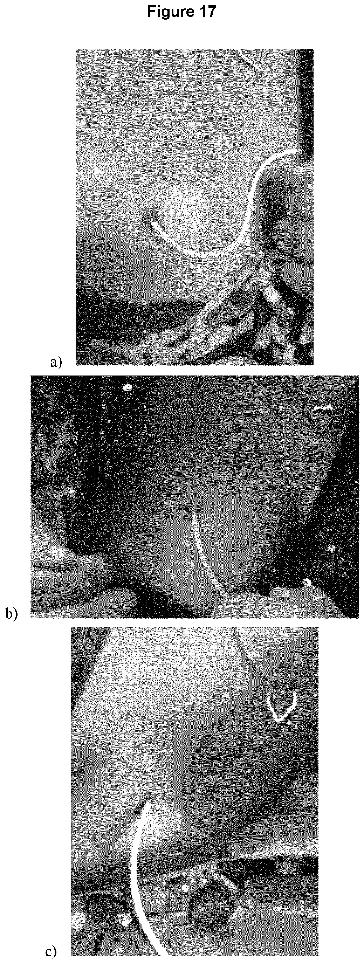

This example describes the results of treatment of infection surrounding the entry point of a catheter using Surgihoney. The results are shown in Figure 17.

The patient was a 41 year old female cancer patient. The patient had infection of breast area surrounding entry point of catheter. The wound was less than 1 week old prior to treatment, mild pain caused.

a) Day 0 - Wound Profile: Wound: 2 cm x 2 cm; 1 % cellulitic, rest granulated tissue; Low volume of exudate, red and serous;

b) Day 6 - Wound Profile: Wound much improved; No exudates; One sachet of surgihoney applied; Swab taken but no growth; nil antibiotics;

c) Day 14 - Wound Profile: Wound improved to the extent that it is no longer an issue.

**Example 20** (Reference Example)

Surgihoney

Surgihoney is unpasteurised honey with added purified glucose oxidase. Three different preparations of Surgihoney were made with different antimicrobial potencies:

**S1 Surgihoney (also referred to as SH1):** unpasteurised honey with 0.1% (w/w) added glucose oxidase. The enzyme used was food grade glucose oxidase, from *Aspergillus niger*, from BIO-CAT, INC, activity 15,000 Units/g. Sealed sachets containing 50g of the S1 Surgihoney were gamma irradiated at a target dose of 11/6-14.2 kGy.

**S2 Surgihoney (also referred to as SH2):** unpasteurised honey with 0.1% (w/w) added glucose oxidase. The enzyme used was glucose oxidase (GO3B2), from *Aspergillus niger*, from BBI Enzymes Limited, activity 274 Units/mg. Unit Definition: the amount of enzyme causing the oxidation of 1 micromole of glucose per minute at 25 degrees centigrade at pH 7.0. Contaminants: alpha amylase no greater than 0.05%, Saccharase no greater than 0.05%, maltase no greater than 0.05% and GO/Cat no less than 2000.

**S3 Surgihoney (also referred to as SH3):** unpasteurised honey with 0.25% (w/w) added glucose oxidase. The enzyme used was glucose oxidase (GO3B2) from BBI Enzymes Limited, activity 274 Units/mg.

Thus, S1 Surgihoney contains 15 units of glucose oxidase per gram of the composition, S2 Surgihoney contains 274 units of glucose oxidase per gram of the composition, and S3 Surgihoney contains 685 units of glucose oxidase per gram of the composition.

**Example 21** (Reference Example)

*In vitro* antimicrobial activity of Surgihoney

This example describes susceptibility testing of a range of wound and ulcer bacterial isolates to Surgihoney by disc diffusion method, minimum inhibitory concentration (MIC) and minimum cidal concentration (MBC) determination, and time bactericidal measurements.

## Summary

**Results:** Surgihoney demonstrates highly potent inhibitory and cidal activity against a wide range of Gram positive and Gram negative bacteria and fungi. MIC/MBC's Are significantly lower than concentrations likely to be achieved in topical clinical use. Topical concentration of Surgihoney in wounds is estimated at approximated 500gms/L. Surgihoney 1 MIC/MBC's for Staph. Aureus are 31 and 125gms/L and Surgihoney 3 MIC/MBC's 0.12 and 0.24gms/L.

Cidal speed depends on the potency. In Surgihoney 1, the least potent, complete cidal activity occurs for all organisms tested within 48 hours. For Surgihoney 3, the most potent, cidal activity occurs within 30 minutes. Maintenance of the Surgihoney inoculums preparation for up to a week demonstrated complete cidal activity and no bacterial persistence.

**Conclusions:** Surgihoney has wide potential as a highly active topical treatment combining the effects of the healing properties of honey with the potent antimicrobial activity of the bioengineered product for skin lesions, wounds, ulcers and cavities. It is highly active against multidrug resistant bacteria. It is more active than other honeys tested and comparable to chemical antiseptics in antimicrobial activity.

Superficial wounds and skin ulcers are becoming increasingly common with the rising age of the population in many countries and the global epidemic of obesity and type 2 diabetes. In the UK, community nurses spend much of their time dressing leg ulcers and supervision by leg ulcer nurses is essential if standards are to be maintained in community leg ulcer services. Most chronic breaks in the skin become colonised with bacteria. It is difficult to know when and if these are pathogenic but it is likely that even if overt infection is not present, bacterial colonisation plays a role in slowing tissue healing, establishing biofilm and resulting in wound slough and an offensive odour.

Tissue viability is challenging particularly when complicated by comorbidities. Chronic wounds always become colonised with bacteria which may destabilise the healing process. There is a temptation to send a microbiological sample and to offer systemic antibiotics when the sample is reported as growing bacteria. All this serves is to select ever more resistant microbes which is why chronic lower extremity ulcers are so often colonised with multidrug resistant organisms such as methicillin-resistant Staphylococcus aureus and Pseudomonas aeruginosa.

Surgihoney has been developed as a prophylactic dressing for wounds. This study examines the *in-vitro* properties of Surgihoney. Surgihoney retains all the established healing properties of natural honey but its antimicrobial activity can be set at whichever potency is required. This study determined minimum inhibitory concentrations (MIC) and minimum bactericidal concentrations (MBC) of Surgihoney 1, 2 and 3 and time kill curves.

## Methods

Surgihoney was provided as potency grades 1, 2 and 3. It was presented as a sterile pharmaceutical grade product in a sachet in semisolid form.

Clinical isolates were collected from soft tissue microbiology samples. Eighteen isolates of *Staphylococcus aureus*, 12 meticillin-sensitive (MSSA) and 6 meticillin-resistant (MRSA), 6 isolates of β haemolytic streptococci, Lancefield groups A (2), B (2), C (1), G (1), 5 isolates of Enterococcus spp. Including vancomycin-resistant *E. faecium*, 6 of *Esch. coli*, including extended spectrum β lactamase producers, 2 of *Klebsiella* spp., 1 *Serratia Marcescens* Amp C producer, 4 of *Pseudomonas aeruginosa*, 1 of *Acinetobacter lwoffii*, 1 of *Propionibacterium acnes*, 1 *Bacteroides fragilis*, and 2 of *Candida albicans*, 1 of *Candida glabrata*, 1 of *Aspergillus fumigates* were tested against Surgihoney.

**Agar diffusion**

Six mm wells were cut in isosenitest agar which had already been inoculated with the test organism at a concentration to give a semiconfluent growth. Test Surgihoney and other honeys in the pilot study were added to the wells.

A pilot study was carried out initially to compare Surgihoney potencies S1, S2, S3 with a variety of honeys from around the world, European, South American, New Zealand, Yemani, Sudanese and with medical honey, Medihoney and with antimicrobial dressings containing silver (Silver Aquacell) and iodine (Iodoflex). Wells were cut in the plates inoculated with *Staphylococcus aureus* and filled with test honey or in the case of the dressings, these were cut to 2x2cm and placed on the surface of the inoculated plates.

Following the pilot studies the Surgihoney potencies S1, S2, S3 were tested alone against the range of bacterial isolates from skin lesions. The wells were filled to the surface with a preparation of approximately 2gms neat Surgihoney of the three potencies, diluted and emulsified in an equal volume of sterile water. Zone sizes were measured after 18-24 hours aerobic incubation (longer for *Candida* and *Aspergillus* spp., and anaerobically for *Propionibacterium* sp. And *Bacteroides* sp.)

**Minimum Inhibitory Concentrations and Minimum Bactericidal Concentrations**

Surgihoney product was warmed to 37°C to liquefy it and 5gms was mixed with 10mLsterile deionised water. This dilution was regarded as the 'neat' substance for serial dilution. The British Society of Antimicrobial Chemotherapy (BSAC) method for performing minimum inhibitory concentrations (MIC's) and minimum bactericidal concentrations (MBC's) was used (Andrews JM. Determination of minimum inhibitory concentrations. J Antimicrob 372 Chemother 2001; 48(Supp 1): 5-16). The Surgihoney products were serially diluted in microtitre tray wells from neat to 1 in 1024. 75µL of each honey dilution was added to each well in the strip of the microtitre tray. The neat concentration represented a concentration of 250gm/L and the 1 in 2048 dilution, approximately 0.12gm/L.

The test organisms were prepared by taking four morphologically identical colonies for each organism from pure culture to create a 0.5 McFarland density. This was further diluted 1:10.

All wells including controls were inoculated with 75µL of the test isolate preparation. The well trays were incubated at 37°C for 18 hours. The MIC was regarded as the most dilute well that showed no detectable turbidity.

The MIC well and those around the MIC well were sub-cultured on blood agar and incubated at 37°C for 18 hours to determine the MBC. The MBC was the most dilute concentration which showed no growth after incubation.

**Time kill curves**

The test organism inoculums was prepared by taking 0.1mL of a 0.5 MacFarlane density of the test organism and inoculating this in 3mL of nutrient broth. The test inoculums was divided into 3 separate bijous, a control and three test preparations to which were added 0.5g of Surgihoney 1 (S1), Surgihoney 3 (S3) or Medihoney (MH). Colony counts of the inocula were determined by serial dilution 1:10 and plating 0.1mL on a blood agar plate, repeated 3 times.

The test and control inocula were kept at 30°C to simulate the temperature of a superficial skin lesion. Colony counts were performed as above in triplicate at time 0.5, 2, 4, 24, 48, 72 and 168 hours.

A terminal culture was performed by inoculating 0.1ml of the original inoculums into nutrient broth to neutralise any residual effect of the Surgihoney and incubating for 72 hours at 37°C, before plating on blood agar to determine test organism survival.

**Results**

**Inhibitory zone sizes.**

The pilot comparative studies demonstrated that all the Surgihoney potencies had greater antimicrobial activity than any other honey tested including the medical grade honey, Medihoney. The inhibitory zones for S1 were larger than those produced by any other honey. Silver dressings produced some inhibitory effect beneath the dressing but there was no zone of inhibition as there was for Surgihoney. Iodine dressings produced a large zone of inhibition (approximately 70mm) to *Staphylococcus aureus* , larger than S1 (36mm) and equivalent to S3 (67mm).

In the quantitative zone size testing, Surgihoney at all potencies produced an inhibitory zone in agar diffusion against all bacteria tested, both Gram positive and Gram negative bacteria including multiply antibiotic resistant bacteria, and fungal species. The zone size for each species increased with increasing Surgihoney potency preparations. Table 11. The inhibitory effect of Surgihoney was not dependant only on direct contact with the active agent as with the silver dressings, but diffused well beyond the well producing the extensive zones listed in Table 11.

**MIC's & MBC's**

Surgihoney demonstrated significant antimicrobial activity against all the isolates tested. MIC's and MBC's were very consistent amongst isolates of the same species whether the isolates were multidrug resistant or highly sensitive. Table 12 lists the MIC and MBC values for isolate species tested by dilution ratio and Table 13 shows the MIC and MBC's in grams per litre. The degree of potency rose with the grade of Surgihoney. The MBC for each isolate was close to the MIC within a single dilution in most cases.

Topical concentration of Surgihoney in wounds is estimated at approximately 500gms/L. Surgihoney 1 MIC/MBC's for *Staph. Aureus* are 31 and 125gms/L and Surgihoney 3 MIC/MBC's 0.12 and 0.24gms/L respectively.

**Time kill curves.**

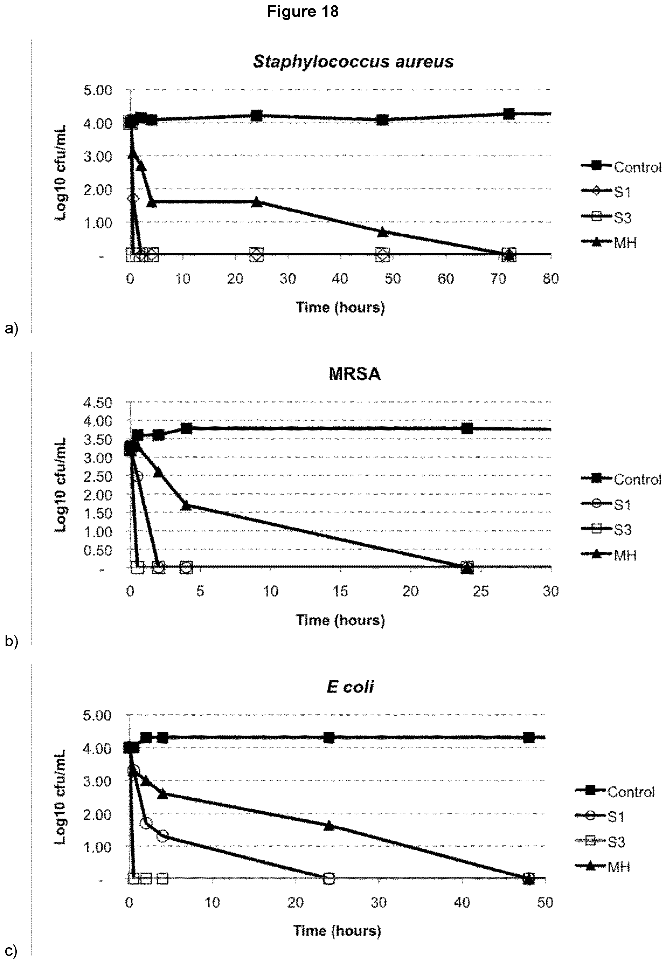

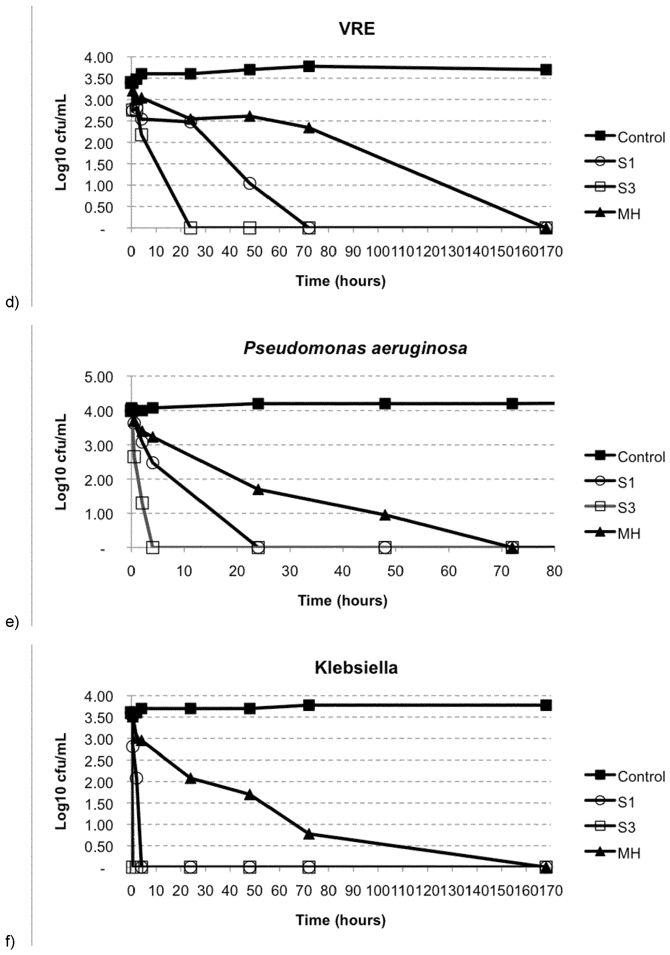

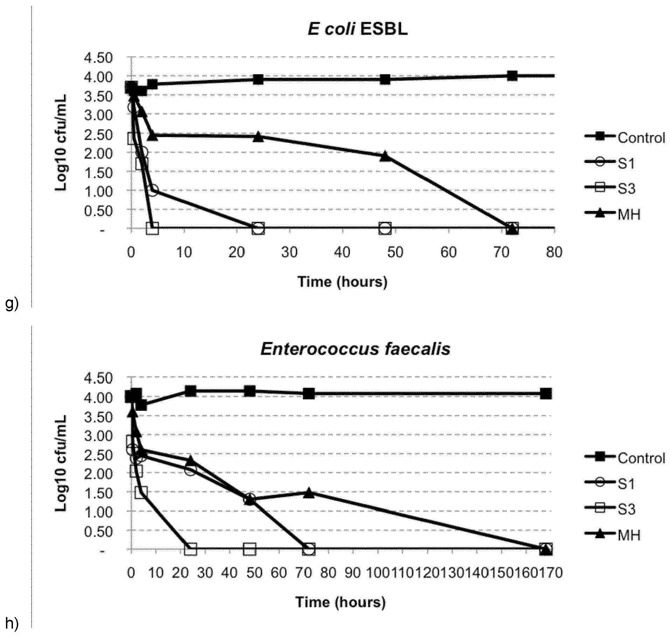

Surgihoney kills bacteria rapidly. Starting with a colony forming units per millilitre (cfu/mL) of approximately 105, cfu/mL numbers in the control rose steadily, whereas in the Surgihoney inocula the cfu/mL fell rapidly after contact with both potencies of Surgihoney. By 30 minutes cfu numbers had fallen 1000 fold in most cases for both S1 and S3 (Figure 18). For S1 bacterial growth was undetectable by 2 hours in most cases and for S3 by 30 minutes. Enterococci appeared more resilient and persisted for 48 hours. Cidal activity was complete for all organisms as terminal culture in nutrient broth with subsequent plating on blood agar failed to detect any organism in the S1 or S3 inocula.

**Discussion**

Surgihoney is natural honey which is also organic in the current sense of the word in that it has no agricultural additives or antimicrobial residues unlike much commercial honey for human consumption. It is not dependant on particular nectar sources, unlike honeys such as manuka which depends on a specific plant nectar source for its enhanced activity. The antimicrobial activity can be controlled in Surgihoney by the preparation process allowing the production of different grades with measured potency which is consistent.

This study has clearly demonstrated the efficacy of Surgihoney as a highly potent antimicrobial, active against all species of bacteria and fungi tested. In the preliminary pilot studies comparing Surgihoney with a variety of honeys sourced from around the world and with medical grade honey, Medihoney, Surgihoney demonstrated significantly greater antimicrobial efficacy. By comparison with the commonly used topical antiseptics silver and iodine, Surgihoney 3 produced an antimicrobial effect as great as iodine dressings and greater than silver dressings (Aquacel Ag) which was only effective at inhibiting bacteria in direct contact with the dressing.

MIC and MBC testing show that Surgihoney not only inhibits but also kills microbes at concentrations 10 to a 1000 fold below those that are likely to be achieved in topical treatment, estimated at 500gms/L. The cidal activity of Surgihoney occurs at concentrations close to its inhibitory activity. There is therefore the potential for Surgihoney to be highly active in polymicrobial inhibition and eradication when applied topically in any colonised or superficially infected wounds or soft tissue cavities. As many chronic wounds are colonised with resistant bacteria, and bacterial persistence in biofilm production delays wounds healing, Surgihoney use may help reduce in appropriate use of antibiotics as well as promote wound healing. In clinical use, the topical Surgihoney concentrations at the site of the wound will be considerably higher than those for systemic antibiotics in serum or deep tissue. This is reflected in the values of the MIC and MBC's for Surgihoney, which are correspondingly higher than those generally expressed for systemic antibiotics.

The speed of cidal activity is shown by the time kill curves to be extremely rapid, within 30 minutes for Surgihoney 3 and within 2 hours for Surgihoney 1. This is the case for both Gram-positive and Gram-negative organisms, although enterococci appear slightly more resilient. Fungi, Candida spp. Aspergillus sp. also require higher concentrations and more prolonged exposure to inhibit growth and kill the organism.

Surgihoney is formulated as a sterile product to be applied as a topical wound dressing to skin lesions and cavities with the aim of providing a moist wound healing environment whilst also reducing microbial colonisation, helping to remove slough and to promote granulation and epithelialisation.

Other antimicrobial preparations are available as topical preparations intended to treat or prevent wound infections. Silver impregnated dressings appear to possess good antimicrobial activity however they also display cytotoxicity compared to honey preparations. Iodine analogues also possess good antimicrobial activity but they also have been reported to be toxic in certain situations. There is also increasing concern about the use of chlorhexidine preparations in wound dressings due to the development of antimicrobial resistance and toxicity.

The clinical utility of Surgihoney is likely to be in topical application, on skin, in wounds and cavities. Wounds may become colonised with bacteria which can form biofilms and delay healing. With increasing concern about antimicrobial resistance and the lack of novel antimicrobial agents, a topical agent with broad antimicrobial activity, could play a role in reducing the use of systemic antibiotics in soft tissue lesions. These in vitro studies have demonstrated the potential of Surgihoney as a wound dressing with high antimicrobial activity whose potency can be controlled and which also delivers other important functions in wound healing: moist barrier, desloughing, local nutrient supply, local immune modulation and is not cytotoxic.

**Conclusion**

These *in vitro* results support the clinical use of Surgihoney as a wound dressing and this may be the first product that can deliver all the required roles in the healing process of wounds as well as being a potent and non-toxic antimicrobial.

Table 11 Inhibitory zones sizes with different potencies of Surgihoney (S1, S2, S3)

| Bacteria | No. of strains | S1 Mean zone (range)/mm | S2 Mean zone (range)/mm | S3 Mean zone (range)/mm |
|---|---|---|---|---|
| *Methicillin-sensitive Staphylococcus aureus (MSSA)* | 12 | 36.2 (32-38) | 53.4 (44-58) | 66.5 (60-72) |
| *Methicillin-resistant Staphylococcus aureus (MRSA)* | 6 | 35.6 (31-38) | 52.6 (48-59) | 67.3 (59-73) |
| *Streptococci Beta haemolytic* | 6 | 40.0 (35-42) | 44.5 (38-51) | 59.2 (53-69) |
| *Enterococcus spp* | 5 | 38.0 (34-39) | 49.5 (44-55) | 61.8 (59-64) |
| *Escherichia coli* | 6 | 33.4 (30-37) | 49.5 (36-55) | 62.7 (59-69) |
| *Klebsiella sp.* | 2 | 34.2 (30-38) | 40.0 (38-42) | 57.0 (52-62) |
| *Pseudomonas aeruginosa* | 4 | 25.8 (20-28) | 34.8 (30-38) | 50.2 (46-51) |
| *Acinetobacter Iwoffii* | 1 | 32.1 | 43.7 | 55.2 |
| *Bacteroides fragilis* | 1 | 22.3 | 28.7 | 34.2 |
| *Propionibacterium acnes* | 1 | 19.7 | 23.4 | 31.9 |
| *Candida sp.* | 2 | 9 (8-10) | 15 (15) | 26 (24-28) |
| *Aspergillus fumigatus* | 1 | 8 | 12 | 18 |

Table 12. Serial double dilutions from neat Surgihoney (S1, S2, S3) showing dilution of minimum inhibitory concentration (MIC) and minimum bactericidal concentration (MBC).

| Organism name | S1 | | S2 | | S3 | |
|---|---|---|---|---|---|---|
| | MIC | MBC | MIC | MBC | MIC | MBC |
| *MSSA* | 1:8 | 1:2 | 1:32 | 1:16 | 1:2048 | 1:1024 |
| *MRSA* | 1:16 | 1:4 | 1:32 | 1:16 | 1:2048 | 1:1024 |
| *Group B Streptococci* | 1:64 | 1:16 | 1:64 | 1:64 | 1:1024 | 1:256 |
| *Group A Streptococci* | 1:32 | 1:16 | 1:128 | 1:64 | 1:1024 | 1:512 |
| *Enterococcus* | 1:8 | 1:2 | 1:32 | 1:4 | 1:256 | 1:64 |
| *E.coli* | 1:8 | 1:4 | 1:64 | 1:64 | 1:256 | 1:128 |
| *E. coli ESBL* | 1:8 | 1:2 | 1:64 | 1:64 | 1:256 | 1:128 |
| *Serr.liquefaciens Amp C* | 1:8 | 1:4 | 1:16 | 1:4 | 1:256 | 1:128 |
| *Kleb.pneumoniae* | 1:4 | 1:2 | 1:32 | 1:32 | 1:256 | 1:128 |
| *Pseud.aeruginosa* | 1:16 | 1:16 | 1:64 | 1:16 | 1:256 | 1:64 |
| *Candida albicans* | Turbid at neat | Growth at neat | 1:16 | 1:16 | 1:64 | 1:64 |

Table 13. Suraihoney MIC and MBC values expressed in Grams/Litre

|  |  | S1 |  | S2 |  | S3 |
|---|---|---|---|---|---|---|
| Organism name | MIC | MBC | MIC | MBC | MIC | MBC |
| *MSSA* | 31 | 125 | 7.8 | 15.6 | 0.12 | 0.24 |
| *MRSA* | 15.6 | 62.5 | 7.8 | 15.6 | 0.12 | 0.24 |
| *Group B Streptococci* | 3.9 | 15.6 | 3.9 | 3.9 | 0.24 | 0.9 |
| *Group A Streptococci* | 7.8 | 15.6 | 1.9 | 3.9 | 0.24 | 0.48 |
| *Enterococcus* | 31 | 125 | 7.8 | 62.5 | 0.9 | 3.9 |
| *E.coli* | 31 | 62.5 | 3.9 | 3.9 | 0.9 | 1.9 |
| *E.coli ESBL* | 31 | 125 | 3.9 | 3.9 | 0.9 | 1.9 |
| *Serr.liquefaciens Amp C* | 31 | 62.5 | 15.6 | 62.5 | 0.9 | 1.9 |
| *Kleb.pneumoniae* | 1:4 | 125 | 7.8 | 7.8 | 0.9 | 1.9 |
| *Pseud.aeruginosa* | 15.6 | 15.6 | 3.9 | 15.6 | 0.9 | 3.9 |
| *Candida albicans* | Turbid at neat | Growth at neat | 15.6 | 15.6 | 3.9 | 3.9 |

**Example 22** (Reference Example)

Anti-viral Activity of Surgihoney

S1 or S2 Surgihoney was mixed with Herpes Simplex Virus in cell culture medium (a 50% mixture of honey and virus in cell culture medium) and then incubated for 1 hour at 37°C. The mixtures were then plated onto cells, and the number of viral plaques formed for each mixture was recorded. Controls with no honey, or with control honey were also performed.

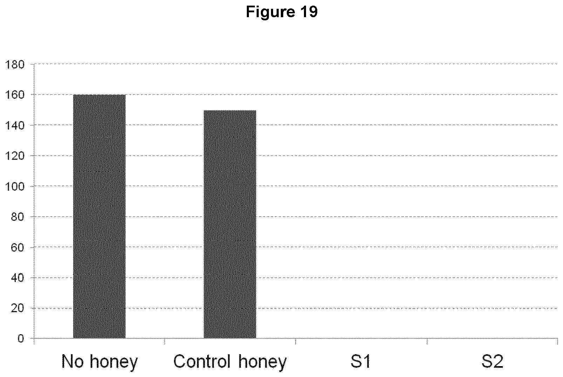

The number of viral (Herpes Simplex Virus) plaques recorded after 1 hour incubation is shown in Figure 19. No plaques were formed for the S1 or S2 Surgihoney mixtures, compared to 160 plaques for the mixture with no honey, and 150 plaques for the mixture with control honey.

The results show that both the S1 and S2 Surgihoney preparations have potent anti-viral activity.

**Example 23** (Reference Example)

Use of Surgihoney with line site dressings

To assess the effectiveness of Surgihoney in preventing infection of peripherally inserted central catheters (PICC lines), S1 Surgihoney was applied topically to the line entry site in the arm of 30 patients. Approximately 3g-8g S1 Surgihoney was applied to a dressing, which was then contacted with the wound, and held in place by a secondary dressing. Line site colonisation and line-associated bacteraemias were assessed and compared with 30 patients who did not receive the Surgihoney dressing. The results are shown in Table 14 below.

Table 14. Effect of S1 Surgihoney in preventing and clearing line site colonisation

|  | **Surgihoney (30)** | **Non-Surgihoney (30)** |
|---|---|---|
| Colonised at initiation | 2 | 4 |
| Colonisation during evaluation | 0 | 6 |
| Colonisation cleared during evaluation | 2 | 0 |

It was concluded that Surgihoney is an effective antimicrobial agent for use with line site dressings.

**Example 24** (Reference Example)

Use of Surgihoney to prevent infection of Caesarean wounds

Infection of surgical wounds is a particular problem with Caesarean sections, which have quite a high infection rate of around 10%. There has been a national increase in Caesarean wound infection (8-24.6%) and a wide variation across NHS hospitals (13.6-31.9%) associated with the 147,726 cases of CS each year in the UK (Bragg et al., 2010. Variation in rates of caesarean section among English NHS trusts after accounting for maternal and clinical risk: cross sectional study. BMJ 2010; 341). Caesarean wound infection is a major cause of prolonged hospital stay, resource consumption, as well as other morbidities and mortality. Recovery from Caesarean section is more difficult for women who develop postoperative wound infection.

To assess the effectiveness of Surgihoney in preventing infection of Caesarean wounds, S1 Surgihoney was applied once topically to the wound post surgery. Approximately 25g-35g S1 Surgihoney was applied to a dressing, which was then contacted with the wound, and held in place by a secondary dressing. Nearly 200 patients were assessed over a three month period.

Clinical Evaluation

Women presenting for Caesarean section (CS) between October 2012 and January 2013 were offered Surgihoney as a dressing to the wound as a single application when the wound was dressed at the end of the procedure. Each 10g sachet of Surgihoney was for single patient use. Using an aseptic technique a 'non-sterile' operative assistant opened the Surgihoney sachet and carefully applied the sterile contents on to the sterile dressing. The dressing was then applied to the surgical wound by the obstetrician or theatre midwife. After the procedure, the attending midwife completed an evaluation record. Data collected were MRSA status, history of diabetes, medications, and body mass index. For 14 days after the procedure the attending midwife also recorded any wound healing problems, specifically the presence of oozing, pain, inflammation. If there was any inflammation, a wound culture swab was requested and microbiological results were recorded. The surgical site infection (SSI) rate during the three months of the evaluation using Surgihoney dressing was compared with the infection rate in the 9 months prior to the evaluation based on data collected by the infection control team. Wound infection was defined clinically as an inflamed wound (erythema, swelling, discharge) which required antibiotic treatment. The rate of SSI was calculated as a percentage of all CS procedures carried out.

Results

The results are shown in Table 15 below. In the 3 month period, Oct 2012 --- Jan 2013, there were 186 CS's, of which 102 (55%) were emergencies. No women were colonised with MRSA. Four (2.23%) had diabetes mellitus. 42 (27.3%) had a body mass index >25. There were 4 out of 186 confirmed CS SSI during the evaluation. This represented an infection rate of 2.15 %. A single patient reported an adverse event related to Surgihoney treatment in the form of wound irritation which resolved without further intervention in 3 days. In the preceding 9 months there were 590 CS procedures (234 elective and 356 emergency) and the infection control surveillance recorded 32 CS SSI, representing an infection rate of 5.42%. The reduction in infection rates is significant: p=0.042 ($\chi^2$ test).

Table 15. Effect of S1 Surgihoney in preventing infection of Caesarean wounds

| Period | Total no of procedures | Elective (%) | Emergency (%) | No. of infected wounds | Infection rate % |
|---|---|---|---|---|---|
| Jan 2012 to Sept 2012 - no S1 Surgihoney | 590 | 234 (39.7%) | 356 (60.3%) | 32 | 5.42% * |
| 22 Oct 2012 Jan 2013 - with S1 Surgihoney | 186 | 84 (45.2%) | 102 (54.8%) | 4 | 2.15% (60% reduction) |
| * From Microbiology sample data which probably under reports historic infection rates in the Trust. UK national average closer to 10% | | | | | |

The results show that there was a lower rate of surgical site infection (a 60% reduction) in the group treated with S1 Surgihoney compared to historic data. The Surgihoney dressing was well tolerated with few reported adverse effects.

The wound infection rates fell by 60.33% when Surgihoney was used. Using the SSI data from the two arms of the study CS SSI rates (expected) were 5.42% before Surgihoney and (observed) 2.15% after. At these levels (which are lower than the rates of infection previously reported at 9.6%) the extrapolated CS SSI infections rates for UK would be (expected) 8007 cases per year and (observed) 3176 case per year. The difference is 4831 cases that could potentially be reduced by using Surgihoney.

It was concluded that S1 Surgihoney effectively reduces the rate of infection of Caesarean wounds post surgery. Prevention of colonisation of wounds with Surgihoney, an agent which is not toxic to healing tissue and which also promotes the healing process, is a novel and potentially important finding which may change the way that surgical wounds are managed. Surgihoney offers a clinically and cost-effective intervention to significantly reduce SSI in women undergoing Caesarean section.

Discussion

This evaluation demonstrated that Surgihoney, a highly effective antimicrobial wound dressing, can be employed as a wound dressing of primary CS wounds to prevent infection. As a 'natural' product with established wound healing properties Surgihoney is likely to promote wound healing in addition to providing potent antimicrobial activity to prevent wound colonisation and infection. Some halogen-based chemical antiseptics may provide the same degree of antimicrobial activity but may delay wound healing (Jan WA. Comparison of conventional pyodine dressing with honey dressing for the treatment of diabetic foot ulcers. JPMI - Journal of Postgraduate Medical Institute 2012; 26(4): 402-7). Iodine wound dressings are contraindicated in CS (Joint Formulary Committee. The British National Formulary. London: The Pharmaceutical Press; 2013) and a range of toxicities are associated with their use (Pietsch & Meakins: Complications of povidone-iodine absorption in topically treated burn patients. The Lancet 1976; 307(7954): 280-2; Scoggin et al.: Hypernatræmia and acidosis in association with topical treatment of burns. The Lancet 1977; 309(8018): 959; Donovan et al.: Seizures in a Patient Treated with Continuous Povidone-Iodine Mediastinal Irrigation. New England Journal of Medicine 1992; 326(26): 1784; Colpaert: Iodine toxicity as a cause of total atrioventricular block in burn patients. Burns 2009; 35: S45-S6; Ramaswamykanive: Cardiovascular collapse following povidone-iodine wash. Anaesthesia and Intensive Care 2011; 39(1): 127-30; Lakhal: Povidone iodine: Features of critical systemic absorption. Annales Francaises d'Anesthesie et de Reanimation 2011; 30(7-8): e1-8):e1-e3.).

Similarly, Cochrane systematic reviews showed there was insufficient evidence to establish whether silver-containing dressings or topical agents promote wound healing, prevent wound infection (Storm-Versloot et al.: Topical silver for preventing wound infection. Cochrane Database of Systematic Reviews 2010) or are effective treatments of infected or contaminated chronic wounds (Vermeulen et al.: Topical silver for treating infected wounds (Review). Cochrane review 2010; (10): 42).

Although previous systematic reviews on the clinical effectiveness of honey as a wound dressing have shown equivocal evidence of benefit, this new preparation appears to offer significant clinical benefits in CS patients (Jull et al.: Honey as a topical treatment for wounds: The Cochrane Collaboration, 2009; Jull et al.: Honey as a topical treatment for wounds. Cochrane database of systematic reviews (Online) 2013; 2). In a temporal comparison of wound infection rates the evaluation has shown a 60.33% reduction in infection rates from 5.42% prior to the intervention to 2.15% using Surgihoney.

Healthcare associated infections are a significant and costly healthcare complication with approximately 8% of patients in hospital and SSIs accounted for 14% of these infections and nearly 5% of patients who had undergone a surgical procedure were found to have developed an SSI. SSIs are associated with considerable morbidity and over a third of postoperative deaths are related, at least in part, to SSI. Antimicrobial prophylaxis is routinely employed in many surgical procedures to reduce surgical wound infection. While skin disinfection is also routinely used by surgeons to reduce the skin bacterial load prior to skin incision, it has not been routine practice to use antimicrobial dressings. A reason for this may be that most topical antiseptics have a deleterious effect on tissue healing.

Surgihoney is a product with potent antimicrobial activity, which is non-toxic and promotes tissue healing. Application of this product topically to 'clean' surgical wounds could actually replace systemic antibiotic prophylaxis in certain types of surgery. Such an advance would assist the reduction of antibiotic volume use and the selection pressure on colonising bacteria.

Caesarean wounds were chosen in this evaluation because the patients are by and large healthy with no, or very few co-morbidities, and CS infection rates are reported to be increasing. Possible reasons for this increase have been an increase in older mothers, mothers with co-morbidities, particularly diabetes and a general increase in mothers with higher body mass index. While it has not previously been routine to use an antimicrobial agent in the primary wound dressing, this evaluation has shown an interesting and effective role for Surgihoney in the prevention of CS wound infections.

**Example 25** (Reference Example)

Use of S1 Suraihoney to treat a pressure sore

The patient was a 50 year old female patient with spina bifida who was disabled and immobile. The patient had a pressure sore in the lower back down to the sacral bone which had persisted for over 1 year. The cavity was infected with *Streptococcus pyogenes.*

S1 Surgihoney was used as a topical dressing. Wound improvement was reported from day 2. By day 30, the soft tissue cavity had almost completely healed. No *Streptococcus* was detected at this point.

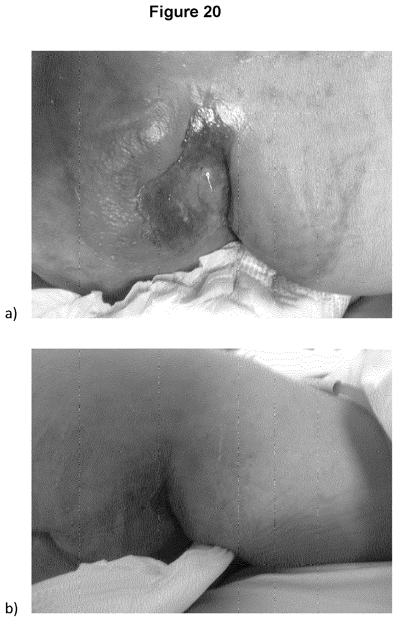

Photographs of the results are shown in Figure 20, in which: (a) shows day 1 of treatment; and (b) shows day 30 of treatment.

**Example 26** (Reference Example)

Antimicrobial activity of Surgihoney

The antimicrobial activity of Surgihoney (SH) and two prototype modified honeys made by *Apis mellifera* (honeybee) against *Staphylococcus aureus* (NCIMB 9518) was tested. We also examined a number of modified types of Surgihoney for the ability to change the level of production of hydrogen peroxide from the samples.

Methods: Surgihoney (SH) was compared with two modified honeys, Prototype 1 (PT1) and Prototype 2 (PT2) using a bioassay method against a standard strain of *Staphylococcus aureus.* Further work studied the rate of generation of hydrogen peroxide from these preparations.

Results: Surgihoney antimicrobial activity was shown to be largely due to hydrogen peroxide production. By modification of Surgihoney, two more potent honey prototypes were shown to generate between a two- and three-fold greater antibacterial activity and up to ten times greater peroxide activity.

Conclusions: Surgihoney is a clinically available wound antiseptic dressing that shows good antimicrobial activity. Two further honey prototypes have been shown to have antimicrobial activity that is possible to be enhanced due to demonstrated increases in peroxide activity.

Methods

1. Determination of Honey Activity by Bioassay Method

The antibacterial activity of Surgihoney (S) and two modified honeys, Prototype 1 (PT1) and Prototype 2 (PT2) was measured using Staphylococcus aureus (NCIMB 9518) and expressed as the equivalent % phenol. Values were calculated of the mean from three sample replicates tested, repeated on three days.

**Assay Method.** The agar well diffusion method used was adapted from the punch plate assay for inhibitory substances described in the Microbiology Standard Methods Manual for the New Zealand Dairy Industry (1982)[ Bee Products Standards Council: Proposed standard for measuring the non peroxide activity of honey. In. New Zealand: Bee Products Standards Council; 1982.].

**Inoculum Preparation.** Overnight culture was adjusted to an absorbance of 0.5 measured at 540 nm using sterile nutrient broth as a blank and a diluents and a cuvette with a 1 cm pathway.

**Assay Plate preparation.** A volume of 100 μl of the culture adjusted to 0.5 absorbance was used to seed 150 ml nutrient agar to make the assay plates. The agar was swirled to mix thoroughly and poured into large petri dishes which had been placed on a level surface. As soon as the agar was set the plates were placed upside down overnight before using the next day. For assay these seeded plates were removed from 4°C and allowed to stand at room temperature for 15 min before cutting 7.0 mm diameter wells into the surface of the agar. 250 μl of test material (sample or standard) was placed into each well.

**Catalase solution.** A 200 mg/ml solution of catalase from bovine liver (Sigma C9322, 2900 units/mg) in distilled water was prepared fresh each day.

**Sample preparation.** Primary sample solutions were prepared by adding 4 g of sample to 4 ml of distilled water in universals and placed at 37°C for 30 minutes to aid mixing. To prepare secondary solutions, 2 ml of the primary sample solution was added to 2 ml of distilled water in universals and mixed for total activity testing and 2 ml of the primary sample solution was added to 2 ml of catalase solution and mixed for non-peroxide activity.

**Preparation of phenol standards.** Standards (w/v) 10%, 30%, 50% phenol were prepared by dissolving phenol in water. Phenol standards were brought to room temperature in the dark before use and were mixed thoroughly before addition to test wells. Each standard was placed in three wells to test in triplicate. Standards were kept at 4°C with an expiry date of one month.

**Sample and standard application.** All samples and standards were tested in triplicate by adding 250 $\mu$l to each of 3 wells.

**Plate incubation.** After application of samples the plates were incubated for approximately 18 hours at 37 °C. The diameter of inhibition zones, including the diameter of the well (7.0 mm), was recorded.

**Calculation of antibacterial activity of samples.** The mean diameter of the clear zone around each phenol standard was calculated and squared. A standard graph was plotted of % phenol against the square of the mean diameter of the clear zone. A best-fit straight line was obtained using linear regression and the equation of this line was used to calculate the activity of each diluted honey sample from the square of the mean measurement of the diameter of the clear zone. To allow for the dilution (assuming the density of the Surgihoney to be 1.35 g/ml) this figure was multiplied by a factor of 4.69 and the activity of the samples was then expressed as the equivalent phenol concentration (% w/v).

Total Activity: all the activity, including activity due to hydrogen peroxide ($H_2O_2$).

Non-Peroxide Activity: $H_2O_2$ is removed by treating samples with catalase enzyme.

2. Determination of Honey Activity by $H_2O_2$ Method

The activity was measured using the *Merckoquant*® *1.10011. & 1.10081.*

**Peroxide Test Kits.** Concentrations expressed as the equivalent mg/L $H_2O_2$.

Samples were diluted 1:10 with purified water. Following 5 min incubation, all samples were measured for $H_2O_2$ production each hour over a 12 hour period followed by 24 and 48 hour time points.

**Method of Determination.** Peroxidase transfers oxygen from the peroxide to an organic redox indicator, which is then converted to a blue coloured oxidation product. The peroxide concentration is measured semi-quantitatively by visual comparison of the reaction zone of the test strip with the fields of a colour scale. The reaction zone of the test strip is immersed into the Surgihoney sample for 1 sec, allowing excess liquid to run off the strip onto an absorbent paper towel and after 15 seconds (Cat. No. 110011), 5 seconds (Cat. No. 110081), after which a determination of the colour formed in the reaction zone more precisely coincided with the colour fields scale.

Results

1. Activity Rating

The antimicrobial activity produced by the modification of the honey samples resulted in a two-fold and almost three-fold respectively increase in phenol activity with PT1 and PT2 compared with Surgihoney alone. The results for the three samples of Surgihoney (SH) and two modified prototypes, PT1 and PT2 are shown in Table 16.

2. Determination of Honey Activity by $H_2O_2$ Method

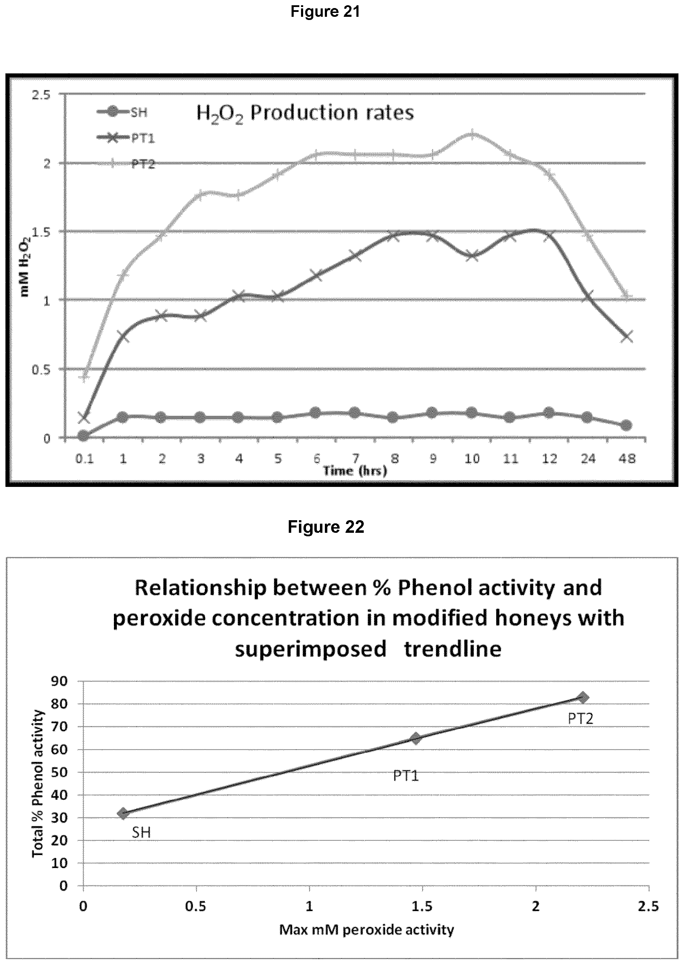

The prototype modifications are observed to generate up to seven and ten times the hydrogen peroxide activity of Surgihoney. The results for the three samples are shown in Figure 21. By taking the maximum level of hydrogen peroxide output for each of the three honey prototypes and plotting this against the total phenol activity a linear relationship is observed (Figure 22).

Discussion

The results from this work show that the main antimicrobial activity of Surgihoney and two modified prototypes, PT1 and PT2 are due to hydrogen peroxide. This is a similar finding to certain other honeys from a variety of floral sources. However, unlike previous work the availability of hydrogen peroxide from the samples is able to be enhanced and at 12 hours is seven and ten times respectively the value for Surgihoney alone. There is a striking linear relationship between the antimicrobial activity and the maximum output of hydrogen peroxide from the three honey prototypes.

This peroxide activity offers potent antimicrobial activity that is ideally suited for a wound dressing that is applied to acute or chronic wounds to treat or prevent wound infections. Whilst a small amount of catalase is present in wounds and serum level of catalase in males has been reported as 50 kU/l it has been shown that catalase activity in healing wounds actually decrease during the first week post-wounding and activity levels of catalase recover to its original level at two weeks post-wounding. Such concentrations of catalase are thus extremely unlikely to influence the antimicrobial activity observed with exogenously applied Surgihoney or the two modified prototypes, PT1 and PT2.

The ideal characteristics for an antimicrobial wound dressing are: effectiveness, lack of toxicity, ease of use, patient and clinician acceptability and value for money. Hydrogen peroxide is an effective antimicrobial and is already used as a biocide for its potent activity against vegetative bacteria, yeasts and spores. It produces its antimicrobial effect through chemical oxidation of cellular components.

The human toxicity of hydrogen peroxide is concentration dependent and one study has claimed that the differential concentrations for antimicrobial and human toxicity might overlap.

By contrast, certain preparations of honey have been shown to be an effective antimicrobial agent by supplying low concentrations of hydrogen peroxide to wounds continuously over time rather than as a large amount at the time of dressing and without such toxicity. Indeed there is compelling evidence that where physiological levels of hydrogen peroxide are applied to mammalian cells there is a stimulation of biological responses and activation of specific biochemical pathways in these cells.

Clearly Surgihoney and the two modified prototypes, PT1 and PT2 are antimicrobial dressings that offer effective hydrogen peroxide release over at least 24 hours.

Conclusions

Surgihoney and the two modified prototypes, PT1 and PT2 have been shown to have potent antimicrobial activity against a standard strain of *Staphylococcus aureus.* These antimicrobial activities have been shown to be due to hydrogen peroxide. The activity is scalable and can be described in terms of hydrogen peroxide activity. These modified honeys offer a dressing that is effective, non-toxic and easy to administer.

Table 16 showing the peroxide and non-peroxide antibacterial activities of Surgihoney (SH) and two modified prototypes, PT1 and PT2 against Staphylococcus aureus (NCIMB 9518).

| Sample Name | Batch No. | Total Activity (% phenol) | Non-Peroxide Activity (% phenol) |
|---|---|---|---|
| **Surgihoney** | 2015-06-018B | 32 | 0 |
| **Surgihoney PT1** | HHI4110311 | 65 | 7 |
| **Surgihoney PT2** | HHI14110312 | 83 | 10 |

Examples 27-30 below describe determination of the *in vitro* antibacterial activity of a composition (Surgihoney) against important biofilm-forming burn wound pathogens.

Surgihoney has previously demonstrated highly potent inhibitory and cidal activity against a wide range of planktonic Gram-positive and Gram-negative bacteria in both laboratory tests and clinical practice. Prophylactic application to caesarean wounds has demonstrated eradication of resistant organisms and reduced rates of colonisation and infection.

Given the global concerns surrounding antimicrobial resistance, and the challenges posed by biofilms, the *in vitro* antibacterial activity of the three different formulations of Surgihoney (S1, S2 and S3 -as described above) against biofilm-forming isolates of *Pseudomonas aeruginosa* and *Acinetobacter baumannii* was investigated to assess whether Surgihoney can i) prevent the formation of a biofilm and ii) eradicate or prevent seeding of a pre-formed biofilm.

The anti-biofilming properties of Surgihoney formulations S1, S2 and S3 were investigated and compared against standard Manuka honey using two biofilm assays. These enabled *in vitro* measurement of the 'Minimum Biofilm Inhibition Concentration' (MBIC), and the 'Minimum Biofilm Eradication Concentration' (MBEC) of SH. Further experiments were performed to compare the anti-biofilming activity of SH to a range of commercial dressings, including one that contains 20% honey (L-Mesitran net).

**Example 27** (Reference Example)

Prevention of biofilm formation by Surgihoney compared with Manuka honey

This example describes a first experiment (Experiment 1), which compares the effect of Surgihoney (S1), and Manuka honey (MH), and a second experiment (Experiment 2), which compares the effect of three different strength preparations of Surgihoney (S1, S2, and S3) on biofilm formation by biofilm-producing isolates of *Pseudomonas aeruginosa* (control strain PA01, and clinical burn wound isolate 1054) and *Acinetobacter baumannii* (control strain AYE, and UK ACI clone NCTC_13420(C59)).

Methods

For each neat honey sample, one loopful of honey was placed into 100µl water in a well of a 96-well microtitre plate. For the diluted honey samples, approximately 2.5ml honey was placed into a universal with 6ml of water. 3ml of this mixture was then serially diluted down to 1 :2048. 100µl of each serial dilution was added to a different well of the 96-well plate.

Overnight cultures of the isolates were diluted in Muller-hinton broth to an OD600 of 0.1. 100µl of the diluted overnight culture was added to each neat or diluted honey sample well. Each positive control well contained 100µl diluted overnight culture, and 100µl water. Each negative control well contained 200µl broth or 200µl neat honey sample.

The plate was incubated for 72 hours at 33°C (wound temperature) to encourage biofilm formation. The plate was then developed using crystal violet dye (which binds to dead and living biofilms), and visualised following solubilisation of the dye using 70% ethanol.

A spectrophotometer was then used to assess the optical density (OD) of the solubilised dye. The OD reading corresponds to the amount of incorporated crystal violet. Higher OD readings represent dark wells, and greater mass of biofilm. The OD readings were then plotted to make a graph.

Results

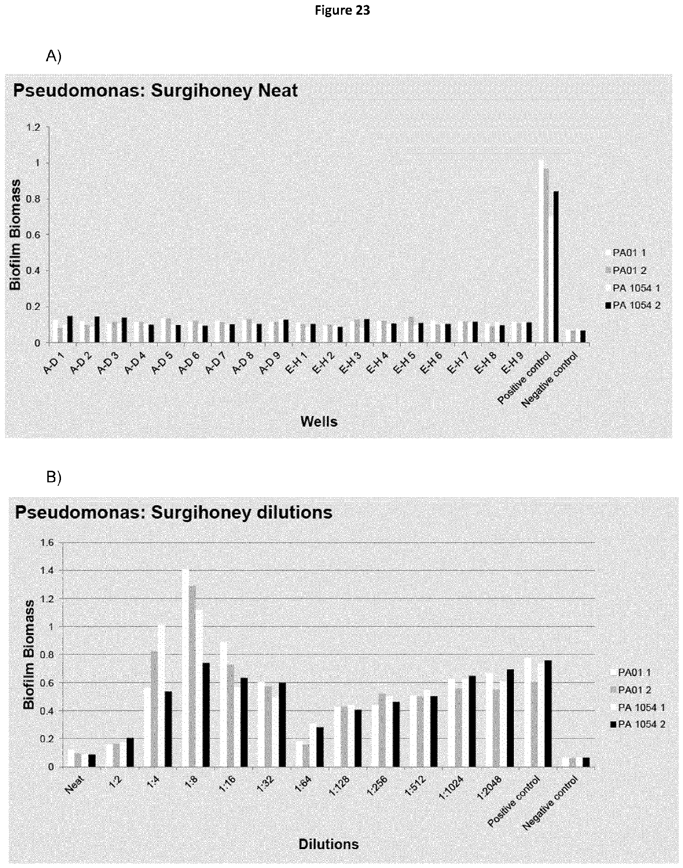

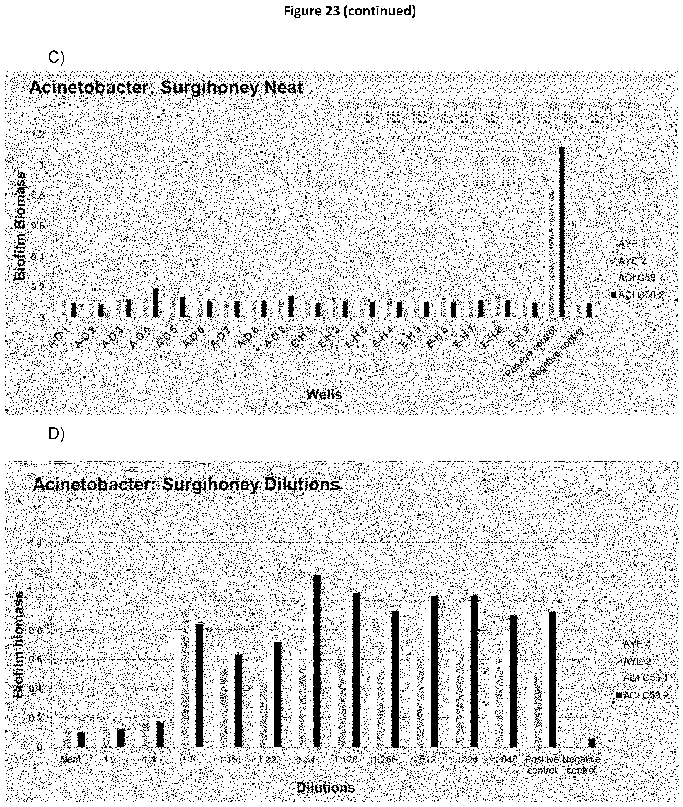

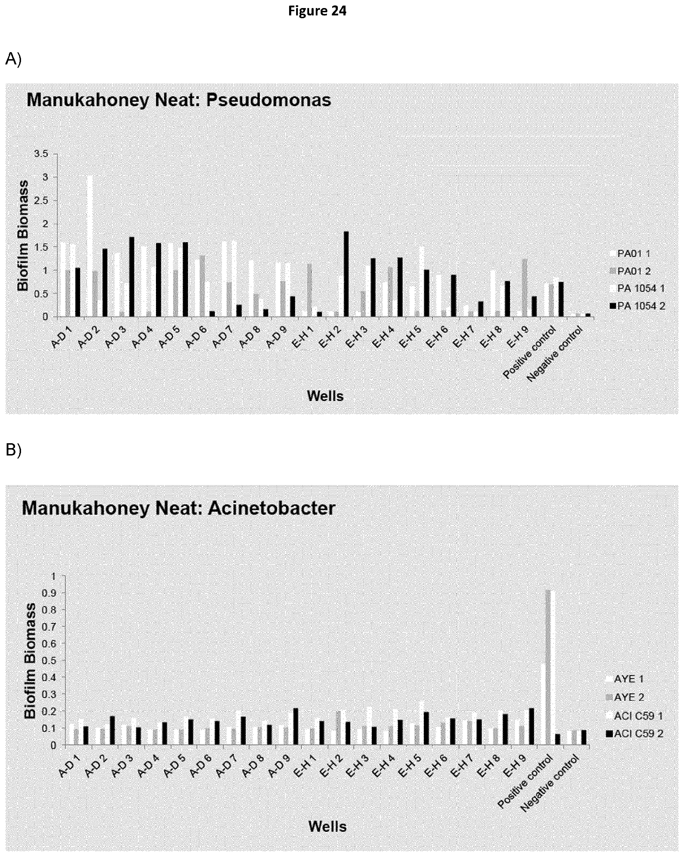

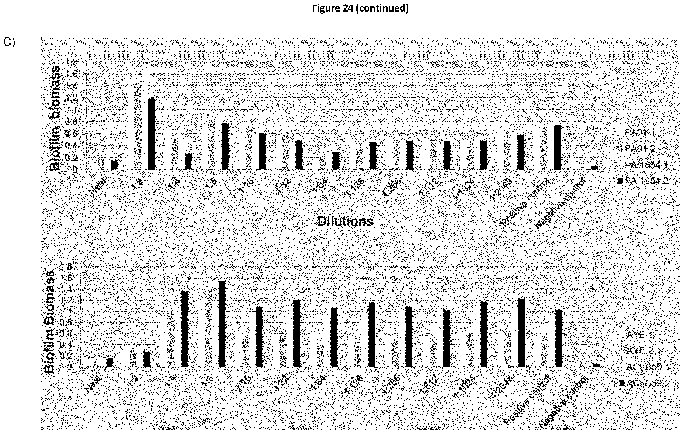

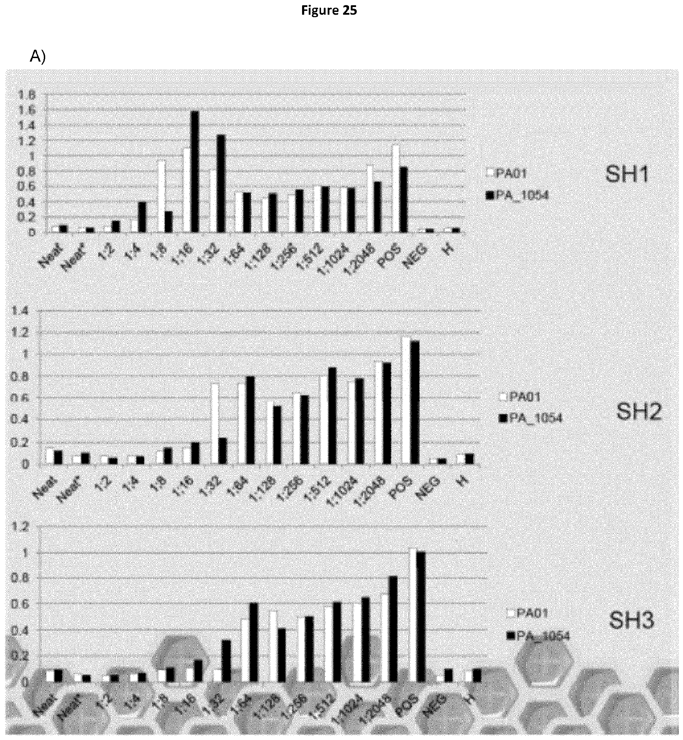

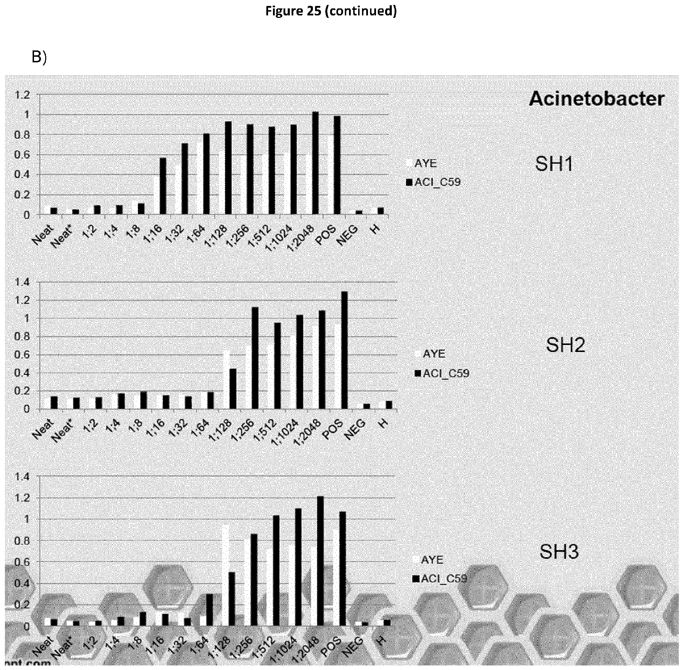

The results are shown in Figures 23-25, and summarised in the table below, which records the Minimum Biofilm Inhibitory Concentration (MBIC) for each strain/isolate (using an OD of 0.3 as a cut-off):

| Strain/Isolate | MBIC | | | | |
|---|---|---|---|---|---|
| | Experiment 1 | | Experiment 2 | | |
| | S1 | MH | S1 | S2 | S3 |
| PA01 | 1:2 | Neat[1] | 1:4* | 1:16* | 1:32* |
| 1054 | 1:2 | Neat[1] | 1:8* | 1:32* | 1:16* |
| AYE | 1:4 | 1:2 | 1:8* | 1:64* | 1:64* |
| C59 | 1:4 | 1:2 | 1:8* | 1:64* | 1:32* |
| [1] The activity of Manuka honey against *Pseudomonas* was sporadic | | | | | |
| * = Statistically significant (P<0.005) when student t-test performed to compare growth with honey and positive control. | | | | | |

The results show that Surgihoney SH1 prevented *Pseudomonas* biofilm formation when used neat or at 1:2 dilution, and prevented *Acinetobacter* biofilm formation when used neat or at 1:2 or 1:4 dilution.

Manuka honey had a sporadic effect on *Pseudomonas* biofilm production when used neat, but prevented *Acinetobacter* biofilm formation when used neat or at 1:2 dilution.

Surgihoney S2 and S3 also prevented *Pseudomonas* and *Acinetobacter* biofilm formation, but were able to do so at lower concentrations than S1 Surgihoney.

There appeared to be little significant difference in the ability of Surgihoney S2 and S3 to inhibit *Pseudomonas* and *Acinetobacter* biofilm formation.

Biofilm formation by *Acinetobacter* appeared to be more sensitive to Surgihoney and Manuka honey treatment than *Pseudomonas* biofilm formation.

Conclusions

It was concluded from these results that Surgihoney is able to prevent biofilm formation of biofilms, and that Surgihoney S2 and S3 were able to prevent biofilm formation at lower concentrations than Surgihoney S1. Each Surgihoney preparation was able to prevent biofilm formation at a lower concentration than Manuka honey.

**Example 28** (Reference Example)

Prevention of biofilm formation by different strength preparations of Surgihoney compared with Manuka honey

This example describes the effect of Surgihoney1 (S1), Surgihoney 2 (S2), Surgihoney 3 (S3) and Manuka honey (MH), on biofilm formation by biofilm-producing isolates of *Pseudomonas aeruginosa* (PS_1586] and PS_6749) and *Acinetobacter baumannii* (ACI_C60 and ACI_19606).

Methods

Each honey was placed in a 37°C incubator for 30 minutes. Approximately 3ml each honey was then placed into a universal, and 3ml sterile water was added. This mixture was then vortexed vigorously and serially diluted down to 1 :4096.

100μl of a diluted overnight culture of each isolate was added to 100μl of diluted honey in a 96-well microtitre plate, and incubated for 72 hours at 33°C (wound temperature) to encourage biofilm formation.

The plate was then developed using crystal violet dye, and visualised following solubilisation of the dye using 70% ethanol.

A spectrophotometer was then used to assess the optical density (OD) of the solubilised dye. The OD reading corresponds to the amount of incorporated crystal violet. Higher OD readings represent dark wells, and greater mass of biofilm. The OD readings were then plotted to make a graph.

Results

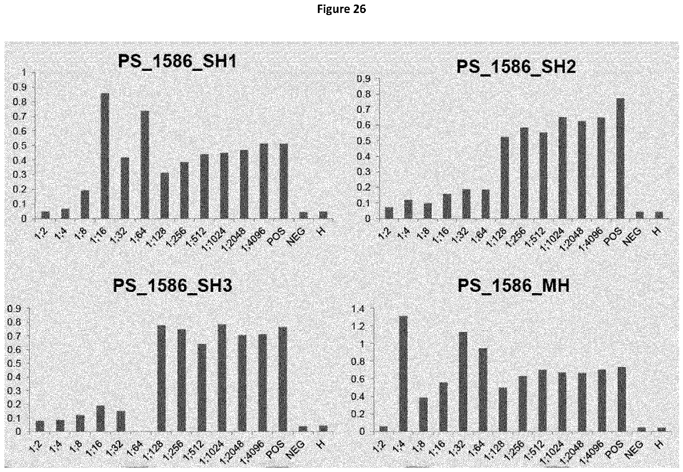

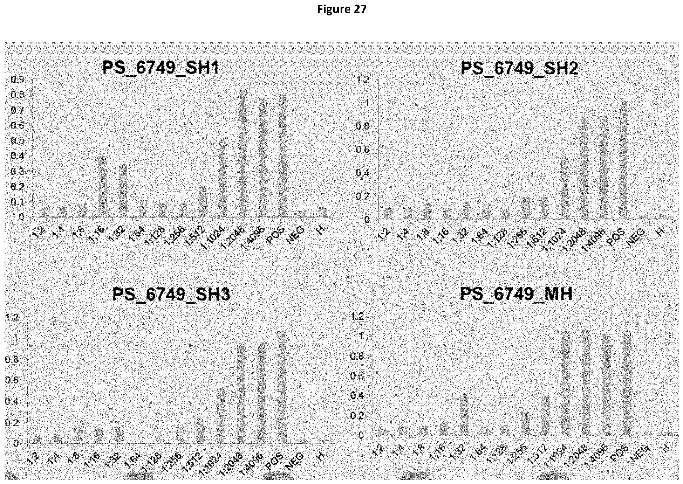

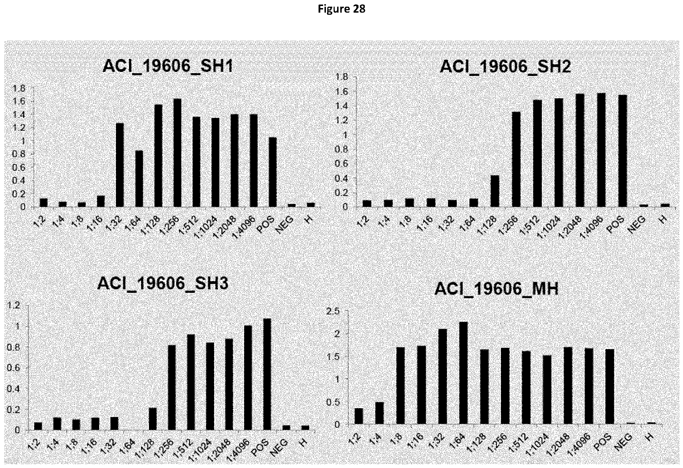

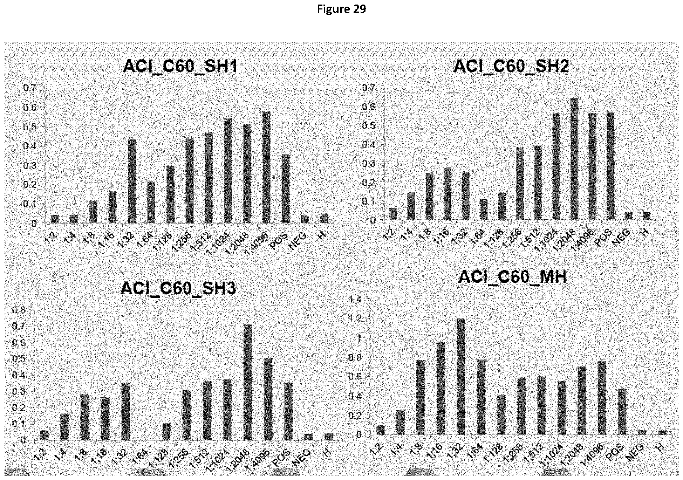

The results are shown in Figures 26-29, and summarised in the table below, which records the Minimum Biofilm Inhibitory Concentration (MBIC) for each isolate (using an OD of 0.3 as a cut-off):

| Isolate | MBIC | | | |
|---------|------|------|------|------|
| | SH1 | SH2 | SH3 | MH |
| PS_1586 | 1:8 | 1:64 | 1:32[1] | 1:2[2] |
| PS_6749 | 1:8 | 1:512 | 1:512 | 1:16[3] |
| ACI_C60 | 1:16[4] | 1:128[4] | 1:16[1] | 1:4 |
| ACI_19606 | 1:16 | 1:64 | 1:128 | 1:2 |
| [1] 1:64 dilution not tested<br>[2] Enhanced biofilming observed at 1:4 dilution compared to lower dilutions (see below)<br>[3] Enhanced biofilming observed at 1:32 dilution compared to lower dilutions (see below)<br>[4] Enhanced biofilming observed at 1:8 to 1:32 dilutions compared to lower dilutions (see below) | | | | |

The positive controls (POS) show that all the isolates were able to form a biofilm, and the negative controls (NEG) show that there was no contamination.

For some of the isolates, enhanced biofilming is observed at certain concentrations, compared with biofilming at higher and lower concentrations, of the Surgihoney or Manuka honey. This effect has been observed occasionally with other biocides. It is believed to be due to the 'stress' of the biocide causing enhanced biofilming.

Conclusions

It was concluded from these results that each Surgihoney preparation (S1, S2, and S3) was able to prevent each of the isolates tested from forming a biofilm. The S2 and S3 Surgihoney preparations were able to do so at lower concentrations than S1 Surgihoney.

The optimum Surgihoney preparation for preventing biofilm formation of the isolates tested was S2. A dilution of 1:64 S2 was able to prevent biofilm formation of each isolate tested.

Each strength preparation of Surgihoney tested (S1, S2, and S3) was generally able to prevent biofilm formation at lower concentrations than Manuka honey.

**Example 29** (Reference Example)

Prevention of pre-formed biofilm seeding by Surgihoney

This example describes the effect of Surgihoney S1, S2, and S3 preparations, and Manuka honey (MH), on the prevention or reduction of the seeding of pre-formed biofilms produced by two isolates of *Acinetobacter baumannii* (ACI_AYE and ACI_C59).

Methods

200μl of a serially diluted overnight culture of *Acinetobacter baumannii* (ACI_AYE or ACI_C59) was added to each well of a 96-well microtitre plate. A PCR peg plate was placed on top of the microtitre plate so that each well contains a 'peg' on which a biofilm can form. The 96-well plate was then incubated for 72 hours at 33°C to encourage biofilms to grow.

After 72 hours, the pegs were washed and then placed into a further 96-well plate with wells containing the test agent (Surgihoney or Manuka honey), or broth alone (for the controls). After 24 hours, the pegs were washed and then placed into another 96-well plate containing sterile broth for overnight incubation. The OD of the broth was assessed the following day using the spectrophotometer (as described in Examples 27 and 28 above). Turbid broth has a high OD and represents successful seeding of the biofilm.

Finally, the pegs were subjected to a crystal violet assay (as described in Examples 27 and 28) to prove that biofilms were present on the pegs in the first place.

Results

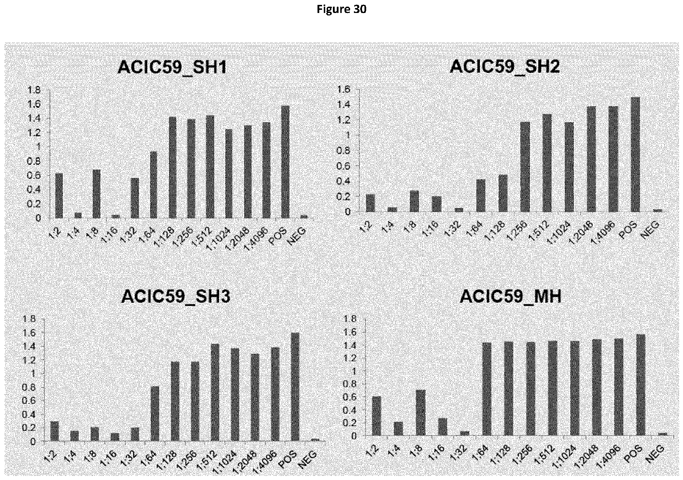

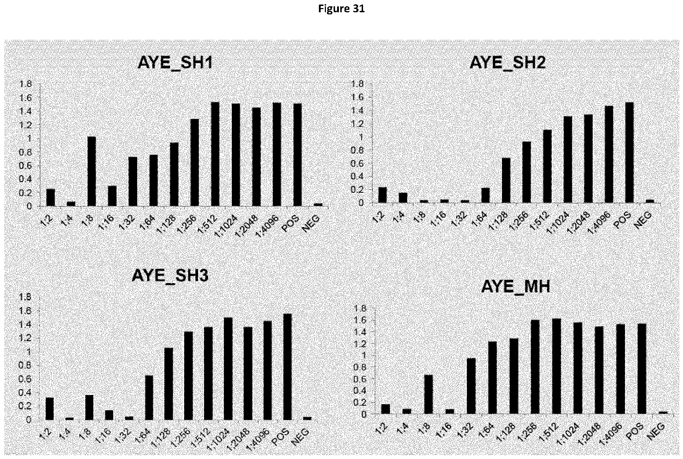

The results are shown in Figures 30 and 31.

In the Figures, the y-axis represents the OD of the broth and, therefore, the amount of seeding. As expected, a large amount of biofilm seeding was observed with each positive control, and minimal background turbidity was observed with each negative control. Biofilms were present on all of the pegs. The results allow determination of the Minimum Biofilm Eradication Concentration (MBEC) of Surgihoney.

**S1:** Reduced seeding was observed as low as the 1:16 dilution of S1 for both isolates. Some turbidity was observed at the 1:2 dilution. It is believed that this may have been an artefact due to some of these test wells being in the corner of the plate and, therefore, being more difficult to wash. Some turbidity was also observed at the 1:8 dilution. Again, this is believed to have been an artefact, possibly because these test wells were next to the positive control on the plate.

**S2:** Reduced seeding was observed for the 1:2 to 1:32/1:64 dilutions for both isolates. As for SH1, some turbidity was observed at the 1:2 dilution. This may have been due to some of these test wells being in the corner of the plate and, therefore, being more difficult to wash.

**S3:** Reduced seeding was observed for the 1:2 to 1:32/1:64 dilutions for both isolates. As for S1 and S2, some turbidity was observed at the 1:2 dilution. This may have been due to some of these test wells being in the corner of the plate and, therefore, being more difficult to wash.

**MH:** Reduced seeding was observed for the 1:2 to 1:16/1:32 dilutions.

Conclusions

The results confirm that Surgihoney prevented or reduced the seeding of pre-formed biofilms. The S2 and S3 preparations of Surgihoney were able to prevent or reduce biofilm seeding at lower concentrations than the S1 preparation, although there appeared to be little difference in potency between the S2 and S3 preparations. The S2 and S3 preparations appeared to be slightly more potent than Manuka honey in reducing biofilm seeding.

**Example 30** (Reference Example)

Prevention of biofilm formation by Surgihoney compared with commercially available wound dressings

This example describes the effect of Surgihoney S1, S2, and S3 preparations on prevention of biofilm formation by *Pseudomonas aeruginosa* (control strain PA01), and *Acinetobacter baumannii* (control strain AYE), in comparison with deactivated Surgihoney (DE), Manuka honey (MH: 'Comvita Manukacare 18+'), acetic acid (AA), and several commercially available wound dressings, and wound creams.

Methods

Overnight cultures of the isolates were diluted in Muller-hinton broth to an OD600 of 0.1.

Surgihoney S1, S2, and S3, deactivated Surgihoney (DE), and Manuka honey (MH: 'Comvita Manukacare 18+'), were tested at serial dilutions of 1:2 - 1:256 (using water as diluent). 1 ml honey was placed in each well with 1ml diluted isolate culture.

$1cm^2$ of each of the following commercially available dressings was added to 1 ml water and 1ml diluted isolate culture: Mepilex Ag; Urgotul Ag; Acticoat (Ag); Urgotul (no AM agent); Mesitran Net (honey-based dressing); Polymem (no AM agent).

Commercially available creams Trimovate and Flamazine (used for treatment of skin infections) were also tested. Acetic acid was also tested from 5% down to 0.04%.

The plate was incubated for 72 hours at 33°C (wound temperature) to encourage biofilm formation. The plate was then developed using crystal violet dye, and visualised following solubilisation of the dye using 70% ethanol.

A spectrophotometer was then used to assess the optical density (OD) of the solubilised dye. The OD reading corresponds to the amount of incorporated crystal violet. Higher OD readings represent dark wells, and greater mass of biofilm. The OD readings were then plotted to make a graph.

Results

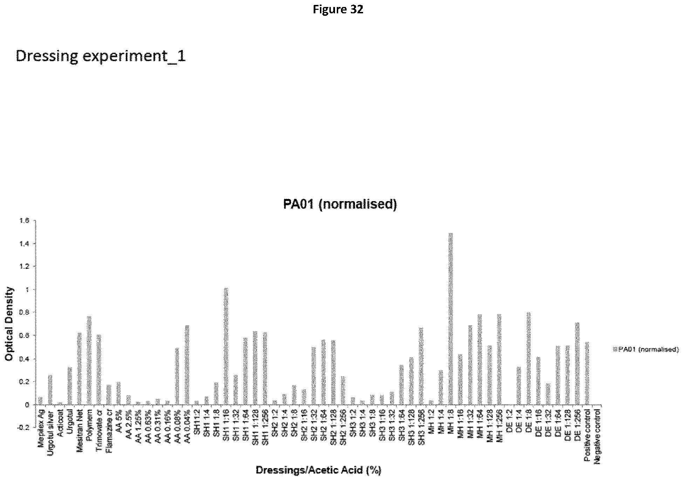

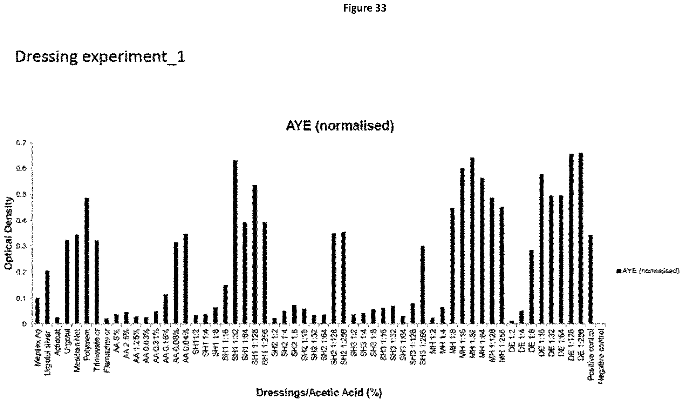

The results are shown in Figures 32 and 33.

The results show that Acticoat and Mepilex Ag dressings (and to a lesser extent, Urgotel silver) were effective at preventing biofilm formation of both strains, as was the Flamazine cream. The Urgotul and Polymem dressings, and Mesitran net (a commercial honey-based dressing), and the Trimovate cream did not appear to be effective in preventing biofilm formation.

Acetic acid was effective at preventing biofilm formation down to 0.31% (AYE) and 0.1% (PA01).

All of the Surgihoneys tested were effective at preventing biofilm formation of both strains at 1:2 and 1:4 dilution, at least, and some at lower concentrations (for example, S2 was effective against AYE at 1:64 dilution).

The S2 and S3 Surgihoneys were more effective in preventing biofilm formation at lower concentrations than S1 Surgihoney, and each Surgihoney preparation was more effective at lower concentrations than Manuka honey (see, for example, the effect of the 1:8 dilution for each). Manuka honey was effective in preventing biofilm formation down to 1:2 to 1:4 dilution.

The results also show that the deactivated Surgihoney (DE) was effective in preventing biofilm formation. However, when the DE honey was tested for hydrogen peroxide activity, it was found to retain some hydrogen peroxide activity, and so did not appear to have been fully inactivated.

Conclusions

It was concluded from these results that Surgihoney was comparable in preventing biofilm formation to several commercially available wound dressings, and more effective than Mesitran net (a commercial honey-based dressing).

In the above examples, four isolates of *A. baumanii*, and three of *P. aeruginosa* were tested and Surgihoney was able to prevent biofilm formation of all isolates in a dose-dependent manner. Pre-formed biofilms of *A. baumannii* were additionally exposed to all Surgihoney formulations for 24 hours. Reduced seeding of the biofilms was observed for both strains tested and was again dose-dependent.

The dressings experiment (Example 30) revealed Surgihoney to be equally or more effective in biofilm prevention than three of the eight commercial creams and dressings tested.

Furthermore, in this *in vitro* test, Surgihoney was more effective than L-Mesitran net at preventing biofilm formation of single isolates of *A. baumanii* and *P. aeruginosa*.

These results show that Surgihoney has potent anti-biofilming activity against key Gram-negative pathogens of burn wounds, and superior activity to the majority of commercial dressings tested. This composition can be used in place of antiboiotics, and in an era of increasing antibiotic resistance, help to reduce inappropriate antibiotic use.

**Example 31** (Reference Example)

Antimicrobial activity of electrospun wound dressings containing Surgihoney

An aqueous solution containing 4% by weight polyethylene oxide (PEO) was mixed with Surgihoney in a weight ratio of 80% PEO solution to 20% Surgihoney. The composition was electrospun on to an alginate substrate using an Elmarco NanoSpider™ electrospinning apparatus, at a voltage of 60kV, a separation distance of 17cm and an electrode rotation of 10 rpm, to form a Surgihoney dressing.

A 5% (by weight) aqueous PEO solution (not containing Surgihoney) was also electrospun on to an alginate substrate, using the same electrospinning apparatus and parameters, to form a control dressing.

In a first test, an overnight culture of *Escherichia coli* was spread over the surface of three petri dishes containing nutrient agar. Samples of control dressings and Surgihoney dressings (approx. 2x2 cm) were placed on the surface of the cultures. The control dressing was placed on the left side of each plate and the Surgihoney dressing was applied to the right side of each plate. The plates were incubated overnight for 18 hrs at 37°C.

In a second test, an overnight culture of *Staphylococcus aureus* was spread over the surface of three petri dishes containing nutrient agar. Samples of control dressings and Surgihoney dressings (approx. 2x2 cm) were placed on the surface of the cultures. The control dressing was placed on the left side of each plate and the Surgihoney dressing was applied to the right side of each plate. The plates were incubated overnight for 18 hrs at 37°C.

In a third test, a Surgihoney dressing and a control dressing were tested for the presence of hydrogen peroxide. The presence of hydrogen peroxide was determined using the Peroxide Test Kits from *Merckoquant*® (code number 1.10011). The dressings were wetted before application of the Peroxide Test Kits.

Results

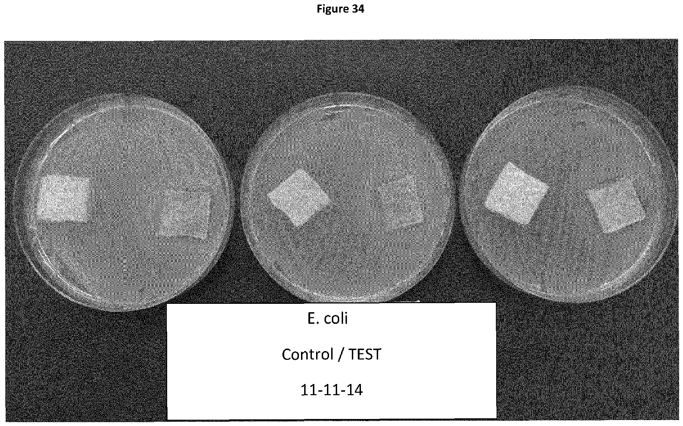

The results of the first test are shown in Figure 34. There was no Zone of Inhibition (ZOI) around the control on the left and a ZOI of approx. 1-2mm around the active sample on the right of each plate, demonstrating activity against Gram-negative bacterium.

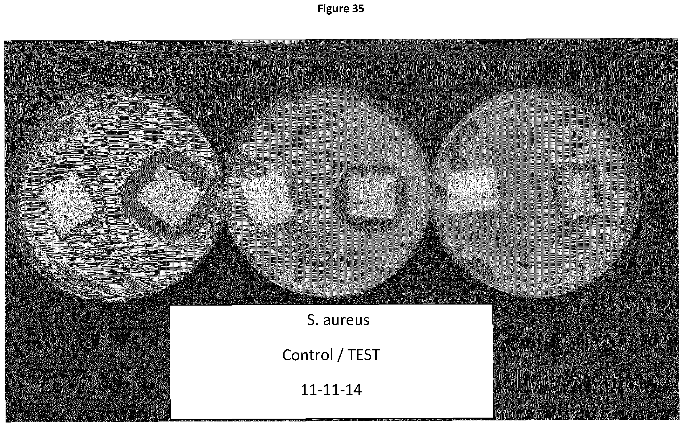

The results of the second test are shown in Figure 35. There was no ZOI around the control on the left and a clear ZOI of approx. 5-10mm around the active sample on the right of each plate, demonstrating activity against Gram-positive bacterium.

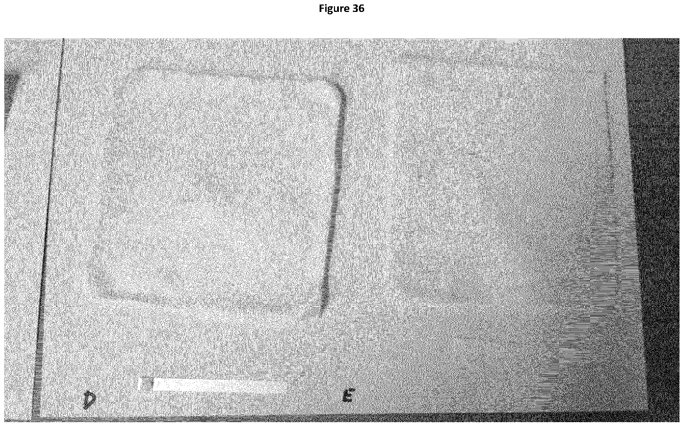

The results of the third test are shown in Figure 36. On the left is the active Surgihoney dressing. The presence of a blue colour indicates that the electrospun Surgihoney dressing (marked D) is producing hydrogen peroxide and the control dressing on the right (marked E) does not have hydrogen peroxide-generating capability.

**Example 32** (Reference Example)

Spravable compositions

A composition containing 1% by weight polyethylene oxide (PEO), 79% by weight deionised water and 20% by weight Surgihoney was added to a pump action spray device.

The spray device was used to spray the composition on to a hydrogen peroxide test strip. The test strip indicated the presence of hydrogen peroxide in the composition.

After five months, the spray was re-tested for its ability to generate hydrogen peroxide, by spraying on to a hydrogen peroxide test strip. The test strip indicated the presence of hydrogen peroxide in the composition.

**Example 33** (Reference Example)

Anti-viral activity of Surgihoney

SH1 or SH2 Surgihoney was mixed with Herpes Simplex Virus (HSV) ($50\mu$g honey and $50\mu$l virus) and incubated for 1 hour at 37°C. A dilution series ($10^{-2}$, $10^{-3}$, $10^{-4}$, $10^{-5}$) was then made from the mixture, and the dilutions were used in a plaque reduction assay. Controls with no honey, or with control honey were also performed. The number of viral plaques formed for each dilution was recorded. The results are shown in the Table below.

| | | Experiment 1 | | | Experiment 2 | | |
|---|---|---|---|---|---|---|---|
| Honey | Dilution | well 1 | well 2 | well 3 | well 1 | well 2 | well 3 |
| SH1 | -2 | * | * | * | * | * | * |
| | -3 | 1 | 1 | 5 | 0 | 0 | 0 |
| | -4 | 0 | 1 | 1 | 0 | 0 | 0 |
| | -5 | 0 | 0 | 0 | 0 | 0 | 0 |
| SH2 | -2 | * | * | * | * | * | * |
| | -3 | 0 | 0 | 0 | 0 | 0 | 0 |
| | -4 | 0 | 0 | 0 | 0 | 0 | 0 |
| | -5 | 0 | 0 | 0 | 0 | 0 | 0 |

(continued)

| Honey | Dilution | Experiment 1 | | | Experiment 2 | | |
|---|---|---|---|---|---|---|---|
| | | well 1 | well 2 | well 3 | well 1 | well 2 | well 3 |
| Control Honey | -2 | 100 | 95 | 88 | 108 | 128 | 106 |
| | -3 | 13 | 15 | 11 | 14 | 12 | 15 |
| | -4 | 2 | 1 | 2 | 3 | 2 | 2 |
| | -5 | 0 | 0 | 0 | 0 | 0 | 1 |
| No Honey | -2 | | | | 160 | 158 | 164 |
| | -3 | | | | 28 | 22 | 18 |
| | -4 | | | | 6 | 4 | 1 |
| | -5 | | | | 1 | 0 | 1 |

The results show that SH1 and SH2 Surgihoney was strongly virucidal against HSV in both experiments.

**Example 34** (Reference Example)

Cytotoxic activity of Surgihoney

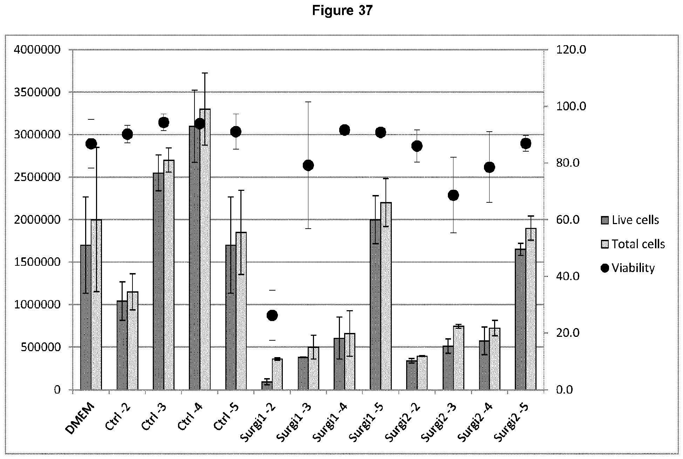

SH1 or SH2 Surgihoney (50$\mu$g honey diluted $10^{-2}$, $10^{-3}$, $10^{-4}$, $10^{-5}$) was incubated on cells for 2 days. The number of live cells, and the total number of cells was counted (percentage viability = live/total x 100). The results are shown in the table below, and in Figure 37.
The results show that SH1 Surgihoney was cytotoxic at the $10^{-2}$ dilution, and cytostatic at the $10^{-3}$ and $10^{-4}$ dilutions, and that SH2 Surgihoney was cytostatic at the $10^{-2}$, $10^{-3}$ and $10^{-4}$ dilutions. SH1 and SH2 Surgihoney were not cytotoxic or cytostatic at the $10^{-5}$ dilution.
It is concluded from the results in Examples 33 and 34 that Surgihoney can be administered at doses which are virucidal but not cytotoxic or cytostatic.

| Condition | Dilution | Number of live cells | | | | Total number of cells | | | | Percentage viability | | | |
|---|---|---|---|---|---|---|---|---|---|---|---|---|---|
| | | rep1 | rep2 | Ave | standard deviation | rep1 | rep2 | Ave | standard deviation | rep1 | rep2 | Ave | standard deviation |
| DMEM | - | 1300000 | 2100000 | 1700000 | 565685.4 | 1400000 | 2600000 | 2000000 | 848528.1 | 92.9 | 80.8 | 86.8 | 8.5 |
| Control honey | -2 | 1200000 | 880000 | 1040000 | 226274.2 | 1300000 | 1000000 | 1150000 | 212132 | 92.3 | 88.0 | 90.2 | 3.0 |
| | -3 | 2700000 | 2400000 | 2550000 | 212132 | 2800000 | 2600000 | 2700000 | 141421.4 | 96.4 | 92.3 | 94.4 | 2.9 |
| | -4 | 3400000 | 2800000 | 3100000 | 424264.1 | 3600000 | 3000000 | 3300000 | 424264.1 | 94.4 | 93.3 | 93.9 | 0.8 |
| | -5 | 2100000 | 1300000 | 1700000 | 565685.4 | 2200000 | 1500000 | 1850000 | 494974.7 | 95.5 | 86.7 | 91.1 | 6.2 |
| SH1 | -2 | 120000 | 70000 | 95000 | 35355.34 | 370000 | 350000 | 360000 | 14142.14 | 32.4 | 20.0 | 26.2 | 8.8 |
| | -3 | 380000 | 380000 | 380000 | 0 | 400000 | 600000 | 500000 | 141421.4 | 95.0 | 63.3 | 79.2 | 22.4 |
| | -4 | 430000 | 780000 | 605000 | 247487.4 | 470000 | 850000 | 660000 | 268700.6 | 91.5 | 91.8 | 91.6 | 0.2 |
| | -5 | 1800000 | 2200000 | 2000000 | 282842.7 | 2000000 | 2400000 | 2200000 | 282842.7 | 90.0 | 91.7 | 90.8 | 1.2 |
| SH2 | -2 | 320000 | 360000 | 340000 | 28284.27 | 390000 | 400000 | 395000 | 7071.068 | 82.1 | 90.0 | 86.0 | 5.6 |
| | -3 | 450000 | 570000 | 510000 | 84852.81 | 760000 | 730000 | 745000 | 21213.2 | 59.2 | 78.1 | 68.6 | 13.3 |
| | -4 | 460000 | 690000 | 575000 | 162634.6 | 660000 | 790000 | 725000 | 91923.88 | 69.7 | 87.3 | 78.5 | 12.5 |
| | -5 | 1600000 | 1700000 | 1650000 | 70710.68 | 1800000 | 2000000 | 1900000 | 141421.4 | 88.9 | 85.0 | 86.9 | 2.7 |

**Example 35** (Reference Example)

Dried Surgihoney

Dried honey granules (K24289) were supplied by Kanegrade Limited (Stevenage, UK). The dried honey granules were produced by vacuum drying and contained honey solids with skimmed milk powder. The water content was less than 3%.

The dried honey granules were activated by adding glucose oxidase at S1 (SH1) and S2 (SH2) levels (see Example 20).

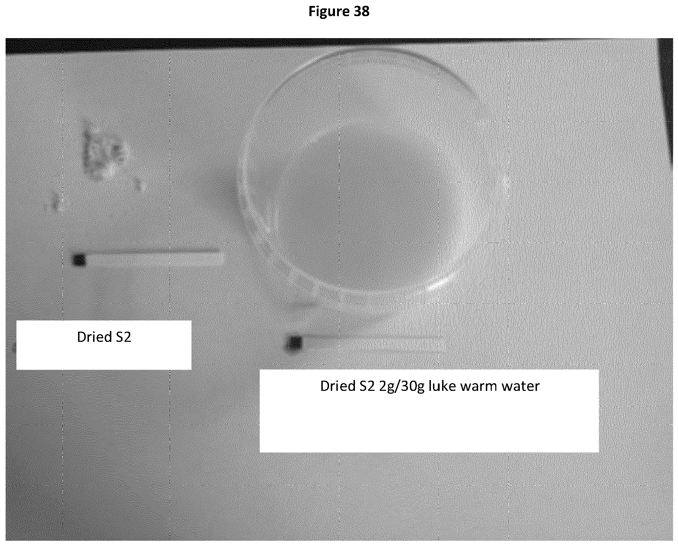

Figure 38 (left) demonstrates that adding water to the activated dried honey granules led to the immediate production of hydrogen peroxide at between 50 and 100 ppm, as detected by a hydrogen peroxide indicator strip.

Figure 38 (right) demonstrates the result of adding 2g of the activated dried honey granules to 30g of sterile warm water (at approximately 35°C). The activated honey granules dissolved easily with no scum formation. Even after four days, there was no settling out, as the granules remained fully dissolved.

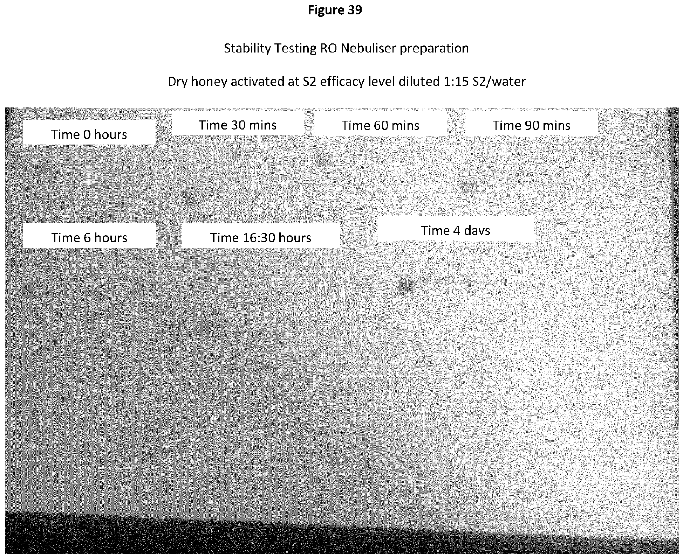

Figure 39 demonstrates that the activity of the dissolved active honey was retained over an extended period of time. Top row (left to right): 0 hours; 30 mins; 60 mins; 90 mins. Bottom row (left to right): 6 hours, 16.5 hours; 4 days. After four days, the level of hydrogen peroxide remained constant at between 50 and 100 ppm. This shows that the glucose oxidase remains active and there was sufficient glucose present to produce hydrogen peroxide over an extended period.

**Example 36** (Reference Example)

Stability of aqueous Surgihoney compositions

A sample was prepared with the following composition, on 9 November 2014:

SH2 Surgihoney: 20 % by weight

PVA: 5 % by weight

Water: 75 % by weight

The sample was tested on 16 October 2015 using a hydrogen peroxide test strip and was found to still be producing hydrogen peroxide at a level of between 30-50 ppm.

A sample was prepared by mixing SH2 Surgihoney with water in a ratio of 1:15 (Surgihoney:water, by weight), on 14 September 2015. The sample was tested on 16 October 2015 using a hydrogen peroxide test strip and was found to still be producing hydrogen peroxide at a level of 30-50 ppm.

**Example 37** (Reference Example)

Use of Surgihoney for the topical treatment of lower genital tract infections

Surgihoney has been used to treat persistent vaginal discharge that had not been responsive to standard therapy. The indications were vaginal discharge with various aetiologies e.g. bacterial vaginosis and general bacterial vaginal discharge.

Tampons coated with Surgihoney were inserted into the vagina and were replaced every 24 hours. The therapeutic outcome was good and there were no reported adverse effects.

**Example 38** (Reference Example)

Use of Surgihoney to treat CPE

A patient developed a soft tissue infection of the foot whilst in India and was diagnosed with necrotising fasciitis. This required extensive debridement of dead tissue as well as antibiotics. Post-operatively, the patient was found to be colonised in the debrided wounds with CPE (Carbapenemase-producing Enterobacteriaceae). The patient required isolation and was treated with Surgihoney, which cleared the organisms.

**Example 39** (Reference Example)

Compositions comprising non-aqueous solvent

Sample 1

Lightweight hydro entangled fabric (40 g per square meter)

Coated with 25% $^{w}/_{w}$ honey/glycerol solution

Weight of coating on fabric 100 g per square meter

Suggested application: Absorbent fabric in a wound dressing.

Sample 2

Lightweight hydro entangled fabric (40 g per square meter)

Coated with 75% $^{w}/_{w}$ honey/glycerol solution

Weight of coating on fabric 100 g per square meter

Suggested application: Absorbent fabric in a wound dressing.

Sample 3

Lightweight hydro entangled fabric (40 g per square meter)

Coated with 25% $^{w}/_{w}$ honey/glycerol solution

Weight of coating on fabric 300 g per square meter

Suggested application: Anti bacterial wipe

Sample 4

Lightweight hydro entangled fabric (40 g per square meter)

Coated with 75% $^{w}/_{w}$ honey/glycerol solution

Weight of coating on fabric 300 g per square meter

Suggested application: Anti bacterial wipe

Sample 5

Paper like fabric (42 g per square meter)

Coated with $25\%$ $^w/_w$ honey/glycerol solution

Weight of coating on fabric 100 g per square meter

Suggested application: Anti bacterial wipe

Sample 6

Boots® Wound Dressing coated with $25\%$ $^w/_w$ honey/glycerol solution

Weight of coating on fabric 400 g per square meter

Sample 7

Boots® Adhesive Wound Dressing with absorbent pad coated with $25\%$ $^w/_w$ honey/glycerol solution

Weight of coating on fabric 150 g per square meter

Sample 8

Lightweight hydro entangled fabric (40 g per square meter)

Coated with 25% $25\%$ $^w/_w$ Surgihoney/glycerol solution

Weight of coating on fabric 100 g per square meter

Suggested application: Absorbent fabric in a wound dressing.

Sample 9

Lightweight hydro entangled fabric (40 g per square meter)

Coated with $25\%$ $^w/_w$ Surgihoney/glycerol solution

Weight of coating on fabric 300 g per square meter.

**Example 40** (Reference Example)

Compositions comprising honey, non-aqueous solvent and additional water.

The following composition has been formulated which readily sprays from a Boots®Travel Spray bottle (all amounts by weight):

| | |
|---|---|
| Honey (Surgihoney): | 25% |
| Glycerol: | 52.5% |
| Water: | 22.5% |

If the water content in the honey is included, this composition has a mole fraction of water of 68.9%.

The spray bottle is operated by a manual pump.

Pump inlet tube length: 100mm

Tube bore: 1.5mm

Outlet bore: 2.5 mm

Pump delivery: 0.14g of water per stroke.

**Example 41** (Reference Example)

Cytotoxicity of Surgihoney against mast cells

Mast cells are sentinel cells located just underneath the epithelium in tissues with close contact to the external environment, such as the nasal mucosal tissues. They play an important role in recognition of pathogens and modulation of immune responses. This example describes the effects of different concentrations of Surgihoney on cells of the human mast cell line HMC-1.

Methods

A) Cell culture

Different concentrations of S1, S2 and S3 preparations of Surgihoney, and the control honey (Acacia), were used: 100g, 40g, 10g, 1g/L. These were made up using a cell growth medium (IMDM, 10% FBS, 2% Penicillin Streptomycin). 2 million HMC-1 cells were added into 12 well plates, 2ml of each concentration was used.

B) Cell survival assay

Cell survival was assessed at 3, 6 and 24 hours by staining a 20$\mu$l sample with Trypan blue.

C) Live and Dead Assay

Live and dead cells were counted using a haemocytometer. The percentage of dead cells was calculated and the data was analysed using GraphPad Prism.

Results

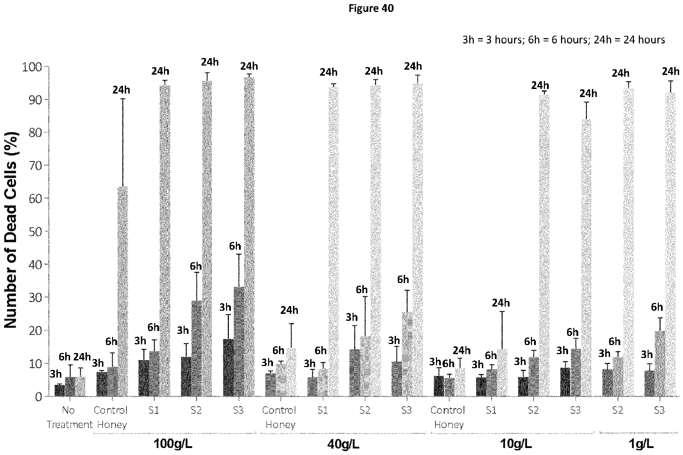

The results of 3, 6 and 24 hour culture of HMC-1 cells with different concentrations of S1, S2, and S3 Surgihoney are shown in Figure 40. High concentrations of both Surgihoney (S1) and control Acacia honey were detrimental to HMC-1 survival. HMC-1 cell morphology was observed to change when using 100g/L of S1 Surgihoney. However, S2 and S3 Surgihoney at 10g/L and 40g/L showed a low percentage cell death of HMC-1 cells at 3 and 6 hours.

Conclusions

These results indicate that it may be beneficial to use Surgihoney (particularly S1, S2, or S3 preparations, preferably S2 or S3 preparations), or other compositions of the invention, at concentrations of less than 100 g/L, preferably 40g/L or less, 10g/L or less, or 1g/L or less, for treatment of microbial infections in areas where mast cells are present, such as epithelium in tissues with close contact to the external environment, for example nasal mucosal tissues.

It may also be beneficial to limit contact of Surgihoney, or other compositions of the invention, with cells for less than 24 hours, preferably less than 6 hours, or less than 3 hours, especially when contacting areas where mast cells are present, such as epithelium in tissues with close contact to the external environment, for example nasal mucosal tissues.

**Claims**

1. A sterile liquid or gel composition for generating antimicrobial activity, comprising: an enzyme that is able to convert a substrate to release hydrogen peroxide; and a substance that includes a substrate for the enzyme, wherein the composition comprises less than 10% by weight of water, and wherein the composition does not comprise honey or any other unrefined natural substance.

2. A composition according to claim 1, comprising 5% or less (by weight) of water.

3. A composition according to any preceding claim, comprising a non-aqueous solvent.

4. A composition according to any preceding claim, wherein the non-aqueous solvent is glycerol.

5. A composition according to claim 3 or claim 4, wherein the non-aqueous solvent is at least 25% by weight of the composition.

6. A composition according to any preceding claim, comprising substantially no hydrogen peroxide.

7. A composition according to any preceding claim, comprising a polymer.

8. A composition according to claim 7, wherein the polymer is selected from polyethylene glycol, polyvinyl alcohol and polyvinylpyrrolidone.

9. A composition according to any preceding claim, which is electrospinnable.

10. A composition according to any preceding claim, in which the substance includes a purified substrate for the enzyme.

11. A composition according to any preceding claim, which does not contain any added peroxidase.

12. A composition as defined in any preceding claim, for use as a medicament.

13. A composition as defined in any of claims 1 to 11, for use in treating a wound.

14. A composition as defined in any of claims 1 to 11, for use in the prevention, treatment, or amelioration of a microbial infection.

15. A method comprising electrospinning a composition according to claim 9, or any claim dependent on claim 9.

**Patentansprüche**

1. Sterile flüssige oder gelartige Zusammensetzung zur Erzeugung antimikrobieller Aktivität, die Folgendes umfasst: ein Enzym, das ein Substrat zum Freisetzen von Wasserstoffperoxid umwandeln kann; und eine Substanz, die ein Substrat für das Enzym enthält, wobei die Zusammensetzung weniger als 10 Gew.-% Wasser umfasst und wobei die Zusammensetzung keinen Honig oder eine irgendeine andere unraffinierte natürliche Substanz umfasst.

2. Zusammensetzung nach Anspruch 1, die 5 Gew.-% oder weniger Wasser enthält.

3. Zusammensetzung nach einem vorherigen Anspruch, die ein nichtwässriges Lösungsmittel enthält.

4. Zusammensetzung nach einem vorherigen Anspruch, wobei das nichtwässrige Lösungsmittel Glycerin ist.

5. Zusammensetzung nach Anspruch 3 oder Anspruch 4, wobei das nichtwässrige Lösungsmittel mindestens 25 Gew.-% der Zusammensetzung ausmacht.

6. Zusammensetzung nach einem vorherigen Anspruch, die im Wesentlichen kein Wasserstoffperoxid enthält.

7. Zusammensetzung nach einem vorherigen Anspruch, die ein Polymer umfasst.

8. Zusammensetzung nach Anspruch 7, wobei das Polymer aus Polyethylenglykol, Polyvinylalkohol und Polyvinylpyrrolidon ausgewählt ist.

9. Zusammensetzung nach einem vorherigen Anspruch, die elektroverspinnbar ist.

10. Zusammensetzung nach einem vorherigen Anspruch, wobei die Substanz ein gereinigtes Substrat für das Enzym enthält.

11. Zusammensetzung nach einem vorherigen Anspruch, die keine zugesetzte Peroxidase enthält.

12. Zusammensetzung nach einem vorherigen Anspruch zur Verwendung als Arzneimittel.

13. Zusammensetzung nach einem der Ansprüche 1 bis 11 zur Verwendung bei der Behandlung einer Wunde.

14. Zusammensetzung nach einem der Ansprüche 1 bis 11 zur Verwendung bei der Vorbeugung, Behandlung oder Verbesserung einer mikrobiellen Infektion.

15. Verfahren, das das Elektroverspinnen einer Zusammensetzung nach Anspruch 9 oder einem von Anspruch 9 abhängigen Anspruch beinhaltet.

**Revendications**

1. Composition de liquide ou de gel stérile permettant de générer une activité antimicrobienne, comprenant : une enzyme qui est capable de convertir un substrat pour libérer du peroxyde d'hydrogène ; et une substance qui comporte un substrat pour l'enzyme, dans laquelle la composition comprend moins de 10 % en poids d'eau, et dans laquelle la composition ne comprend pas de miel ou toute autre substance naturelle non raffinée.

2. Composition selon la revendication 1, comprenant 5 % ou moins (en poids) d'eau.

3. Composition selon l'une quelconque revendication précédente, comprenant un solvant non aqueux.

4. Composition selon l'une quelconque revendication précédente, dans laquelle le solvant non aqueux est le glycérol.

5. Composition selon la revendication 3 ou la revendication 4, dans laquelle le solvant non aqueux représente au moins 25 % en poids de la composition.

6. Composition selon l'une quelconque revendication précédente, ne comprenant pratiquement pas de peroxyde d'hydrogène.

7. Composition selon l'une quelconque revendication précédente, comprenant un polymère.

8. Composition selon la revendication 7, dans laquelle le polymère est choisi parmi le polyéthylène glycol, l'alcool polyvinylique et la polyvinylpyrrolidone.

9. Composition selon l'une quelconque revendication précédente qui est électrofilable.

10. Composition selon l'une quelconque revendication précédente, dans laquelle la substance comporte un substrat purifié pour l'enzyme.

11. Composition selon l'une quelconque revendication précédente, qui ne contient aucune peroxydase ajoutée.

12. Composition selon l'une quelconque revendication précédente, destinée à être utilisée en tant que médicament.

13. Composition selon l'une quelconque des revendications 1 à 11, destinée à être utilisée dans le traitement d'une plaie.

14. Composition selon l'une quelconque des revendications 1 à 11, destinée à être utilisée dans la prévention, le traitement, ou l'amélioration d'une infection microbienne.

**15.** Procédé comprenant l'électrofilage d'une composition selon la revendication 9 ou une quelconque revendication dépendante de la revendication 9.

# Figure 1

a)

b)

Figure 2

## Figure 3

a)

b)

# Figure 4

a)

b)

c)

# Figure 5

a)

b)

## Figure 6

a)

b)

# Figure 7

a)

b)

Figure 8

a)

b)

## Figure 9

a)

b)

c)

# Figure 10

a)

b)

c)

# Figure 11

a)

b)

c)

# Figure 12

a)

b)

# Figure 13

a)

b)

c)

# Figure 14

a)

b)

c)

## Figure 15

a)

b)

## Figure 16

a)

b)

# Figure 17

a)

b)

c)

## Figure 18

a)

b)

c)

**VRE**

d)

*Pseudomonas aeruginosa*

e)

**Klebsiella**

f)

**E coli ESBL**

g)

**Enterococcus faecalis**

h)

Figure 19

**Figure 20**

a)

b)

Figure 21

Figure 22

**Figure 23**

A)

Pseudomonas: Surgihoney Neat

B)

Pseudomonas: Surgihoney dilutions

**Figure 23 (continued)**

C)

D)

Figure 24

A)

Manukahoney Neat: Pseudomonas

B)

Manukahoney Neat: Acinetobacter

Figure 24 (continued)

C)

**Figure 25**

A)

Figure 25 (continued)

B)

Figure 26

Figure 27

**Figure 28**

**Figure 29**

Figure 30

EP 3 858 392 B1

Figure 31

**Figure 32**

Dressing experiment_1

PA01 (normalised)

Figure 33

Dressing experiment_1

Figure 34

E. coli

Control / TEST

11-11-14

Figure 35

S. aureus

Control / TEST

11-11-14

Figure 36

Figure 37

**Figure 38**

Dried S2

Dried S2 2g/30g luke warm water

**Figure 39**

Stability Testing RO Nebuliser preparation

Dry honey activated at S2 efficacy level diluted 1:15 S2/water

Figure 40

3h = 3 hours; 6h = 6 hours; 24h = 24 hours

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008049578 A
- WO 2008049251 A
- WO 2011059497 A
- US 1987893 A

### Non-patent literature cited in the description

- **LAZARUS et al.** *Arch Dermatol.,* 1994, vol. 130 (4), 489-493
- **COWAN et al.** *Ulcers,* 2013
- **KAPLAN.** *J Dent Res,* 2010, vol. 89 (3), 205-218
- *Revised Codex Standard for Honey,* 2001
- **RCIEGG, M ; BLANC, B.** The water activity of honey and related sugar solutions. *Lebensmittel-Wissenschaft und Technologie,* 1981, vol. 14, 1-6
- **MOLAN, P. C.** The antibacterial activity of honey: 1. The nature of the antibacterial activity. *Bee World,* 1992, vol. 73 (1), 5-28
- *CHEMICAL ABSTRACTS,* 9001-37-0
- **SIDDIQUI AR ; BERNSTEIN JM.** Chronic wound infection: Facts and controversies. *Clinics in Dermatology,* 2010, vol. 28, 519-26
- **EDMONDS, M. ; FOSTER, A.** The use of antibiotics in the diabetic foot. *Am J Surg,* 2004, vol. 187 (5A), 25S-28S
- **ANDREWS JM.** Determination of minimum inhibitory concentrations. *J Antimicrob 372 Chemother,* 2001, vol. 48 (1), 5-16
- **BRAGG et al.** Variation in rates of caesarean section among English NHS trusts after accounting for maternal and clinical risk: cross sectional study. *BMJ,* 2010, vol. 2010, 341
- **JAN WA.** Comparison of conventional pyodine dressing with honey dressing for the treatment of diabetic foot ulcers. *JPMI - Journal of Postgraduate Medical Institute,* 2012, vol. 26 (4), 402-7
- Joint Formulary Committee. The British National Formulary. The Pharmaceutical Press, 2013
- **PIETSCH ; MEAKINS.** Complications of povidone-iodine absorption in topically treated burn patients. *The Lancet,* 1976, vol. 307 (7954), 280-2
- **SCOGGIN et al.** Hypernatræmia and acidosis in association with topical treatment of burns. *The Lancet,* 1977, vol. 309 (8018), 959
- **DONOVAN et al.** Seizures in a Patient Treated with Continuous Povidone-Iodine Mediastinal Irrigation. *New England Journal of Medicine,* 1992, vol. 326 (26), 1784
- **COLPAERT.** Iodine toxicity as a cause of total atrioventricular block in burn patients. *Burns,* 2009, vol. 35, S45-S6
- **RAMASWAMYKANIVE.** Cardiovascular collapse following povidone-iodine wash. *Anaesthesia and Intensive Care,* 2011, vol. 39 (1), 127-30
- **LAKHAL.** Povidone iodine: Features of critical systemic absorption. *Annales Francaises d'Anesthesie et de Reanimation,* 2011, vol. 30 (7-8), e1-8, e1-e3
- **STORM-VERSLOOT et al.** Topical silver for preventing wound infection. *Cochrane Database of Systematic Reviews,* 2010
- **VERMEULEN et al.** Topical silver for treating infected wounds (Review. *Cochrane review,* 2010, vol. 10, 42
- **JULL et al.** Honey as a topical treatment for wounds. *The Cochrane Collaboration,* 2009
- **JULL et al.** Honey as a topical treatment for wounds. *Cochrane database of systematic reviews,* 2013, 2